(19) Europäisches Patentamt
European Patent Office
Office européen des brevets

(11) EP 4 458 812 A1

(12) **EUROPEAN PATENT APPLICATION**
published in accordance with Art. 153(4) EPC

(43) Date of publication:
**06.11.2024 Bulletin 2024/45**

(21) Application number: **22914793.9**

(22) Date of filing: **27.12.2022**

(51) International Patent Classification (IPC):
***C07D 237/32*** *(2006.01)* ***C07D 403/10*** *(2006.01)*
***C07D 487/00*** *(2006.01)* ***A61K 31/502*** *(2006.01)*
***A61P 35/00*** *(2006.01)*

(52) Cooperative Patent Classification (CPC):
**A61K 31/502; A61P 35/00; C07D 237/32; C07D 403/10; C07D 487/00**

(86) International application number:
**PCT/CN2022/142362**

(87) International publication number:
**WO 2023/125540 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 CN 202111616977**
**19.01.2022 CN 202210062133**
**16.02.2022 CN 202210142771**
**29.09.2022 CN 202211213451**

(71) Applicant: **Medshine Discovery Inc.**
**Nanjing, Jiangsu 210032 (CN)**

(72) Inventors:
- **FU, Zhifei**
  **Shanghai 200131 (CN)**
- **LUO, Miaorong**
  **Shanghai 200131 (CN)**
- **YU, Chenxi**
  **Shanghai 200131 (CN)**
- **ZHOU, Kai**
  **Shanghai 200131 (CN)**
- **GAO, Na**
  **Shanghai 200131 (CN)**
- **ZHANG, Yang**
  **Shanghai 200131 (CN)**
- **LI, Jian**
  **Shanghai 200131 (CN)**
- **CHEN, Shuhui**
  **Shanghai 200131 (CN)**

(74) Representative: **Dehns**
**10 Old Bailey**
**London EC4M 7NG (GB)**

(54) **PYRAZOLE-1(2H)-PHTHALAZINONE COMPOUND AND APPLICATION THEREOF**

(57) Provided are a pyrazole-1(2H)-phthalazinone compound and an application thereof as a PRMTS. MTA complex inhibitor in the preparation of a drug for treating related diseases.

EP 4 458 812 A1

## Description

The present invention claims the right of the following priorities:

CN202111616977.2, application date: December 27, 2021;
CN202210062133.6, application date: January 19, 2022;
CN202210142771.9, application date: February 16, 2022;
CN202211213451.4, application date: September 29, 2022.

TECHNICAL FIELD

The present disclosure relates to a series of pyrazole-1(2H)-phthalazinone compounds and a use thereof, specifically to a compound of formula (III) and a pharmaceutically acceptable salt thereof.

BACKGROUND

Protein arginase methyltransferase 5 (PRMT5) is a clinically potential epigenetic target. It is a type II arginine methyltransferase that post-translationally modifies histones and other proteins through symmetric dimethylation. Current research indicates that PRMT5 expression levels are elevated in various tumors.

The gene for the metabolic enzyme 5'-methylthioadenosine phosphorylase (MTAP) is located very close to the tumor suppressor gene CDKN2A on human chromosome 9p21. Therefore, in tumors with CDKN2A gene deletion, MTAP deletion is often observed, making it one of the most frequently mutated genes in tumors. MTAP deletion leads to the accumulation of its substrate methylthioadenosine (MTA). MTA can selectively inhibit the activity of PRMT5 methyltransferase, making it more sensitive to further PRMT5 inhibition. PRMT5 inhibitors can be a highly selective treatment for patients with MTAP-deficient or low-expression tumors.

In recent years, PRMT5 has garnered significant attention as a potential target for tumor therapy. Numerous small molecule inhibitors of PRMT5 have been reported, which can be classified into two categories based on whether the compounds occupy the SAM binding site: SAM non-competitive inhibitors and SAM competitive inhibitors. SAM non-competitive inhibitors occupy the substrate binding site and compete with the substrate, whereas SAM competitive inhibitors occupy the SAM binding site.

For SAM non-competitive inhibitors, although they can effectively inhibit the *in vitro* growth of many cell lines, they lack selectivity for tumor cell lines and their ability to inhibit tumor cells is independent of the status of MTAP in the cells, which may lead to a potential risk of clinical toxicity. In MTAP-deficient cells, MTA accumulates and partially inhibits PRMT5 in the cells, making tumor cells sensitive to PRMT5 inhibitors. Therefore, designing a small molecule that synergistically binds MTA and PRMT5, and specifically binds to the PRMT5- MTA complex, thereby inhibiting tumor cell growth, is an effective means to enhance the selectivity of PRMT5 inhibitors.

CONTENT OF THE PRESENT INVENTION

The present disclosure provides a compound of formula (III) or a pharmaceutically acceptable salt thereof,

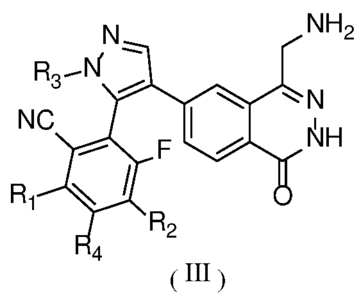

(III)

,

wherein

$R_1$ is selected from $C_{2-5}$ alkenyl, $C_{2-5}$ alkynyl, -O-$C_{3-5}$ cycloalkyl, -O-$C_{2-3}$ alkynyl, and $C_{3-5}$ cycloalkyl, and the $C_{2-5}$ alkenyl, $C_{2-5}$ alkynyl, -O-$C_{3-5}$ cycloalkyl, -O-$C_{2-3}$ alkynyl, and $C_{3-5}$ cycloalkyl are each independently and optionally substituted by 1, 2, or 3 $R_a$;

$R_2$ is selected from halogen and $C_{2-3}$ alkynyl, and the $C_{2-3}$ alkynyl is optionally substituted by 1, 2, or 3 halogens;

$R_3$ is selected from $CH_3$ and $CD_3$;
$R_4$ is selected from H and halogen;
each $R_a$ is independently selected from halogen and $C_{1-3}$ alkyl, and the $C_{1-3}$ alkyl is optionally substituted by 1, 2, or 3 halogens;
and at least one of the following conditions is satisfied:

(1) when $R_2$ is selected from halogen, $R_1$ is selected from $C_{2-5}$ alkenyl, $C_{2-5}$ alkynyl, - O-$C_{3-5}$ cycloalkyl, -O-$C_{2-3}$ alkynyl, and $C_{3-5}$ cycloalkyl; the $C_{2-5}$ alkenyl, $C_{2-5}$ alkynyl, and -O-$C_{2-3}$ alkynyl are optionally substituted by 1, 2, or 3 $R_a$, and the -O-$C_{3-5}$ cycloalkyl and $C_{3-5}$ cycloalkyl are substituted by 1, 2, or 3 $R_a$;
(2) when $R_2$ is selected from halogen, $R_3$ is selected from $CD_3$;
(3) when $R_2$ is selected from halogen, $R_4$ is selected from halogen.

In some embodiments of the present disclosure, the $R_a$ is independently selected from F, Cl, $CH_3$, $CHF_2$, and $CF_3$, and other variables are as defined in the present disclosure.

In some embodiments of the present disclosure, the $R_1$ is selected from $C_{2-3}$ alkenyl, $C_{2-3}$ alkynyl, -O-cyclopropyl, -O-$C_{2-3}$ alkynyl, and cyclopropyl; the $C_{2-3}$ alkenyl, $C_{2-3}$ alkynyl, -O-cyclopropyl, -O-$C_{2-3}$ alkynyl, and cyclopropyl are each independently and optionally substituted by 1, 2, or 3 $R_a$, and other variables are as defined in the present disclosure.

In some embodiments of the present disclosure, the $R_1$ is selected from

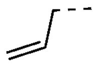,

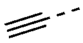, 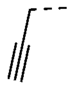, 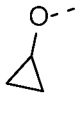, 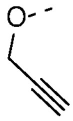, and 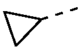;

the

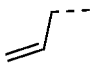, 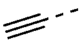, 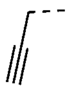, 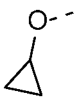, 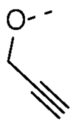,

and

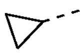

are each independently and optionally substituted by 1, 2, or 3 $R_a$, and other variables are as defined in the present disclosure.

In some embodiments of the present disclosure, the $R_1$ is selected from

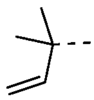,

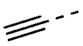, 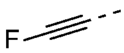, 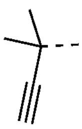, 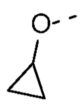, 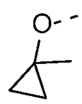, 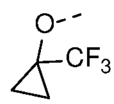, 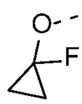, 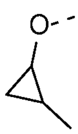, 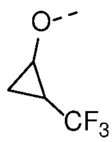, 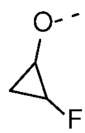,

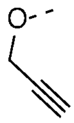, 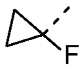, and 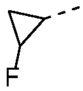,

and other variables are as defined in the present disclosure.

In some embodiments of the present disclosure, the $R_2$ is selected from F, Cl, and 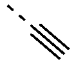; the 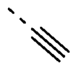 is optionally substituted by 1, 2, or 3 halogens, and other variables are as defined in the present disclosure.

In some embodiments of the present disclosure, the $R_2$ is selected from Cl,

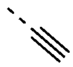,

and

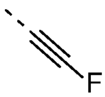,

and other variables are as defined in the present disclosure.

In some embodiments of the present disclosure, the $R_2$ is selected from Cl, the $R_1$ is selected from

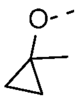, 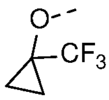, 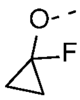, 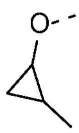, 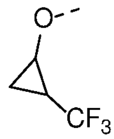, and 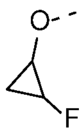,

and other variables are as defined in the present disclosure.

In some embodiments of the present disclosure, the $R_2$ is selected from Cl, the $R_3$ is selected from $CD_3$, and other variables are as defined in the present disclosure.

In some embodiments of the present disclosure, the $R_3$ is selected from $CD_3$, and other variables are as defined in the present disclosure.

In some embodiments of the present disclosure, the $R_4$ is selected from H and F, and other variables are as defined in the present disclosure.

In some embodiments of the present disclosure, the compound or the pharmaceutically acceptable salt thereof is selected from:

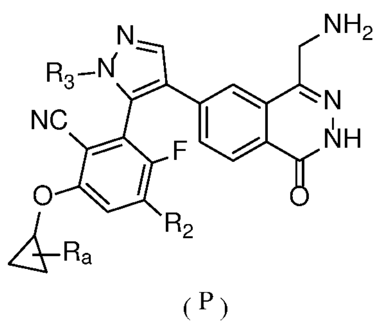

(P) ,

wherein $R_2$, $R_3$, and $R_a$ are as defined in the present disclosure.

In some embodiments of the present disclosure, the compound or the pharmaceutically acceptable salt thereof is selected from:

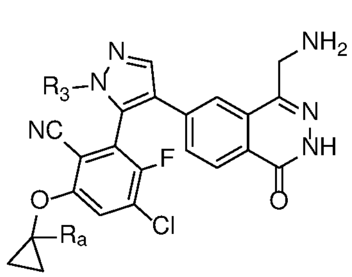
(P-1) ,
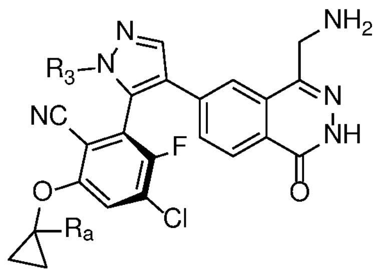
(P-1a) , and
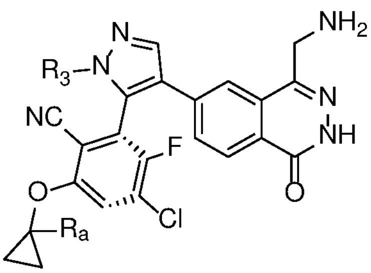
(P-1b) ,

wherein $R_3$ and $R_a$ are as defined in the present disclosure.

In some embodiments of the present disclosure, the compound or the pharmaceutically acceptable salt thereof is selected from:

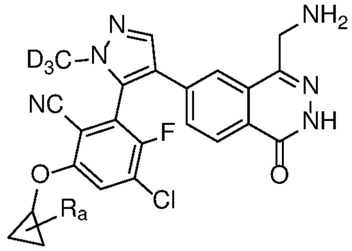
(P-2) ,
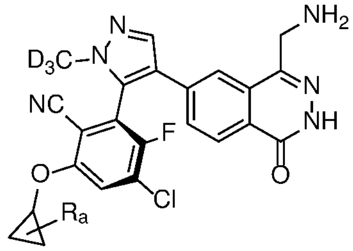
(P-2a) , and
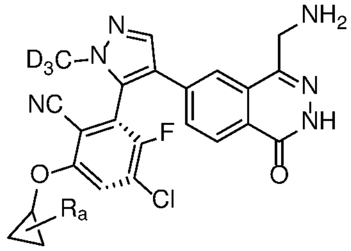
(P-2b) ,

wherein $R_a$ is as defined in the present disclosure.

In some embodiments of the present disclosure, the compound or the pharmaceutically acceptable salt thereof is selected from:

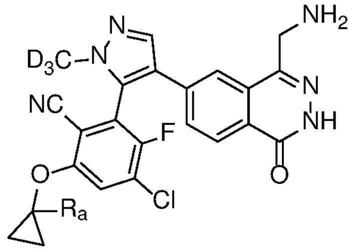
(P-3) ,
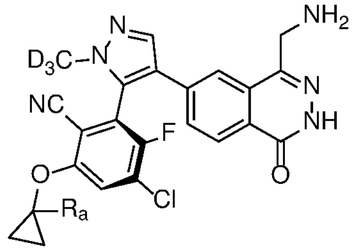
(P-3a) , and
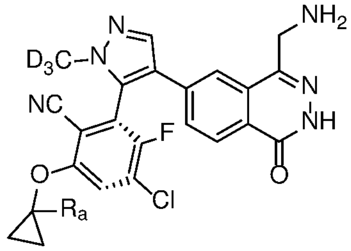
(P-3b) ,

wherein $R_a$ is as defined in the present disclosure.

The present disclosure further provides a compound of formula (III) or a pharmaceutically acceptable salt thereof,

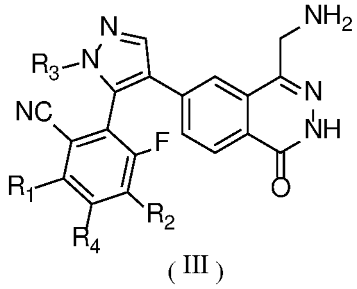
(III) ,

wherein

$R_1$ is selected from $C_{2-5}$ alkenyl, $C_{2-5}$ alkynyl, $-O-C_{3-5}$ cycloalkyl, $-O-C_{2-3}$ alkynyl, and $C_{3-5}$ cycloalkyl, and the $C_{2-5}$ alkenyl, $C_{2-5}$ alkynyl, $-O-C_{3-5}$ cycloalkyl, $-O-C_{2-3}$ alkynyl, and $C_{3-5}$ cycloalkyl are each independently and optionally substituted by 1, 2, or 3 $R_a$;
$R_2$ is selected from halogen and $C_{2-3}$ alkynyl, and the $C_{2-3}$ alkynyl is optionally substituted by 1, 2, or 3 halogens;
$R_3$ is selected from $CH_3$ and $CD_3$;
$R_4$ is selected from H and halogen;
each $R_a$ is independently selected from halogen and $C_{1-3}$ alkyl;
and at least one of the following conditions is satisfied:

1) when $R_2$ is selected from halogen, $R_1$ is selected from $C_{2-5}$ alkenyl, $C_{2-5}$ alkynyl, $-O-C_{3-5}$ cycloalkyl, $-O-C_{2-3}$ alkynyl, and $C_{3-5}$ cycloalkyl; the $C_{2-5}$ alkenyl, $C_{2-5}$ alkynyl, and $-O-C_{2-3}$ alkynyl are optionally substituted by 1, 2, or 3 $R_a$, and the $-O-C_{3-5}$ cycloalkyl and $C_{3-5}$ cycloalkyl are substituted by 1, 2, or 3 $R_a$; or
2) when $R_2$ is selected from halogen, $R_3$ is selected from $CD_3$;
3) when $R_2$ is selected from halogen, $R_4$ is selected from halogen.

In some embodiments of the present disclosure, the $R_a$ is independently selected from F, Cl, $CH_3$, $CHF_2$, and $CF_3$, and other variables are as defined in the present disclosure.
In some embodiments of the present disclosure, the $R_1$ is selected from $C_{2-3}$ alkenyl, $C_{2-3}$ alkynyl, -O-cyclopropyl, $-O-C_{2-3}$ alkynyl, and cyclopropyl; the $C_{2-3}$ alkenyl, $C_{2-3}$ alkynyl, -O-cyclopropyl, $-O-C_{2-3}$ alkynyl, and cyclopropyl are each independently and optionally substituted by 1, 2, or 3 $R_a$, and other variables are as defined in the present disclosure.
In some embodiments of the present disclosure, the $R_1$ is selected from

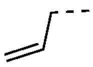,

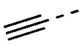, 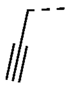, 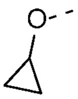, 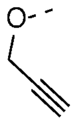, and 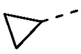; the 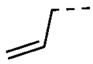, 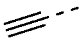, 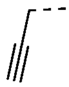, 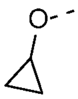, 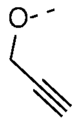,

and

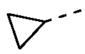

are each independently and optionally substituted by 1, 2, or 3 $R_a$, and other variables are as defined in the present disclosure.
In some embodiments of the present disclosure, the $R_1$ is selected from

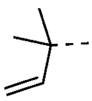,

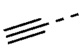, 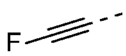, 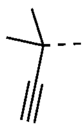, 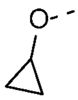, 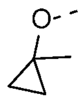, 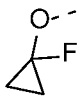, 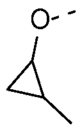, 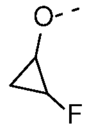, 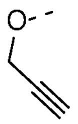, 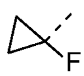,

and

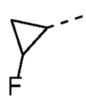

, and other variables are as defined in the present disclosure.

In some embodiments of the present disclosure, the $R_2$ is selected from F, Cl, and

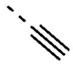;

the

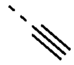

is optionally substituted by 1, 2, or 3 halogens, and other variables are as defined in the present disclosure.

In some embodiments of the present disclosure, the $R_2$ is selected from Cl,

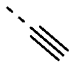,

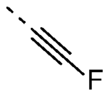,

and other variables are as defined in the present disclosure.

In some embodiments of the present disclosure, the $R_3$ is selected from $CD_3$, and other variables are as defined in the present disclosure.

In some embodiments of the present disclosure, the $R_4$ is selected from H and F, and other variables are as defined in the present disclosure.

The present disclosure further provides a compound of formula (II) or a pharmaceutically acceptable salt thereof,

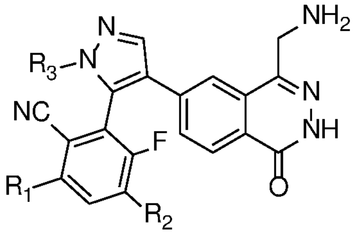

(II)

,

wherein

$R_1$ is selected from $C_{2-5}$ alkenyl, $C_{2-5}$ alkynyl, -O-$C_{3-5}$ cycloalkyl, -O-$C_{2-3}$ alkynyl, and $C_{3-5}$ cycloalkyl, and the $C_{2-5}$ alkenyl, $C_{2-5}$ alkynyl, -O-$C_{3-5}$ cycloalkyl, -O-$C_{2-3}$ alkynyl, and $C_{3-5}$ cycloalkyl are each independently and optionally substituted by 1, 2, or 3 $R_a$;

$R_2$ is selected from halogen and $C_{2-3}$ alkynyl, and the $C_{2-3}$ alkynyl is optionally substituted by 1, 2, or 3 halogens;

$R_3$ is selected from $CH_3$ and $CD_3$;

each $R_a$ is independently selected from halogen and $C_{1-3}$ alkyl;

provided that

1) when $R_2$ is selected from halogen, $R_1$ is selected from $C_{2-5}$ alkenyl, $C_{2-5}$ alkynyl, - O-$C_{3-5}$ cycloalkyl, -O-$C_{2-3}$ alkynyl, and $C_{3-5}$ cycloalkyl; the $C_{2-5}$ alkenyl, $C_{2-5}$ alkynyl, and -O-$C_{2-3}$ alkynyl are optionally substituted by 1, 2, or 3 $R_a$, and the -O-$C_{3-5}$ cycloalkyl and $C_{3-5}$ cycloalkyl are substituted by 1, 2, or 3 $R_a$; or

2) when $R_2$ is selected from halogen, $R_3$ is selected from $CD_3$.

In some embodiments of the present disclosure, the $R_a$ is independently selected from F, Cl, $CH_3$, $CHF_2$, and $CF_3$, and other variables are as defined in the present disclosure.

In some embodiments of the present disclosure, the $R_1$ is selected from $C_{2-3}$ alkenyl, $C_{2-3}$ alkynyl, -O-cyclopropyl, -O-$C_{2-3}$ alkynyl, and cyclopropyl; the $C_{2-3}$ alkenyl, $C_{2-3}$ alkynyl, -O-cyclopropyl, -O-$C_{2-3}$ alkynyl, and cyclopropyl are each independently and optionally substituted by 1, 2, or 3 $R_a$, and other variables are as defined in the present disclosure.

In some embodiments of the present disclosure, the $R_1$ is selected from

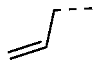,

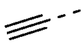, 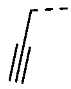, 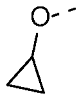, 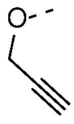, and 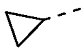;

the

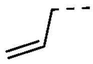, 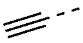, 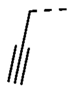, 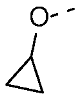, 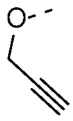,

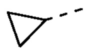

are each independently and optionally substituted by 1, 2, or 3 $R_a$, and other variables are as defined in the present disclosure.

In some embodiments of the present disclosure, the $R_1$ is selected from

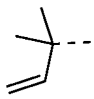,

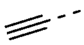, 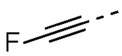, 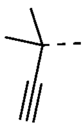, 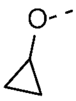, 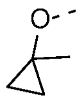, 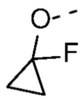, 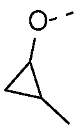, 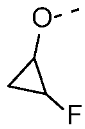, 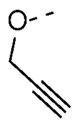, 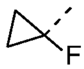,

and

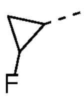,

and other variables are as defined in the present disclosure.

In some embodiments of the present disclosure, the $R_2$ is selected from F, Cl, and

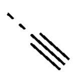;

the

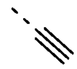

is optionally substituted by 1, 2, or 3 halogens, and other variables are as defined in the present disclosure.

In some embodiments of the present disclosure, the $R_2$ is selected from Cl,

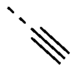, ;

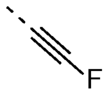,

and other variables are as defined in the present disclosure.

The present disclosure further provides a compound of formula (I) or a pharmaceutically acceptable salt thereof,

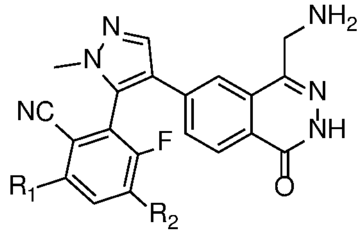

(I)

wherein

$R_1$ is selected from $C_{2-5}$ alkenyl, $C_{2-5}$ alkynyl, -O-$C_{3-5}$ cycloalkyl, -O-$C_{2-3}$ alkynyl, and $C_{3-5}$ cycloalkyl, and the $C_{2-5}$ alkenyl, $C_{2-5}$ alkynyl, -O-$C_{3-5}$ cycloalkyl, -O-$C_{2-3}$ alkynyl, and $C_{3-5}$ cycloalkyl are each independently and optionally substituted by 1, 2, or 3 $R_a$;
$R_2$ is selected from halogen and $C_{2-3}$ alkynyl, and the $C_{2-3}$ alkynyl is optionally substituted by 1, 2, or 3 halogens;
each $R_a$ is independently selected from halogen and $C_{1-3}$ alkyl;
provided that
when $R_2$ is selected from halogen, $R_1$ is selected from $C_{2-5}$ alkenyl, $C_{2-5}$ alkynyl, -O-$C_{3-5}$ cycloalkyl, -O-$C_{2-3}$ alkynyl, and $C_{3-5}$ cycloalkyl; the $C_{2-5}$ alkenyl, $C_{2-5}$ alkynyl, and -O-$C_{2-3}$ alkynyl are optionally substituted by 1, 2, or 3 $R_a$, and the -O-$C_{3-5}$ cycloalkyl and $C_{3-5}$ cycloalkyl are substituted by 1, 2, or 3 $R_a$.

In some embodiments of the present disclosure, the $R_a$ is independently selected from F, Cl, $CH_3$, $CHF_2$, and $CF_3$, and other variables are as defined in the present disclosure.

In some embodiments of the present disclosure, the $R_1$ is selected from $C_{2-3}$ alkenyl, $C_{2-3}$ alkynyl, -O-cyclopropyl, -O-$C_{2-3}$ alkynyl, and cyclopropyl; the $C_{2-3}$ alkenyl, $C_{2-3}$ alkynyl, -O-cyclopropyl, -O-$C_{2-3}$ alkynyl, and cyclopropyl are each independently and optionally substituted by 1, 2, or 3 $R_a$, and other variables are as defined in the present disclosure.

In some embodiments of the present disclosure, the $R_1$ is selected from

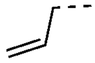,

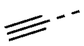, 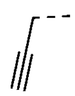, 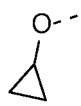, 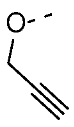, and 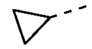; the 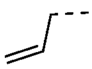, 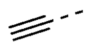, 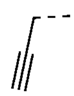, 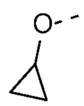, 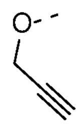,

and

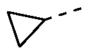

are each independently and optionally substituted by 1, 2, or 3 $R_a$, and other variables are as defined in the present disclosure.

In some embodiments of the present disclosure, the $R_1$ is selected from

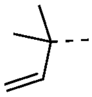,

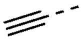, 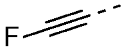, 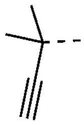, 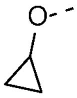, 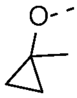, 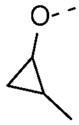, 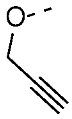, 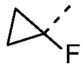, and 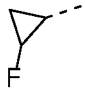,

and other variables are as defined in the present disclosure.

In some embodiments of the present disclosure, the $R_2$ is selected from F, Cl, and

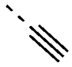;

the

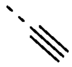

is optionally substituted by 1, 2, or 3 halogens, and other variables are as defined in the present disclosure.

In some embodiments of the present disclosure, the $R_2$ is selected from Cl,

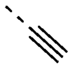,

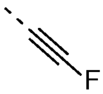,

and other variables are as defined in the present disclosure.

There are still some embodiments of the present disclosure which are obtained by any combination of the above variables.

The present disclosure further provides a compound of the following formula or a pharmaceutically acceptable salt thereof,

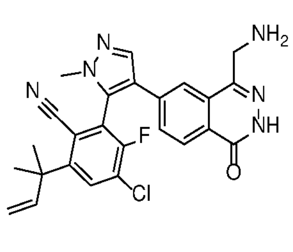 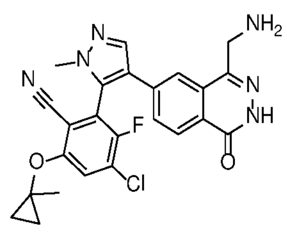 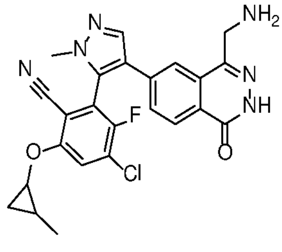 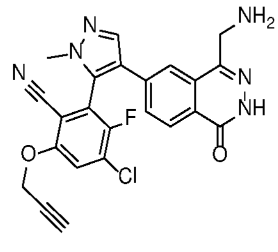

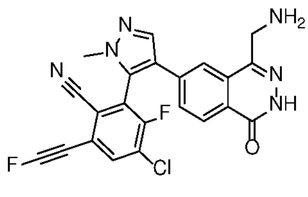 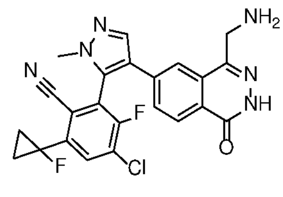 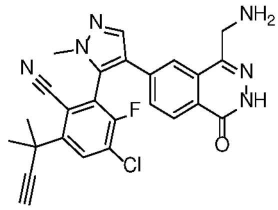 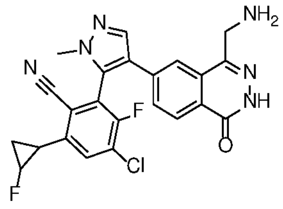

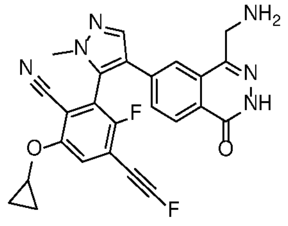 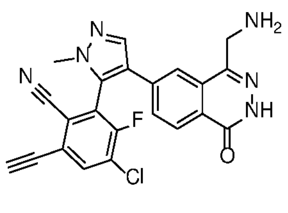 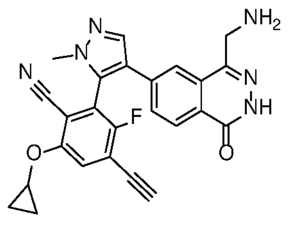 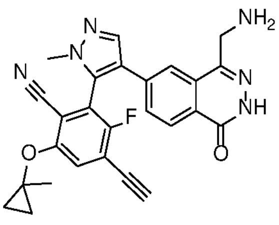

and

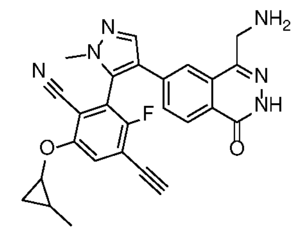 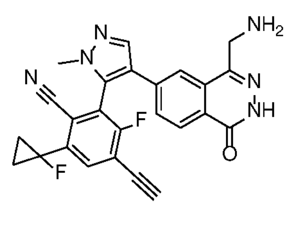 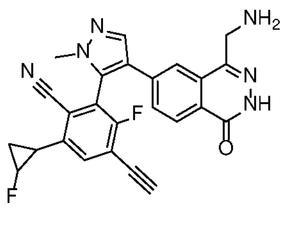 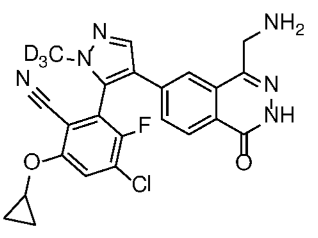

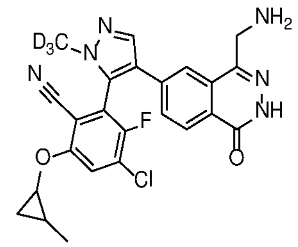 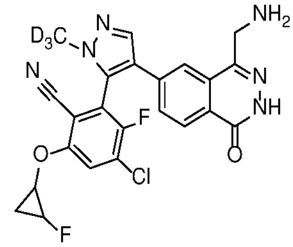 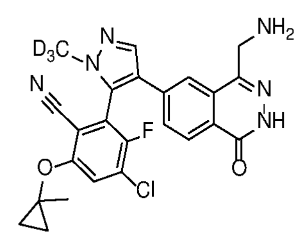 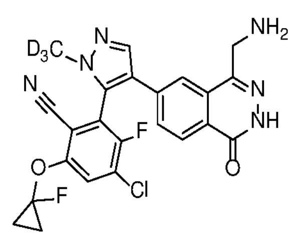

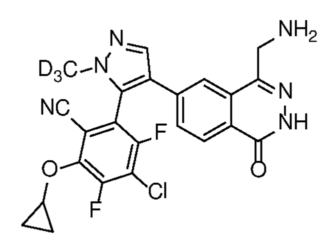 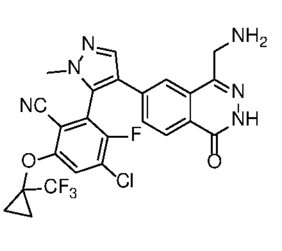 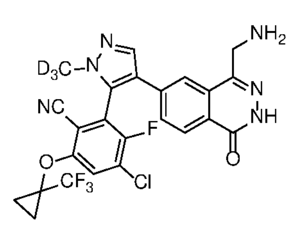 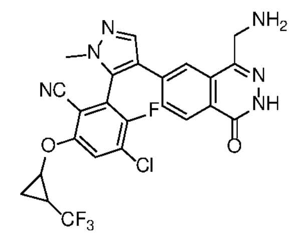

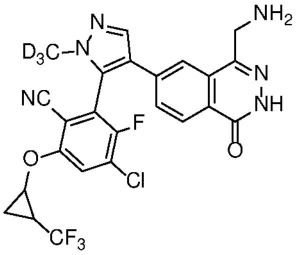

.

In some embodiments of the present disclosure, the compound or the pharmaceutically acceptable salt thereof is selected from:

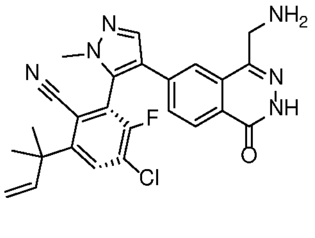 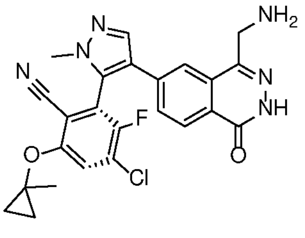 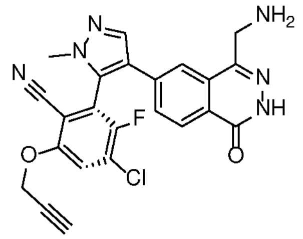

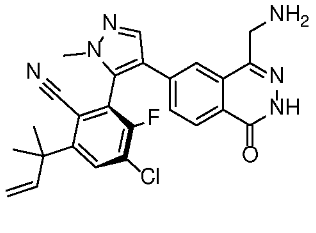 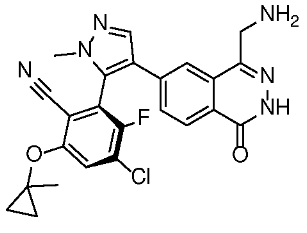 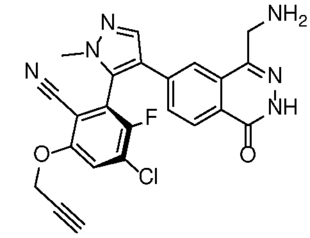

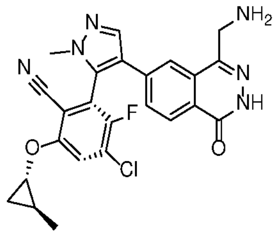 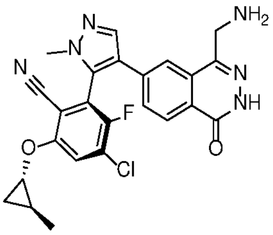 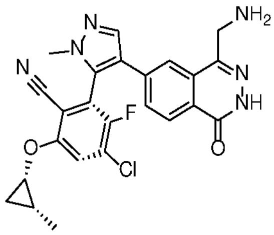 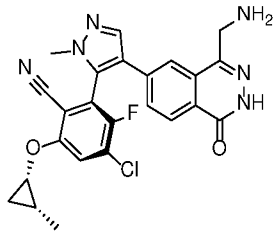

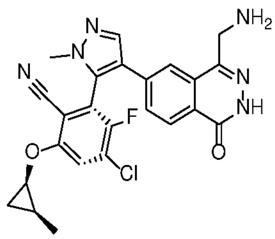 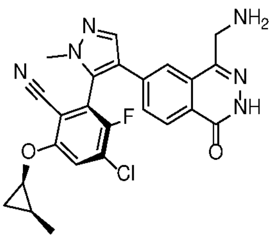 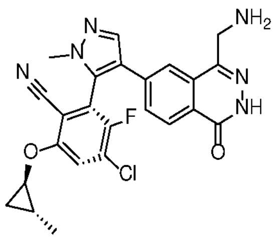 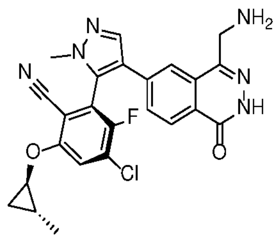

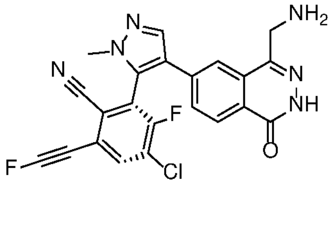 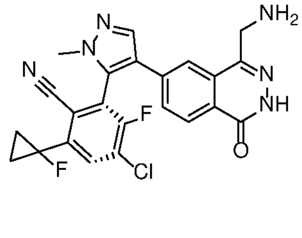 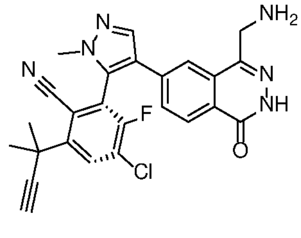

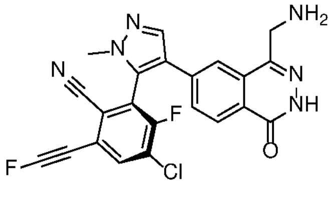
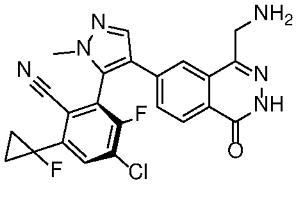
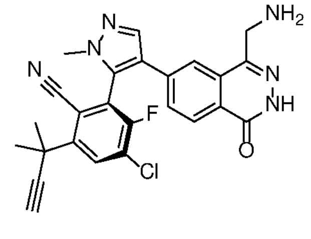

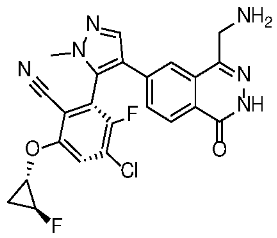
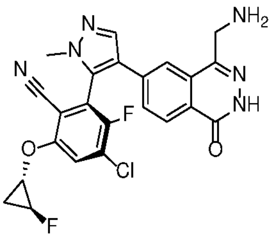
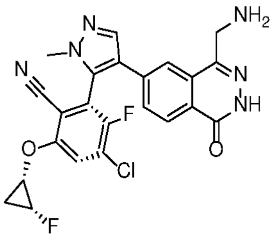
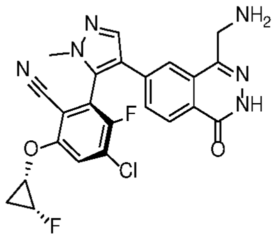

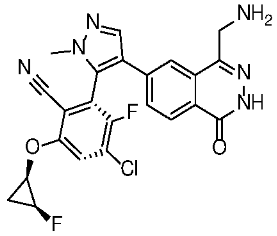
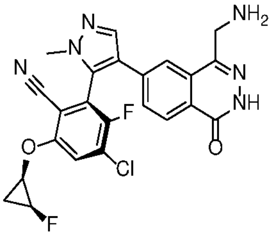
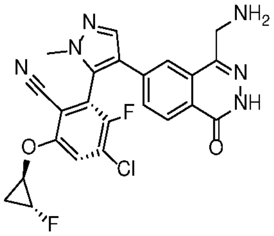
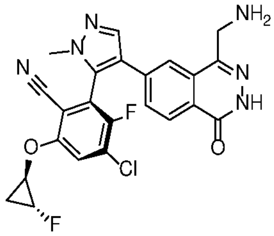

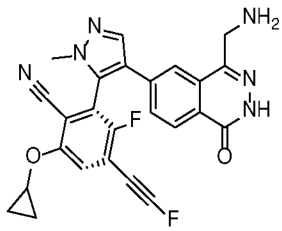
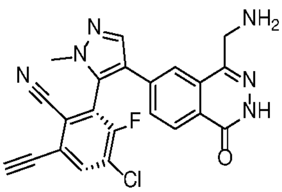
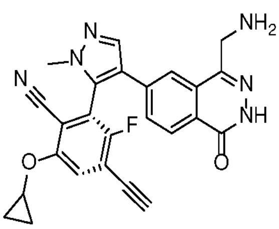
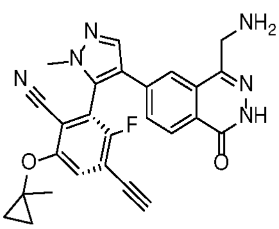

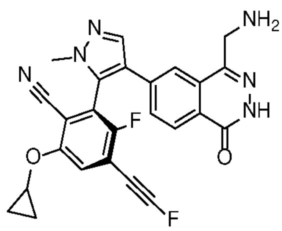
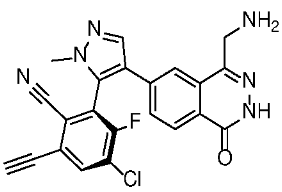
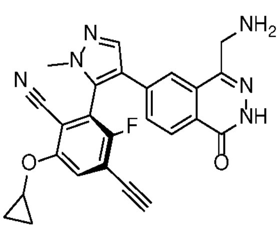
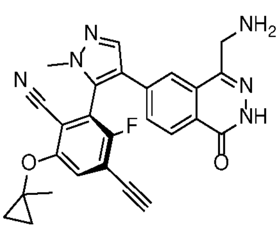

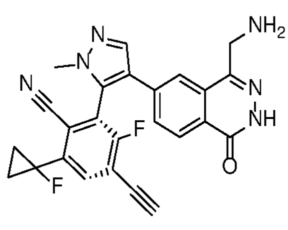
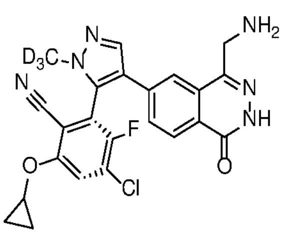
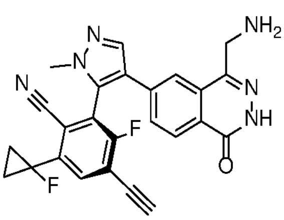
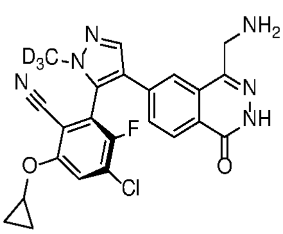

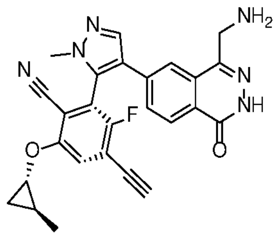
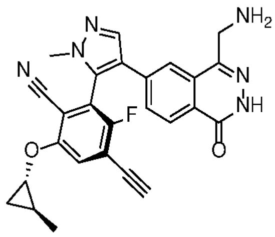
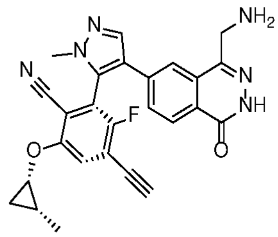
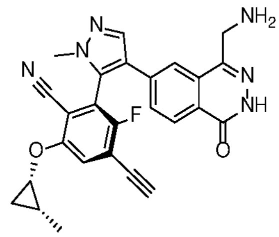
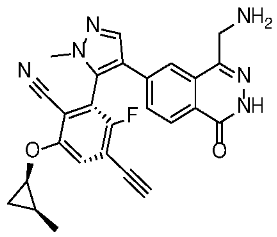
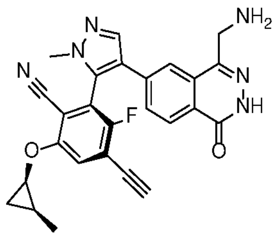
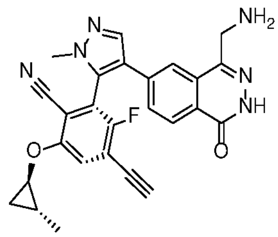
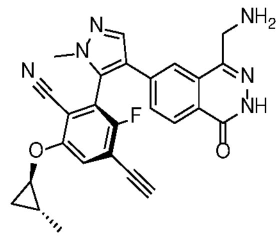
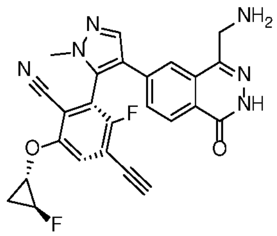
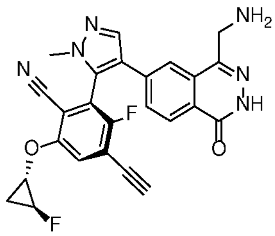
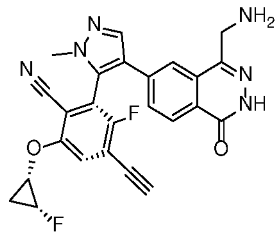
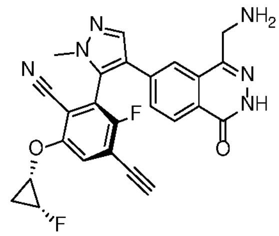
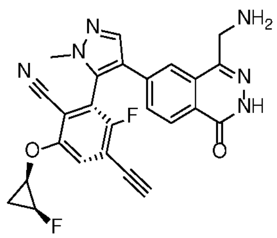
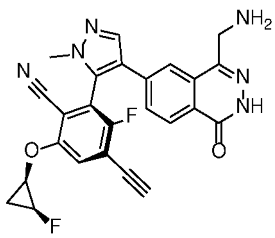
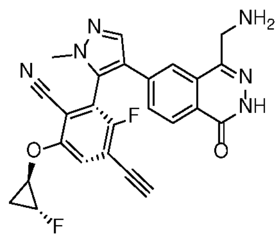
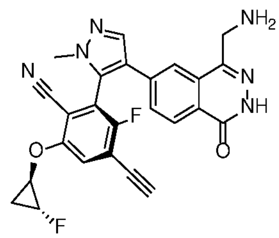
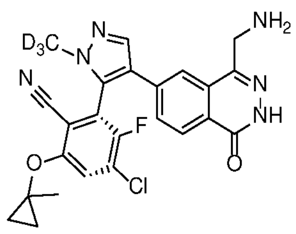
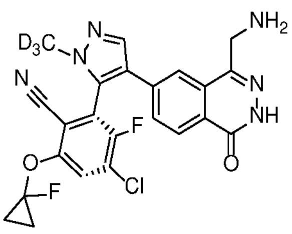
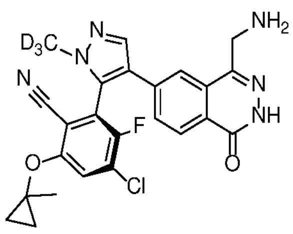
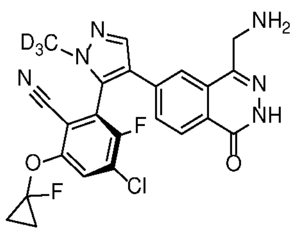
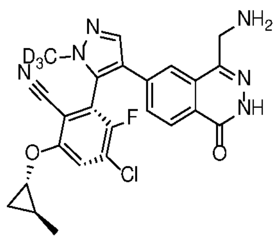
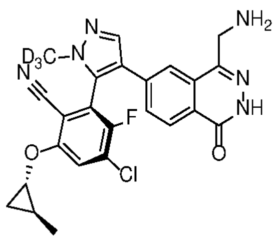
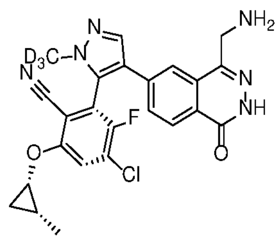
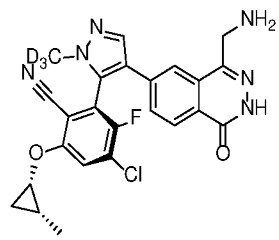

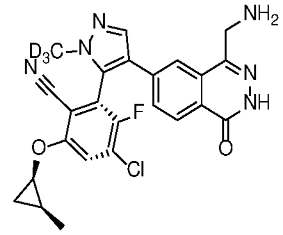
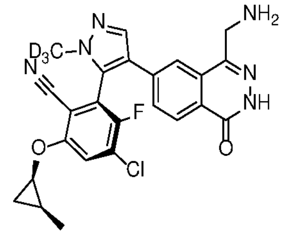
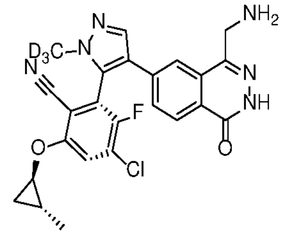
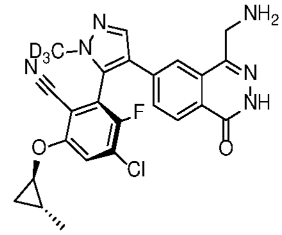

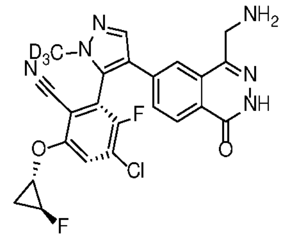
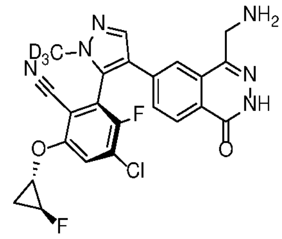
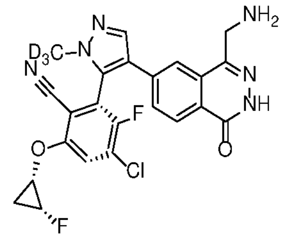
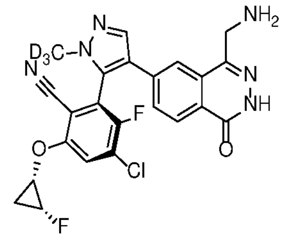

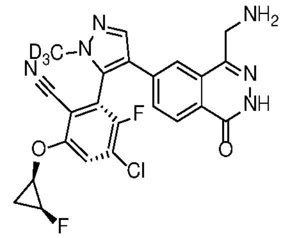
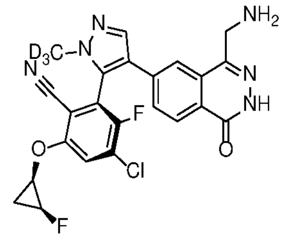
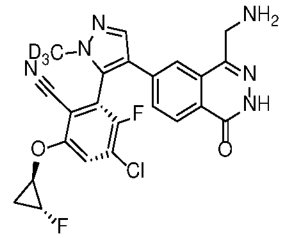
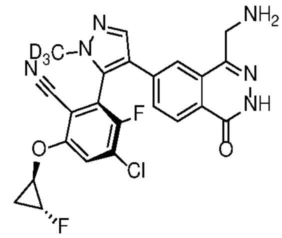

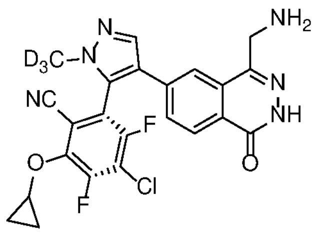
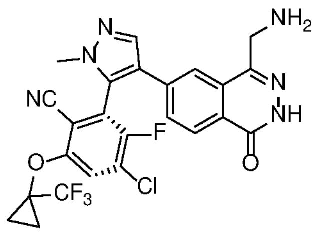
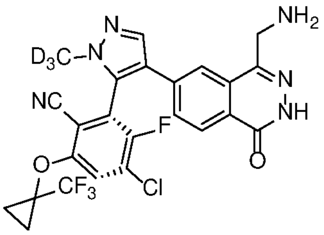

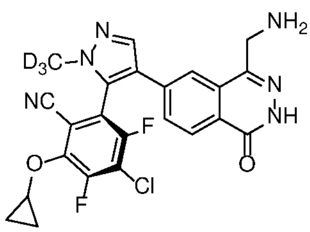
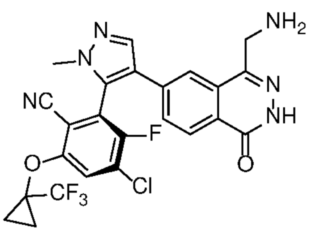
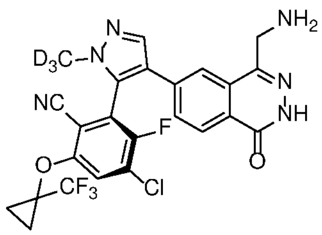

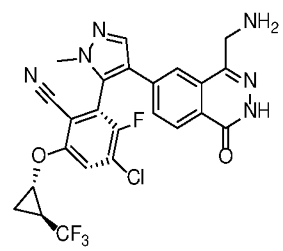
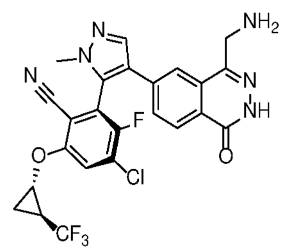
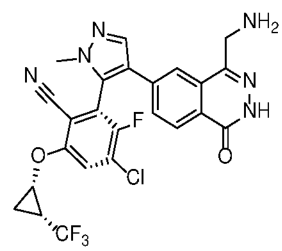
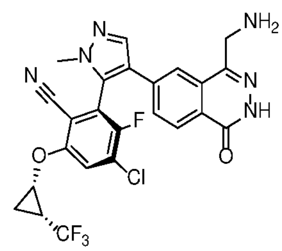

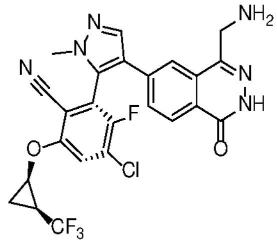
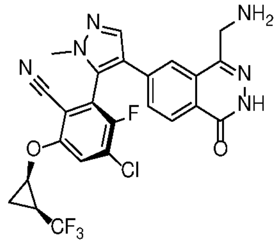
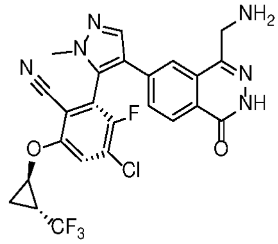
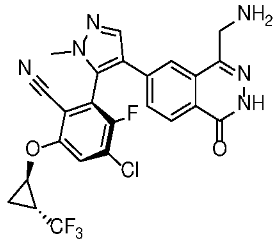
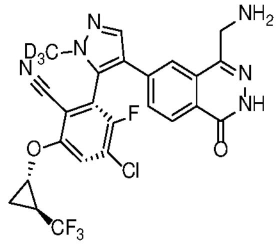
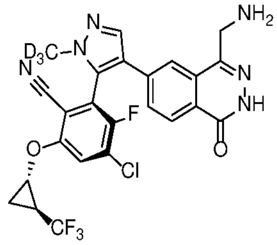
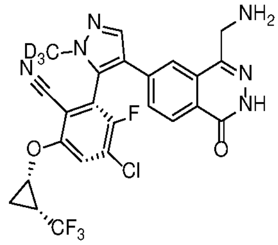
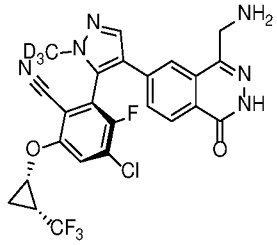
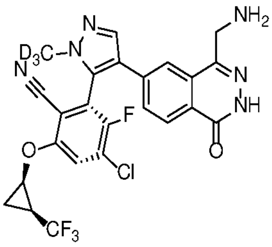
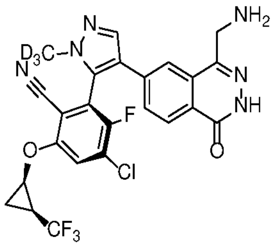
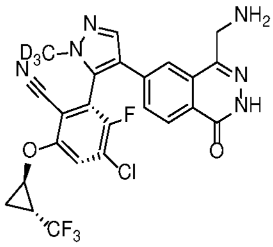
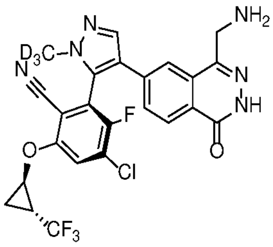
.

The present disclosure further provides a use of the compound or the pharmaceutically acceptable salt thereof in a medicament related to PRMT5 inhibitors.

The present disclosure further provides a use of the compound or the pharmaceutically acceptable salt thereof in a medicament related to PRMT5·MTA complex inhibitors.

The present disclosure further provides the following synthesis methods:

Method 1

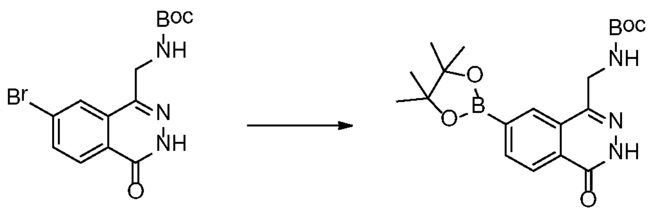

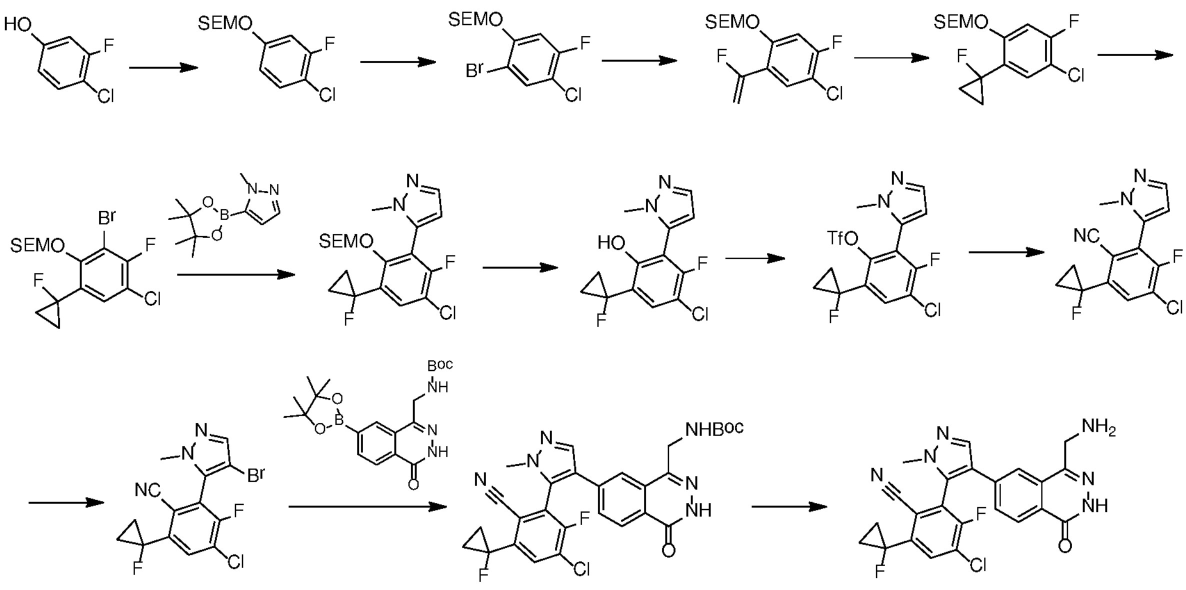

Method 2

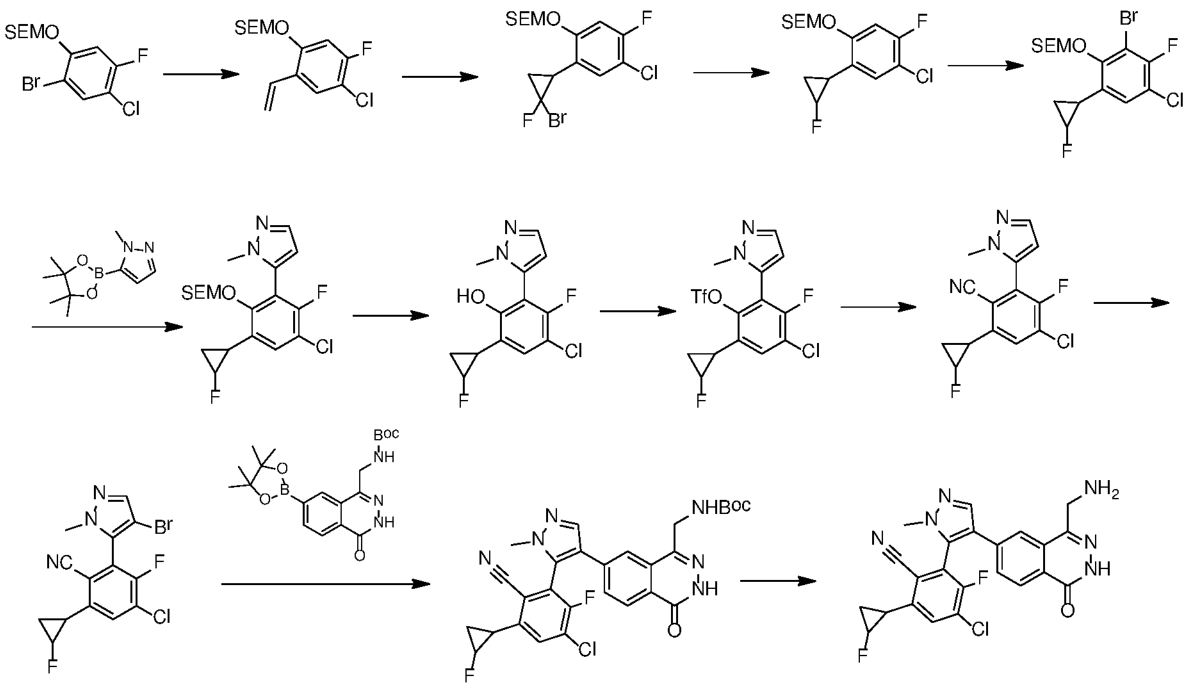

Method 3

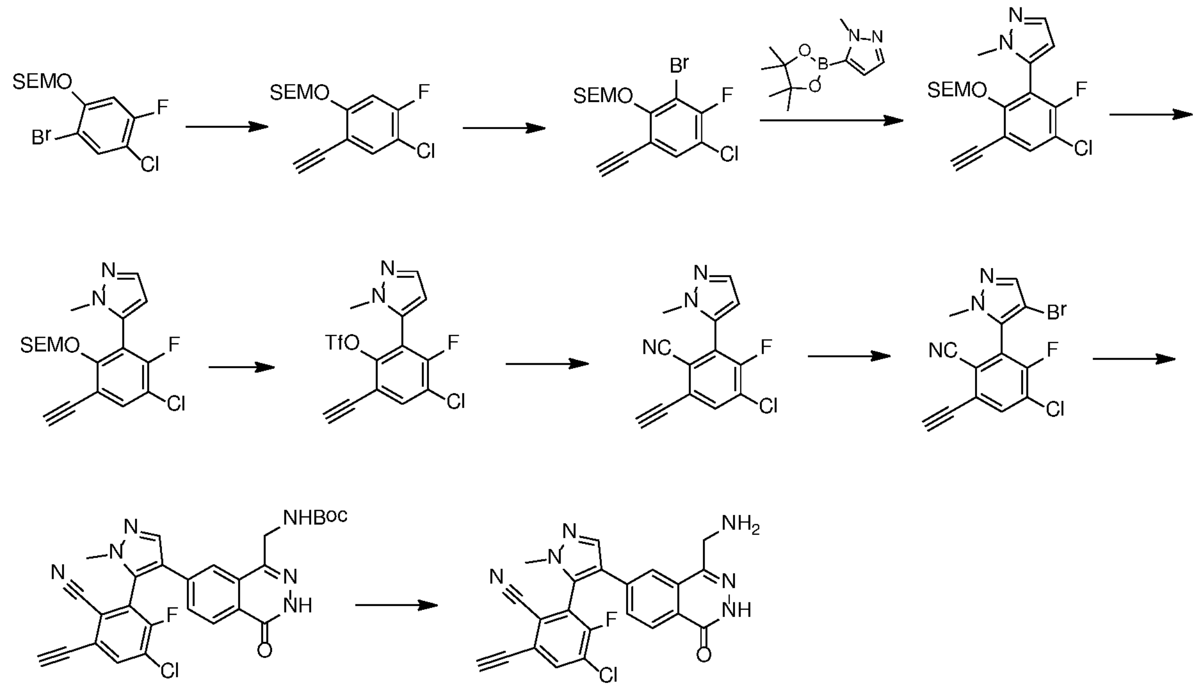

## Method 4

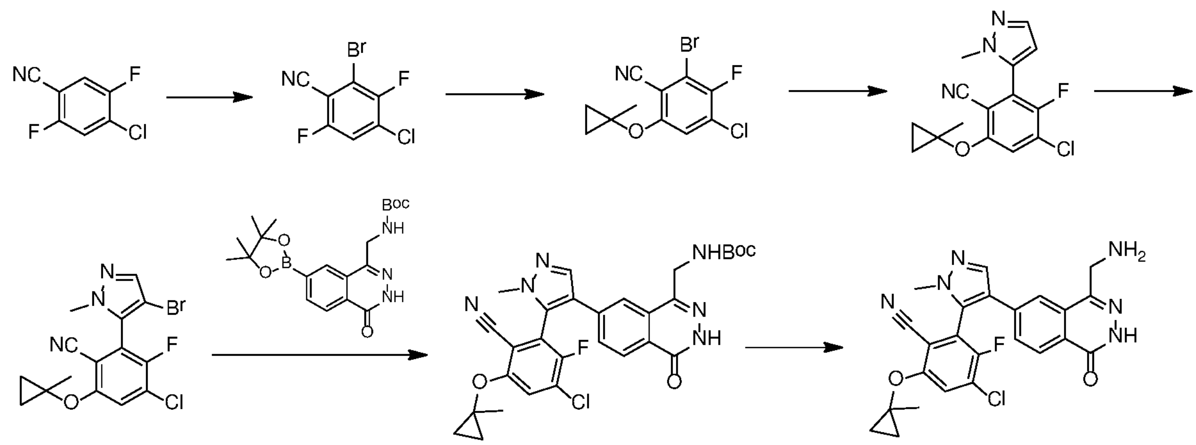

## Method 5

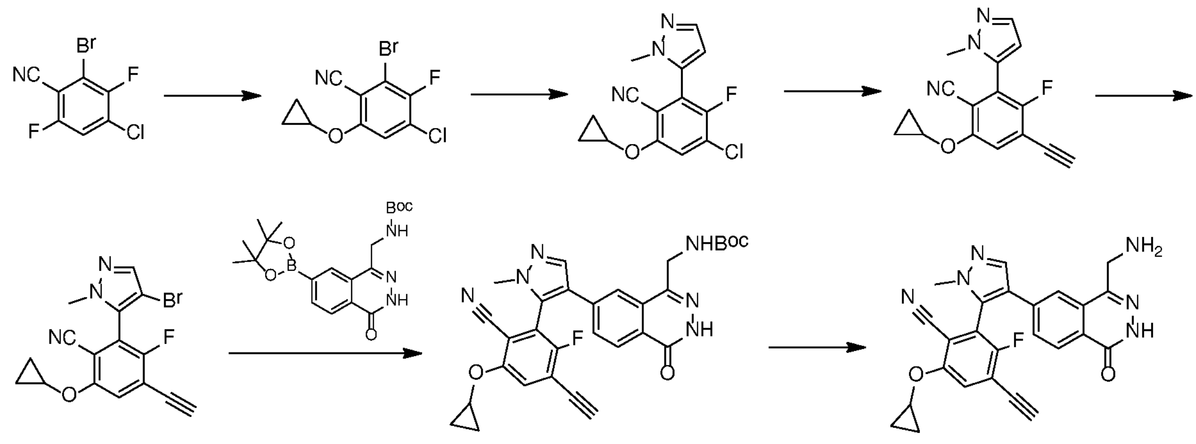

**[0094]** Method 6

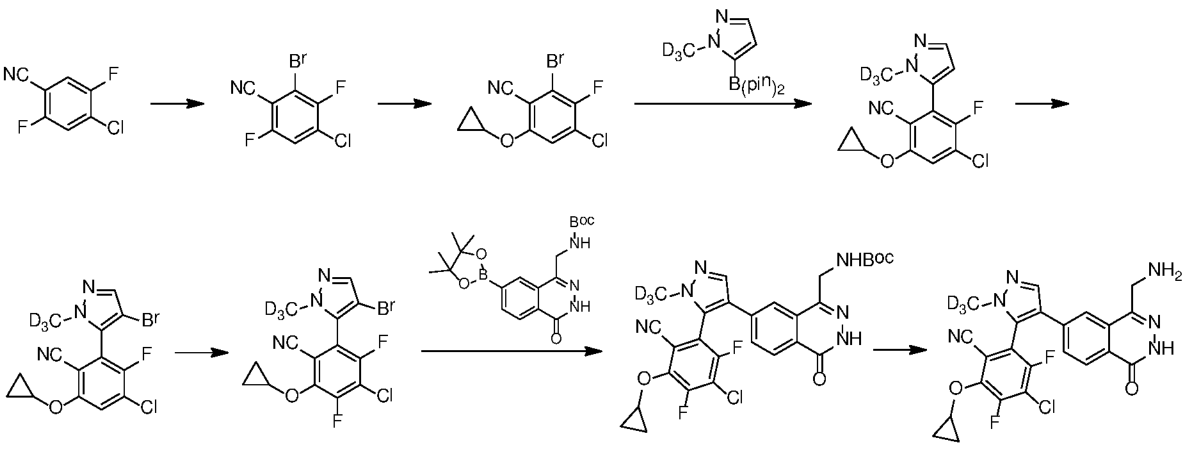

The present disclosure further provides the following test methods:

Test method 1: PRMT5 enzyme inhibitory activity assay

Experimental purpose: To evaluate the inhibitory effect of the compound on PRMT5 enzyme activity.

Experimental material: PRMT5 enzyme.

Experimental operation: The test compound is added at a certain concentration to a white clear-bottom 384-well plate using LABCYTE Echo 550. PRMT5 enzyme is added to the wells. A vehicle control (DMSO without the compound) and a blank control (DMSO without PRMT5 enzyme) are established. The plate is incubated at 25°C for 30 minutes. Subsequently, the substrate is added and the mixture is reacted at 25°C for 90 minutes. After 90 minutes, the detection reagent was added to the 384-well plate using the PerkinElmer LANCE Ultra TR-FRET standard method. The detection reagent is allowed to interact with the enzyme-substrate reaction product, and the mixture is reacted at 25°C for 1 hour. The signal is detected on a PerkinElmer EnVision 2105 Multimode Plate Reader. The raw data are used to calculate the inhibition rate of the test compound using the following formula:

$$\text{Inhibition rate\%} = \frac{\text{RLU vehicle control} - \text{RLU compound}}{\text{RLU vehicle control} - \text{RLU blank control}} \times 100\%$$

The inhibition rates are imported into Excel to calculate the inhibition rates of compounds at different concentrations, and then XLFit software is used to plot inhibition curves and calculate relevant parameters, including minimum inhibition rate, maximum inhibition rate, and $IC_{50}$.

Test method 2: Pharmacokinetic evaluation in mice

Experimental purpose: To determine the plasma concentration of the compound at different time points following intravenous and oral administration using LC/MS/MS, with male C57BL/6 mice as test animals. The pharmacokinetic behavior of the compounds in mice is studied to evaluate their pharmacokinetic characteristics.

Experimental operation: An appropriate amount of sample is weighed and dissolved in sterile normal saline to form a clear solution. Two healthy male C57BL/6 mice, purchased from Beijing Vital River Laboratory Animal Technology Co., Ltd., are maintained on a normal diet. After administration, 25 μL of blood is collected from the animal at 0.083, 0.25, 0.5, 1, 2, 4, 8, 12, and 24 hours respectively and placed in a commercial anticoagulant tube pre-loaded with EDTA-K2. The tube is centrifuged for 10 minutes to separate the plasma, which is stored at -60°C. The plasma samples are analyzed for the content of the corresponding compounds using LC/MS/MS.

**Technical effect**

The compound of the present disclosure has an excellent binding effect on PRMT5, can effectively inhibit PRMT5, and has a significant inhibitory effect on the PRMT5·MTA complex. It has pronounced inhibitory activity against

MTAP-deficient tumor cells while exhibiting a weak inhibitory effect on wild-type tumor cells, thereby showing good selectivity. The compound possesses favorable liver microsomal stability and favorable *in vivo* pharmacokinetic properties with minimal species differences. In a human large cell lung cancer LU99 subcutaneous xenograft tumor model, the compound shows a significant tumor inhibitory effect, and the mice maintain stable body weight post-administration.

## Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. When the compound of the present disclosure contains a relatively basic functional group, an acid addition salt can be obtained by contacting such compounds with a sufficient amount of acid in a pure solution or a suitable inert solvent. Certain specific compounds of the present disclosure contain both basic and acidic functional groups, and thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*L*)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the absolute configuration of a stereogenic center is represented by a wedged solid bond ( 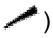 ) and a wedged dashed bond ( 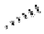 ), and the relative configuration of a stereogenic center is represented by a straight solid bond ( 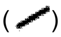 ) and a straight dashed bond ( 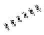 ), a wave line ( 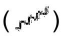 ) is used to represent a wedged solid bond ( 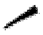 ) or a wedged dashed bond ( 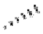 ), or the wave line ( 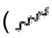 ) is used to represent a straight solid bond ( 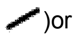 )or a straight dashed bond ( 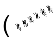 ).

Certain compounds of the present disclosure may exist as atropisomers, which are conformational isomers occurring when rotation around a single bond in the molecule is hindered or significantly slowed due to steric interactions with other parts of the molecule. The compounds disclosed in the present disclosure include all atropisomers, which may be pure individual atropisomers, enriched in one of the atropisomers, or non-specific mixtures thereof. If the rotational potential energy around a single bond is high enough and the interconversion between conformations is slow enough, isomers are allowed to be separated. For example,

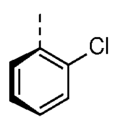

(or

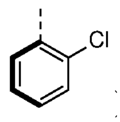

) and

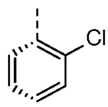

(or

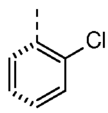

) are a pair of atropisomers, wherein

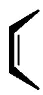

or

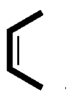

on the phenyl group indicates that the stereo orientation on this side is outward, while

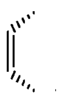

or

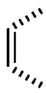

indicates that the stereo orientation on this side is inward.

The term "optional" or "optionally" means that the subsequent event or condition may occur but not requisite, that the term includes the instance in which the event or condition occurs and the instance in which the event or condition does not occur.

The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the

definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond

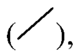,

a straight dashed bond

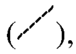,

or a wavy line

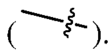.

For example, the straight solid bond in $-OCH_3$ means that it is linked to other groups through the oxygen atom in the group; the straight dashed bond in

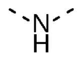

means that it is linked to other groups through the two ends of the nitrogen atom in the group; the wave lines in

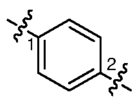

means that the phenyl group is linked to other groups through carbon atoms at position 1 and position 2;

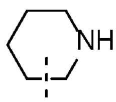

means that it can be linked to other groups through any linkable sites on the piperidinyl by one chemical bond, including at least four types of linkage, including

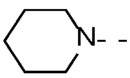, 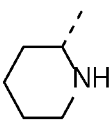, 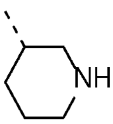, 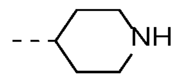.

Even though the H atom is drawn on the -N-,

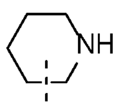

still includes the linkage of

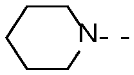

,

merely when one chemical bond was connected, the H of this site will be reduced by one to the corresponding monovalent piperidinyl.

Unless otherwise specified, the term "halogen element" or "halogen" by itself or as part of another substituent refers to fluorine, chlorine, bromine, or iodine atom.

Unless otherwise specified, "$C_{2-5}$ alkenyl" is used to mean a linear or branched hydrocarbon group consisting of 2 to 5 carbon atoms containing at least one carbon-carbon double bond, wherein the carbon-carbon double bond can be located at any position within the group. The $C_{2-5}$ alkenyl includes $C_{2-3}$, $C_5$, $C_4$, $C_3$, $C_2$ alkenyl, *etc.*; the $C_{2-5}$ alkenyl can be monovalent, divalent, or multivalent. Examples of $C_{2-4}$ alkenyl include, but are not limited to, vinyl, propenyl, butenyl, butadienyl, *etc.*

Unless otherwise specified, "$C_{2-3}$ alkenyl" is used to mean a linear or branched hydrocarbon group consisting of 2 to 3 carbon atoms containing at least one carbon-carbon double bond, wherein the carbon-carbon double bond can be located at any position within the group. The $C_{2-3}$ alkenyl includes $C_3$ and $C_2$ alkenyl; the $C_{2-3}$ alkenyl can be monovalent, divalent, or multivalent. Examples of $C_{2-3}$ alkenyl include, but are not limited to, vinyl, propenyl, *etc.*

Unless otherwise specified, "$C_{2-5}$ alkynyl" is used to mean a linear or branched hydrocarbon group consisting of 2 to 5 carbon atoms containing at least one carbon-carbon triple bond, wherein the carbon-carbon triple bond can be located at any position within the group. The $C_{2-4}$ alkynyl includes $C_{2-3}$, $C_5$, $C_4$, $C_3$, $C_2$ alkynyl, *etc.* It can be monovalent, divalent, or multivalent. Examples of $C_{2-5}$ alkynyl include, but are not limited to, ethynyl, propynyl, butynyl, *etc.*

Unless otherwise specified, "$C_{2-3}$ alkynyl" is used to mean a linear or branched hydrocarbon group consisting of 2 to 3 carbon atoms containing at least one carbon-carbon triple bond, wherein the carbon-carbon triple bond can be located at any position within the group. It can be monovalent, divalent, or multivalent. The $C_{2-3}$ alkynyl includes $C_3$ and $C_2$ alkynyl. Examples of $C_{2-3}$ alkynyl include, but are not limited to, ethynyl, propynyl, *etc.*

Unless otherwise specified, "$C_{3-5}$ cycloalkyl" refers to a saturated cyclic hydrocarbon group consisting of 3 to 5 carbon atoms, which is a monocyclic system, and the $C_{3-5}$ cycloalkyl includes $C_{3-4}$, $C_{4-5}$ cycloalkyl, *etc.;* it can be monovalent, divalent, or multivalent. Examples of $C_{3-5}$ cycloalkyl include, but are not limited to, cyclopropyl, cyclobutyl, cyclopentyl, *etc.*

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

Abbreviations used in the present disclosure are as follows: solutol refers to polyethylene glycol-15-hydroxystearate; MC (4000 cps) refers to methyl cellulose (viscosity: 4000 cps); tolbutamide refers to Tolbutamide; labetalol refers to Labetalol.

The solvents used in the present disclosure are commercially available.

The compounds of the present disclosure are named according to the conventional naming principles in the art or by ChemDraw® software, and the commercially available compounds use the supplier catalog names.

BRIEF DESCRIPTION OF THE DRAWINGS

Fig. 1 shows the predicted binding mode of compound A with PRMT5.

Fig. 2 shows the predicted binding mode of compound B (conformation 1) with PRMT5.

Fig. 3 shows the predicted binding mode of compound B (conformation 2) with PRMT5.

Fig. 4 shows the predicted binding mode of compound B (conformation 3) with PRMT5.

Fig. 5 shows the predicted binding mode of compound B (conformation 4) with PRMT5.

Fig. 6 shows the predicted binding mode of compound C (conformation 1) with PRMT5.

Fig. 7 shows the predicted binding mode of compound C (conformation 2) with PRMT5.

Fig. 8 shows the predicted binding mode of compound C (conformation 3) with PRMT5.

Fig. 9 shows the predicted binding mode of compound C (conformation 4) with PRMT5.

Fig. 10 shows the predicted binding mode of compound D with PRMT5.

Fig. 11 shows the predicted binding mode of compound F with PRMT5.

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

**Computing example 1**

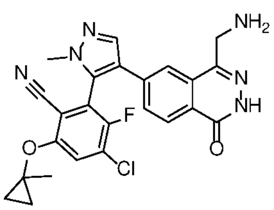

Compound A

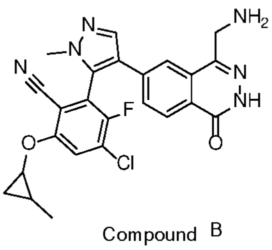

Compound B

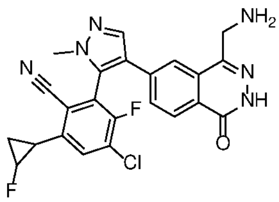

Compound C

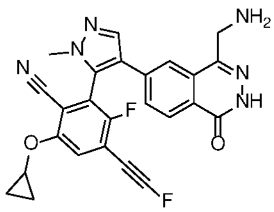

Compound D

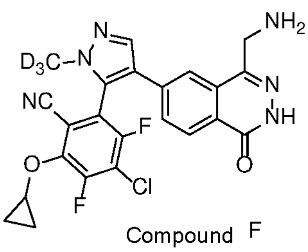

Compound F

The molecular docking process was conducted by using the Induced Fit Docking[1-2] default settings in Maestro (Schrödinger version 2021-2). The crystal structure of PRMT5 in the PDB database (PDB ID: 5FA5) was selected as the docking template. To prepare the protein, hydrogen atoms were added using the Protein Preparation Wizard module in Maestro[2], and energy minimization was performed using the OPLS4 force field. For ligand preparation, the three-dimensional structure of the molecule was generated using LigPrep[3], and energy minimization was performed using the OPLS4 force field. A box was automatically generated centered on Trp579 of 5FA5, in which the example compounds were placed during the molecular docking process. The interactions between the protein and the example compounds were analyzed, and then reasonable docking conformations were selected and saved based on the calculated IFD scores and binding modes. The predicted binding mode of compound A with PRMT5 is shown in Fig. 1. The predicted binding modes of compound B with PRMT5 are shown in Figs. 2 to 5. The predicted binding modes of compound C with PRMT5 are shown in Figs. 6 to 9. The predicted binding mode of compound D with PRMT5 is shown in Fig. 10. The predicted binding mode of compound F with PRMT5 is shown in Fig. 11.

[1] Induced Fit Docking protocol; Glide, Schrödinger, LLC, New York, NY, 2021.
[2] Prime, Schrödinger, LLC, New York, NY, 2021.
[3] Maestro, Schrödinger, LLC, New York, NY, 2021.
[4] LigPrep, Schrödinger, LLC, New York, NY, 2021.

Conclusion: The compounds of the present disclosure exhibit excellent binding to PRMT5.

**Reference example 1: Compound M1**

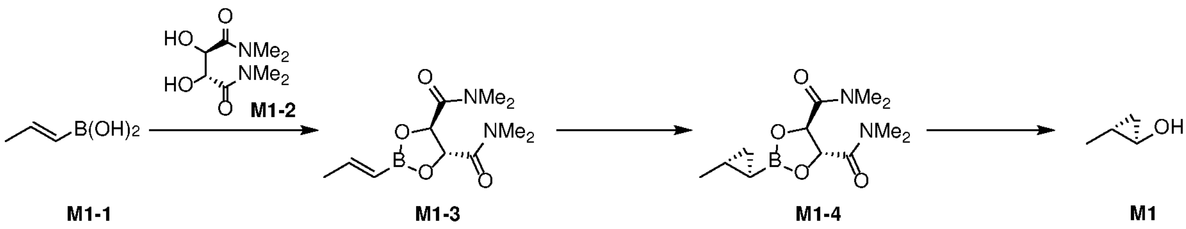

**Step 1**

Compound **M1-1** (4.2 g, 48.90 mmol) and compound **M1-2** (9.99 g, 48.90 mmol) were dissolved in anhydrous dichloromethane (80 mL), and 4.2 g of 3Å molecular sieves were added thereto. The reaction mixture was replaced with nitrogen three times and stirred at 25°C for 16 hours. The reaction mixture was filtered, and the filter cake was washed with 30 mL of dichloromethane. The filtrate was concentrated under reduced pressure to obtain compound **M1-3,** which was directly used in the next step without purification. $^1H$ NMR (400 MHz, $CDCl_3$) $\delta$: 6.62 - 6.78 (m, 1H), 5.57 (s, 2H), 5.41 - 5.50 (m, 1H), 3.21 (s, 6H), 2.98 (s, 6H), 1.81 - 1.89 (m, 3H).

**Step 2**

Anhydrous dichloromethane (120 mL) was added to a pre-dried three-necked flask, which was replaced with nitrogen three times. Diethylzinc (1 M, 140.50 mL) was then added thereto, and the mixture was cooled to -65°C. Diiodomethane (56.45 g, 210.75 mmol) was added dropwise thereto, and the mixture was stirred for 20 minutes. A solution of compound **M1-3** (11.9 g, 46.83 mmol) in anhydrous dichloromethane (120 mL) was added dropwise thereto at -65°C. After the dropwise addition was completed, the mixture was stirred at -65°C for 2 hours. The reaction mixture was slowly added dropwise with 150 mL of saturated ammonium chloride aqueous solution at -65°C, and slowly warmed to room temperature. The phases were separated, and the aqueous phase was extracted with dichloromethane (30 mL * 3). The organic phases were combined, dried over anhydrous sodium sulfate, and filtered. The filtrate was concentrated under reduced pressure to obtain compound **M1-4,** which was directly used in the next step without purification. $^1H$ NMR (400 MHz, $CDCl_3$) $\delta$: 5.49 (s, 2H), 3.17 (s, 6H), 2.97 (s, 6H), 0.95 - 1.12 (m, 4H), 0.68 - 0.78 (m, 1H), 0.38 - 0.46 (m, 1 H), 0.46 - 0.34 (m, 1H).

**Step 3**

Compound **M1-4** (4.95 g, 18.46 mmol) was dissolved in a mixture of methyl *tert*-butyl ether (75 mL) and water (75 mL). Sodium perborate tetrahydrate (28.41 g, 184.62 mmol) was added thereto, and the mixture was stirred at 25°C for 12 hours. The phases were separated, and the aqueous phase was extracted with methyl *tert*-butyl ether (15 mL * 3). The organic phases were combined, dried over anhydrous sodium sulfate, and filtered. The filtrate was concentrated under reduced pressure to obtain compound **M1,** which was directly used in the next step without purification. $^1H$ NMR (400 MHz, $CDCl_3$) $\delta$: 3.32 - 3.43 (m, 1H), 2.19 (br s, 1H), 1.08 - 1.19 (m, 4H), 0.83 - 0.96 (m, 1H), 0.48 (q, *J*= 5.6 Hz, 1H).

**Reference example 2: Compounds M2A and M2B**

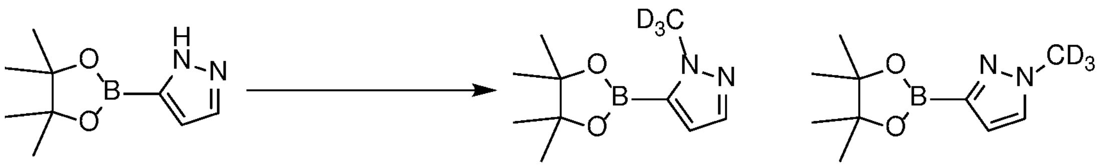

M2-1 M2A M2B

Under a nitrogen atmosphere, compound **M2-1** (30 g, 154.61 mmol) was dissolved in acetonitrile (300 mL), followed by the addition of cesium carbonate (50.37 g, 154.61 mmol) and deuterated iodomethane (22.38 g, 157.70 mmol). The reaction mixture was reacted at 20°C for 24 hours. The reaction system was filtered, and the filter cake was washed with acetonitrile (300 mL). The filtrate was concentrated under reduced pressure. The residue was then added with methyl *tert*-butyl ether (400 mL) and filtered. The filter cake was washed with methyl *tert*-butyl ether (300 mL), and the filtrate was concentrated under reduced pressure. The crude product was purified by SFC (chromatographic column: DAICEL CHIRALCEL OD (250 mm * 50 mm, 10 $\mu$m); mobile phase: phase A ($CO_2$) and phase B (isopropanol containing 0.1% ammonia water); gradient: B% = 11% to 11%) to obtain compound **M2A.** $^1$H NMR (400 MHz, $CDCl_3$) $\delta$: 7.47 (d, *J* = 1.6 Hz, 1H), 6.70 (d, *J* = 1.6 Hz, 1H), 1.33 (s, 12H).

SFC analytical method: chromatographic column: Chiralcel OD-3, 150 $\times$ 4.6 mm I.D., 3 $\mu$m; mobile phase: phase A ($CO_2$) and phase B (ethanol containing 0.1% isopropylamine); gradient: B% = 10% to 50%. The retention time for compound **M2A** was 0.895 minutes, and the retention time for its isomer **M2B** was 1.398 minutes.

**Reference example 3: Compound M3**

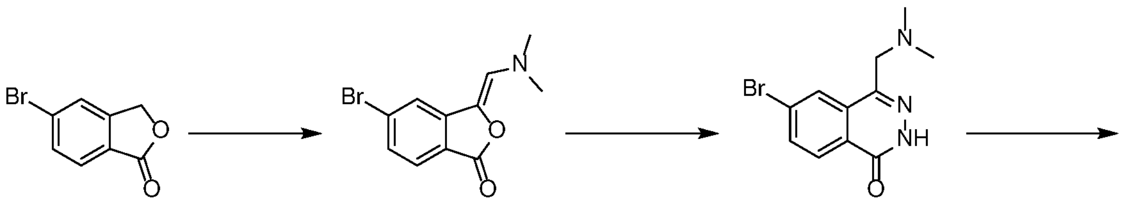

M3-1 M3-2 M3-3

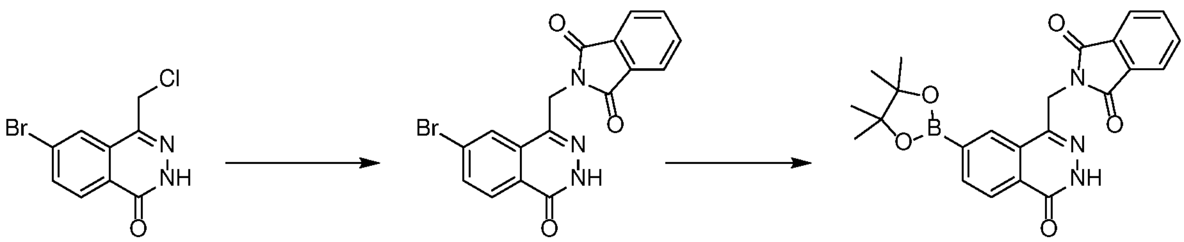

M3-4 M3-5 M3

**Step 1**

Compound **M3-1** (50 g, 234.71 mmol) was dissolved in *N,N*-dimethylformamide dimethyl acetal (180.12 g, 1.51 mol), and potassium *tert*-butoxide (2.63 g, 23.47 mmol) was added thereto. The reaction mixture was replaced with nitrogen three times, heated to 110°C, and stirred for 20 hours. The reaction mixture was concentrated under reduced pressure. The crude product was added with 100 mL of petroleum ether and stirred at 16°C for 0.5 hours. The reaction mixture was filtered to collect a solid, added with 200 mL of ethyl acetate, heated to 80°C, and stirred for 5 hours. The reaction mixture was filtered to collect a solid to obtain compound **M3-2.** $^1$H NMR (400 MHz, DMSO-$d_6$) $\delta$: 7.98 (d, *J* = 0.8 Hz, 1H), 7.60 (d, *J* = 8.4 Hz, 1H), 7.29 (dd, *J* = 8.4, 1.6 Hz, 1H), 7.01 (s, 1H), 3.11 (s, 6H).

**Step 2**

Compound **M3-2** (29.1 g, 108.54 mmol) was dissolved in anhydrous ethanol (450 mL), and 98% hydrazine

hydrate (11.85 g, 236.71 mmol) was added thereto. The reaction mixture was replaced with nitrogen three times, stirred at 16°C for 0.5 hours, then heated to 70°C, and stirred for 12 hours. The reaction mixture was cooled to room temperature and filtered. The filter cake was washed with 30 mL of ethanol, collected, and concentrated under reduced pressure to remove the residual solvent to obtain compound **M3-3.** $^1$H NMR (400 MHz, DMSO-$d_6$) δ: 12.66 (s, 1 H), 8.34 (d, *J* = 1.6 Hz, 1H), 8.13 (d, *J* = 8.4 Hz, 1H), 7.93 - 8.04 (m, 1H), 3.61 (s, 2H), 2.19 (s, 6H).

**Step 3**

Compound **M3-3** (20.7 g, 73.37 mmol) was dissolved in anhydrous tetrahydrofuran (300 mL), and the reaction mixture was replaced with nitrogen three times. The reaction mixture was cooled to 0°C, and isobutyl chloroformate (12.02 g, 88.04 mmol) was added dropwise thereto. After the addition was completed, the reaction mixture was warmed to 25°C and stirred for 12 hours. The reaction mixture was cooled to 0°C, added dropwise with 300 mL of 0.5 M hydrochloric acid aqueous solution, and stirred for 0.5 hours. The reaction mixture was filtered, and the filter cake was washed with tetrahydrofuran (30 mL * 3). The solid was collected and dried under reduced pressure to remove the solvent to obtain compound **M3-4.** $^1$H NMR (400 MHz, DMSO-$d_6$) δ: 12.92 (s, 1H), 7.90 - 8.52 (m, 3H), 5.07 (s, 2H).

**Step 4**

Compound **M3-4** (15.6 g, 57.04 mmol) was dissolved in *N*,*N*-dimethylformamide (170 mL), and potassium phthalimide (11.62 g, 62.74 mmol) was added thereto. The reaction mixture was replaced with nitrogen three times, warmed to 25°C, and stirred for 4 hours. The reaction mixture was added dropwise with 200 mL of 0.5 M dilute hydrochloric acid aqueous solution and filtered. The filter cake was sequentially washed with 100 mL of saturated sodium bicarbonate aqueous solution and 100 mL of water. The filter cake was then added with 50 mL of ethanol and stirred at 70°C for 1 hour. The reaction mixture was filtered, and the solid was collect and dried under reduced pressure to obtain compound **M3-5.** $^1$H NMR (400 MHz, DMSO-$d_6$) δ: 12.60 (s, 1H), 8.43 (d, *J* = 1.6 Hz, 1H), 8.03 - 8.23 (m, 2H), 7.83 - 8.00 (m, 4H), 5.19 (s, 2H).

**Step 5**

Compound **M3-5** (2.5 g, 6.51 mmol), bis(pinacolato)diboron (2.48 g, 9.76 mmol), and potassium acetate (1.92 g, 19.52 mmol) were dissolved in anhydrous dioxane (25 mL). The reaction mixture was replaced with nitrogen three times, and 1,1'-bis(diphenylphosphino)ferrocene palladium chloride (476.14 mg, 650.73 μmol) was added thereto. The reaction mixture was replaced with nitrogen three times, heated to 80°C, and stirred for 2 hours. The reaction mixture was concentrated under reduced pressure. The crude product was stirred with 20 mL of acetonitrile for 0.5 hours and filtered. The filter cake was collected to obtain compound **M3.** $^1$H NMR (400 MHz, DMSO-$d_6$) δ: 12.40 (s, 1H), 8.01 - 8.35 (m, 3H), 7.84 - 7.98 (m, 4H), 5.17 (s, 2H), 1.24 (s, 12H).

**Reference example 4: Compound M4**

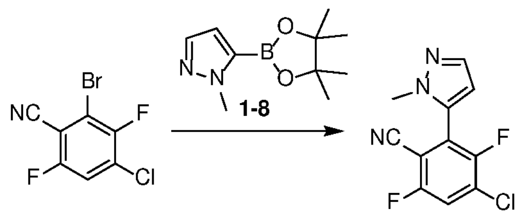


1-2 M4

Compound **1-2** (20 g, 79.23 mmol), compound **1-8** (32.97 g, 158.45 mmol), sodium carbonate (25.19 g, 237.68 mmol), and [1,1'-bis(di-*tert*-butylphosphino)ferrocene]dichloropalladium(II) (2.58 g, 3.96 mmol) were dissolved in 600 mL of *N,N*-dimethylformamide. The reaction mixture was replaced with nitrogen three times and stirred at 80°C for 48 hours. After the reaction was completed, the reaction mixture was concentrated. The crude product was diluted with 300 mL of water and extracted with ethyl acetate (300 mL × 3). The organic phase was dried over anhydrous sodium sulfate, filtered, and concentrated under reduced pressure. The crude product was purified by flash column chromatography (silica gel; eluent: ethyl acetate/petroleum ether; ethyl acetate ratio: 0 to 20%) to obtain compound **M4.** $^1$H NMR

(400 MHz, $CDCl_3$) δ = 7.67 (d, *J* = 2.0 Hz, 1H), 7.47 (dd, *J* = 5.6, 7.6 Hz, 1H), 6.57 (d, *J* = 2.0 Hz, 1H), 3.86 (d, *J* = 1.2 Hz, 3H).

**Reference example 5: Compound M5**

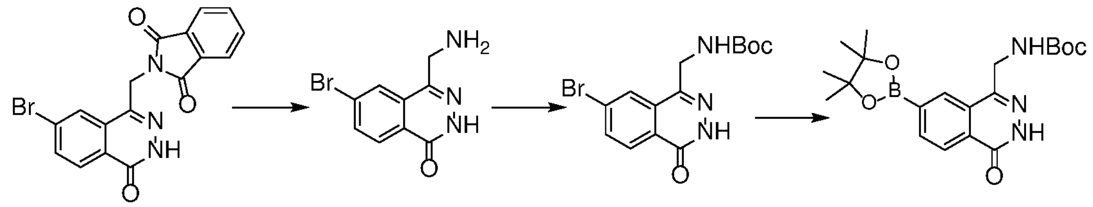

**M3-5** **M5-1** **M5-2** **M5**

Step 1

Compound **M3-5** (6 g, 15.62 mmol) and hydrazine hydrate (3.68 g, 62.47 mmol, purity of 85%) were dissolved in 100 mL of ethanol. The mixture was stirred at 80°C for 15 hours. After the reaction was completed, the reaction mixture was concentrated to obtain compound **M5-1.** MS-ESI calculated for $[M+1]^+$ 254.0 and 256.0, found 253.8 and 255.8.

Step 2

Compound **M5-1** (3.9 g, 15.35 mmol), di-*tert*-butyl dicarbonate (6.70 g, 30.70 mmol), and triethylamine (4.66 g, 46.05 mmol) were dissolved in 100 mL of dichloromethane. The mixture was stirred at 25°C for 24 hours. After the reaction was completed, the reaction mixture was filtered, and the filter cake was collected and dried to obtain compound **M5-2.** $^1$H NMR (400 MHz, DMSO-$d_6$) δ = 12.67 (br s, 1H), 8.27 (s, 1H), 8.17 (d, *J* = 8.3 Hz, 1H), 8.02 (d, *J*= 8.5 Hz, 1H), 4.41 (s, 2H), 1.41 (s, 9H).

Step 3

Compound **M5-2** (4.1 g, 11.58 mmol), bis(pinacolato)diboron (4.41 g, 17.36 mmol), 1,1'-bis(diphenylphosphino)ferrocene palladium chloride (846.98 mg, 1.16 mmol), and potassium acetate (3.41 g, 34.73 mmol) were added to 100 mLof anhydrous 1,4-dioxane. The reaction mixture was replaced with nitrogen three times and stirred at 100°C for 15 hours. After the reaction was completed, the reaction mixture was cooled to room temperature, allowed to stand for 2 hours, and filtered. The filtrate was concentrated and added with 20 mLof a mixed solvent of petroleum ether/ethyl acetate (10:1, v/v). The reaction mixture was slurried and stirred at room temperature for 1 hour, filtered, and the filter cake was collected to obtain compound **M5.** MS-ESI calculated for $[M+1]^+$ 402.2, found 402.2.

**Example 1**

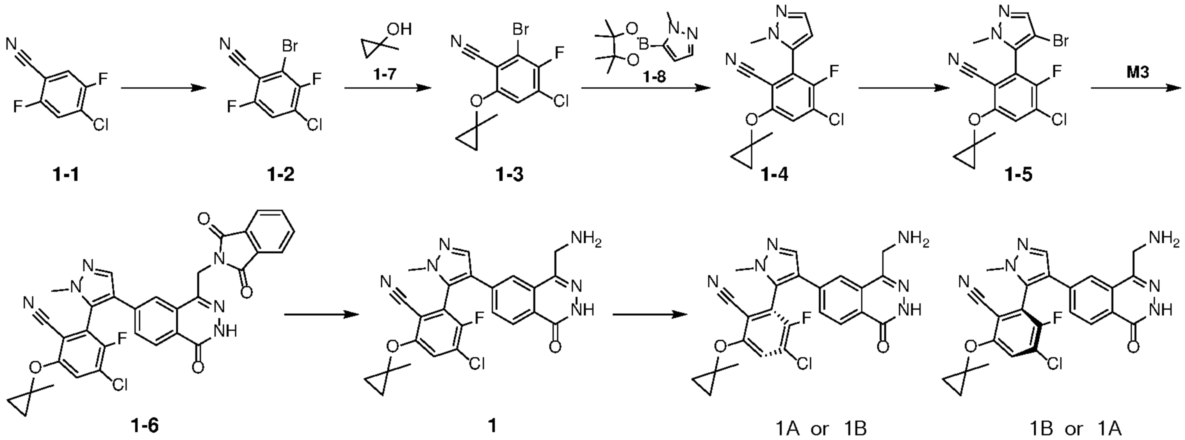


**1-1** **1-2** **1-3** **1-4** **1-5**

**1-6** **1** 1A or 1B 1B or 1A

**Step 1**

Compound 1 (5 g, 28.81 mmol), *N*-bromosuccinimide (10.26 g, 57.62 mmol), *p*-toluenesulfonic acid (2.48 g, 14.41 mmol), 1,2-dichloroethane (50 mL), and palladium acetate (646.82 mg, 2.88 mmol) were added to a pre-dried single-necked flask. The reaction mixture was thoroughly replaced with nitrogen and stirred at 75°C for 20 hours. The reaction mixture was filtered, and the filter cake was washed with dichloromethane (20 mL * 2). The filtrate was concentrated under reduced pressure. The crude product was purified by silica gel column chromatography (ethyl acetate/petroleum ether, ethyl acetate ratio of 0 to 10%) to obtain compound **1-2.** $^1H$ NMR (400 MHz, $CDCl_3$) δ ppm 7.28 (d, *J* = 5.50 Hz, 1 H).

**Step 2**

Compound **1-7** (500 mg, 6.93 mmol) was dissolved in anhydrous tetrahydrofuran (50 mL). The reaction mixture was replaced with nitrogen three times, cooled to 0°C, added with sodium hydride (416.06 mg, 10.40 mmol, purity of 60%), and stirred for 10 minutes. Compound **1-2** (1.75 g, 6.93 mmol) was then added thereto at 0°C, and the reaction mixture was slowly warmed to 16°C and stirred for 2 hours. The reaction mixture was poured into 100 mLof saturated ammonium chloride aqueous solution and extracted with ethyl acetate (10 mL * 3). The organic phases were combined, dried over anhydrous sodium sulfate, filtered, and the filtrate was concentrated under reduced pressure. The crude product was purified by silica gel column chromatography (petroleum ether: ethyl acetate = 100:1 to 10:1) to obtain compound **1-3.**

**Step 3**

Compound **1-3** (1.2 g, 3.94 mmol), compound **1-8** (2.46 g, 11.82 mmol), and sodium carbonate (1.67 g, 15.76 mmol) were dissolved in a mixture of dioxane (20 mL) and water (5 mL). The reaction mixture was replaced with nitrogen three times, added with [1,1'-bis(di-*tert*-butylphosphino)ferrocene]dichloropalladium(II) (256.81 mg, 394.03 μmol), heated to 80°C, and stirred for 16 hours. The reaction mixture was poured into 100 mL of saturated brine and extracted with ethyl acetate (30 mL * 3). The organic phases were combined, dried over anhydrous sodium sulfate, filtered, and concentrated under reduced pressure. The resulting crude product was purified by silica gel column chromatography (petroleum ether: ethyl acetate = 10:1 to 3:1) to obtain compound **1-4.** $^1H$ NMR (400 MHz, $CDCl_3$) δ ppm 7.61 (d, *J* = 2.0 Hz, 1H), 7.42 (d, *J* = 6.0 Hz, 1H), 6.50 (d, *J* = 2.0 Hz, 1H), 3.81 (d, *J* =1.2 Hz, 3H), 1.63 (s, 3H), 1.09 - 1.16 (m, 2H), 0.82 - 0.90 (m, 2H).

**Step 4**

Compound **1-4** (760 mg, 2.49 mmol) was dissolved in acetonitrile (40 mL). The reaction mixture was replaced with nitrogen three times, added with *N*-bromosuccinimide (884.87 mg, 4.97 mmol), heated to 40°C, and stirred for 2 hours. The reaction mixture was concentrated under reduced pressure. The resulting crude product was purified by silica gel column chromatography (petroleum ether: ethyl acetate = 10:1 to 3:1) to obtain compound **1-5.** $^1H$ NMR (400 MHz, $CDCl_3$) δ ppm 7.61 (s, 1H), 7.48 (d, *J* = 6.0 Hz, 1H), 3.80 (s, 3H), 1.65 (s, 3H), 1.10 - 1.19 (m, 2H), 0.83 - 0.94 (m, 2H).

**Step 5**

Compound **1-5** (400 mg, 1.04 mmol), compound **M3** (538.18 mg, 1.25 mmol), and potassium phosphate tribasic (662.24 mg, 3.12 mmol, 3 eq) were dissolved in a mixture of dioxane (6 mL) and water (1.5 mL). The reaction mixture was replaced with nitrogen three times, added with [1,1'-bis(di-*tert*-butylphosphino)ferrocene]dichloropalladium(II) (67.78 mg, 104.00 μmol), heated to 80°C, and stirred for 12 hours. The reaction mixture was poured into 30 mL of saturated brine and extracted with ethyl acetate (10 mL * 3). The organic phases were combined, dried over anhydrous sodium sulfate, filtered, and the filtrate was concentrated under reduced pressure. The crude product was purified by silica gel column chromatography (dichloromethane: methanol = 100:1 to 30:1) to obtain compound **1-6.**

**Step 6**

Compound **1-6** (330 mg, 541.87 μmol) was dissolved in anhydrous ethanol (7 mL), and 98% hydrazine hydrate (310 mg, 6.19 mmol) was added thereto. The mixture was heated to 80°C and stirred for 2 hours. The reaction mixture was then concentrated under reduced pressure. The crude product was added with 4 mL of DMF, filtered, and the filtrate was collected. The crude product was separated by prep-HPLC (chromatographic column: C18 75 * 30 mm * 3 μm; mobile phase A: water (0.1% HCl), mobile phase B: acetonitrile; gradient: B%: 10% to 45%) to obtain compound **1.**

**Step 7**

Compound **1** was further separated by SFC (chromatographic column: DAICEL CHIRAL PAKIC (250 mm * 30 mm, 10 μm); mobile phase: phase A ($CO_2$) and phase B (ethanol containing 0.1% ammonia water); gradient (B%): 50% to 50%) to obtain compound **1A** and compound **1B.**

Compound **1A:** SFC analytical method: chromatographic column: Chiralpak IC-3, 150 × 4.6 mm I.D., 3 μm; mobile phase: [A ($CO_2$) and B (isopropanol containing 0.1% diethylamine)]; gradient: B% = 50%; the retention time was 4.221 minutes, with ee being 98.74%. $^1$HNMR (400 MHz, $CD_3OD$) δ ppm 8.27 (d, *J* = 8.53 Hz, 1H), 7.76 - 7.82 (m, 2H), 7.61 (d, *J* = 8.03 Hz, 1H), 7.25 (s, 1H), 4.05 - 4.18 (m, 2H), 3.85 (s, 3H), 1.64 (s, 3H), 1.07 - 1.13 (m, 2H), 0.89 - 0.94 (m, 2H). MS m/z: 479.0 $[M+H]^+$.

Compound **1B:** SFC analytical method: chromatographic column: Chiralpak IC-3, 150 × 4.6 mm I.D., 3 μm; mobile phase: [A ($CO_2$) and B (isopropanol containing 0.1% diethylamine)]; gradient: B% = 50%; the retention time was 6.128 minutes, with ee being 99.01%. $^1$HNMR (400 MHz, $CD_3OD$) δ ppm 8.24 - 8.31 (m, 1H), 7.74 - 7.84 (m, 2H), 7.61 (br d, *J* = 9.79 Hz, 1H), 7.17 - 7.29 (m, 1H), 4.03 - 4.17 (m, 2H), 3.85 (s, 3H), 1.64 (s, 3H), 1.06 - 1.13 (m, 2H), 0.88 - 0.94 (m, 2H). MS m/z: 479.0 $[M+H]^+$.

**Example 2**

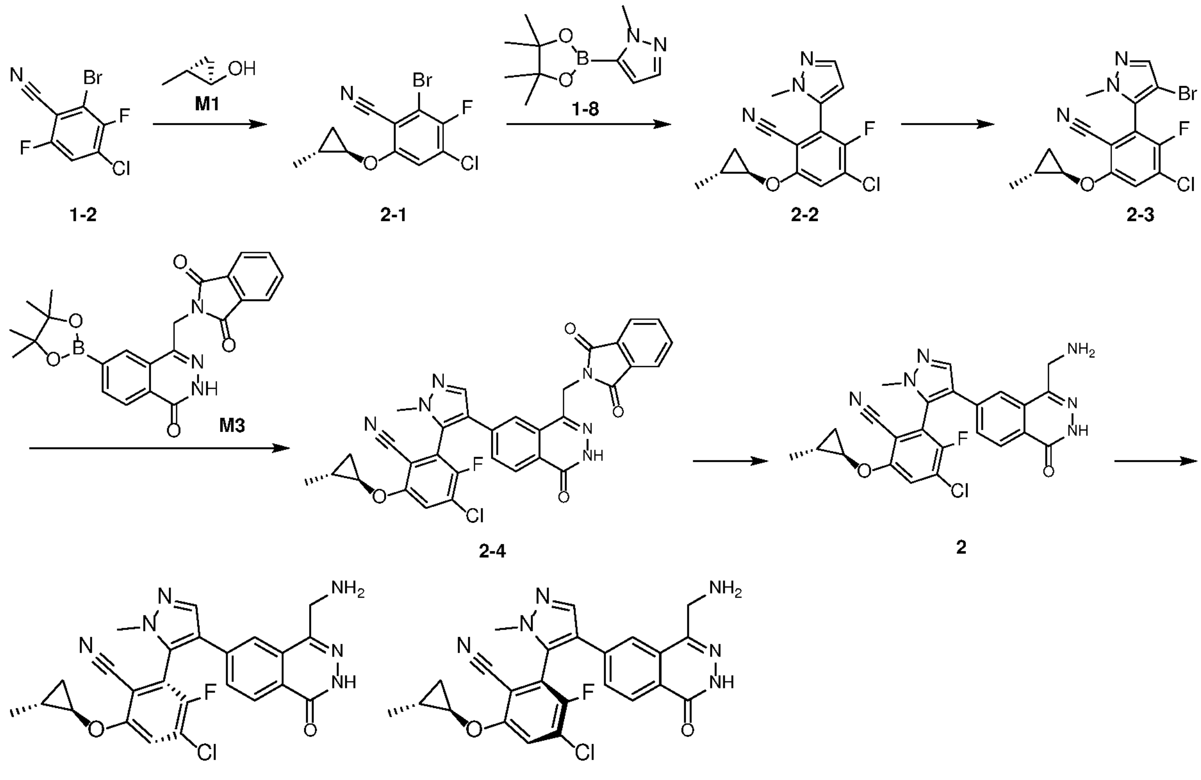


1-2 2-1 2-2 2-3

2-4 2

2A or 2B 2B or 2A

**Step 1**

Compound **M1** (270 mg, 3.74 mmol) and tetrahydrofuran (4 mL) were added to a pre-dried three-necked flask, then sodium hydride (299.53 mg, 7.49 mmol, purity of 60%) was added thereto at 0°C, and the mixture was stirred at 0°C for 10 minutes. A solution of compound **1-2** (945.28 mg, 3.74 mmol) in tetrahydrofuran (2 mL) was then added thereto, and the mixture was stirred at 20°C for 2 hours. The reaction mixture was quenched with an aqueous solution (10 mL), added with saturated brine (10 mL), and extracted with ethyl acetate (10 mL * 3). The organic phases were collected and combined, dried over anhydrous sodium sulfate, and filtered. The filtrate was concentrated under reduced pressure. The resulting crude product was purified by silica gel column chromatography (ethyl acetate: petroleum ether = 1:10) to obtain compound **2-1.**

**Step 2**

Compound **2-1** (2.1 g, 6.90 mmol), compound **1-8** (2.87 g, 13.80 mmol), 1,4-dioxane (40 mL), water (10 mL), and potassium phosphate (4.39 g, 20.70 mmol) were added to a pre-dried single-necked flask. The reaction mixture was thoroughly replaced with nitrogen, added with 1,1'-bis(di-*tert*-butylphosphino)ferrocene palladium chloride (539.30 mg, 828.00 μmol), and stirred at 80°C for 20 hours. The reaction mixture was concentrated under reduced pressure, added with saturated brine (15 mL), and extracted with ethyl acetate (20 mL * 3). The organic phases were collected and combined, dried over anhydrous sodium sulfate, and filtered. The filtrate was concentrated under reduced pressure. The resulting crude product was purified by silica gel column chromatography (ethyl acetate: petroleum ether = 1:10) to obtain compound **2-2.** MS m/z: 305.9 $[M+H]^+$.

**Step 3**

Compound **2-2** (320 mg, 1.05 mmol), *N*-bromosuccinimide (372.58 mg, 2.09 mmol), and acetonitrile (5 mL) were added to a pre-dried single-necked flask, and the mixture was stirred at 40°C for 2 hours. The reaction mixture was concentrated under reduced pressure. The crude product was purified by silica gel column chromatography (ethyl acetate: petroleum ether = 1:10) to obtain compound **2-3.** MS m/z: 383.9 $[M+H]^+$, 385.9 $[M+H]^+$.

**Step 4**

Compound **2-3** (500 mg, 1.30 mmol), compound **M3** (689.54 mg, 1.60 mmol), potassium phosphate (551.87 mg, 2.60 mmol), 1,4-dioxane (10 mL), and water (2.5 mL) were added to a pre-dried single-necked flask. The reaction mixture was thoroughly replaced with nitrogen, then added with 1,1'-bis(di-*tert*-butylphosphino)ferrocene palladium chloride (101.67 mg, 155.99 μmol), and stirred at 85°C for 3 hours. The reaction mixture was added with saturated brine (50 mL) and extracted with ethyl acetate (15 mL * 3). The organic phases were collected and combined, and concentrated under reduced pressure. The crude product was purified by silica gel column chromatography (ethyl acetate: petroleum ether = 1:10) to obtain compound **2-4.** MS m/z: 609.1 $[M+H]^+$.

**Step 5**

Compound **2-4** (100 mg, 164.20 μmol), ethanol (2 mL), and 98% hydrazine hydrate (16.78 mg, 328.40 μmol) were added to a pre-dried single-necked flask, and the mixture was stirred at 80°C for 2 hours. The reaction mixture was concentrated under reduced pressure and separated by prep-HPLC (chromatographic column: Phenomenex Luna C18 80 * 40 mm * 3 μm; mobile phase: [water (hydrochloric acid)-acetonitrile]; gradient (acetonitrile%): 1% to 35%) to obtain compound **2.**

**Step 6**

Compound **2** was further separated by SFC (chromatographic column: DAICEL CHIRALPAK IC (250 mm * 30 mm,10 μm); mobile phase: [A: $CO_2$, B: isopropanol containing 0.1% ammonia water]; B%: 65% to 65%) to obtain compound **2A** and compound **2B.**

Compound **2A:** SFC analytical method: chromatographic column: Chiralpak IC-3, 50 × 4.6 mm I.D., 3 μm; mobile phase: [A: $CO_2$, B: isopropanol containing 0.1% isopropylamine]; A:B = 50:50; the retention time was 1.352 minutes, with ee being 96.56%. $^1H$ NMR (400 MHz, $CDCl_3$) δ ppm 9.50 - 10.07 (m, 1 H) 8.24 (br dd, *J* = 3.94, 2.31 Hz, 1 H) 7.83 (s, 1 H) 7.56 (s, 1 H) 7.40 - 7.46 (m, 1 H) 7.38 (br d, *J* = 5.75 Hz, 1 H) 3.96 (br d, *J* = 1.88 Hz, 2 H) 3.76 (s, 3 H) 3.40 - 3.49 (d, 1 H) 1.14 - 1.21 (m, 1 H) 1.04 - 1.12 (m, 3 H) 0.90 - 1.02 (m, 1 H) 0.71 - 0.73 (m, 1 H). MS m/z: 479.2 $[M+H]^+$.

Compound **2B** was subjected to SFC (chromatographic column: Chiralpak IC-3, 50 × 4.6 mm I.D., 3 μm; mobile phase: [A: $CO_2$, B: isopropanol containing 0.1% isopropylamine]; A:B = 50:50; the retention time was 2.269 minutes, with ee being 97.23%). $^1H$ NMR (400 MHz, $CDCl_3$) δ ppm 10.07 - 10.67 (s, 1 H) 8.26 (d, *J* = 7.75 Hz, 1 H) 7.84 (s, 1 H) 7.58 (s, 1 H) 7.33 - 7.49 (m, 2 H) 3.98 (d, 2 H) 3.76 (s, 3 H) 3.45 (d, *J* = 3.00 Hz, 1 H) 1.18 (m, *J* = 1.75 Hz, 1 H) 1.05 - 1.14 (m, 3 H) 0.85 - 0.99 (m, 1 H) 0.52 - 0.72 (m, 1 H). MS m/z: 479.2 $[M+H]^+$.

**Example 3**

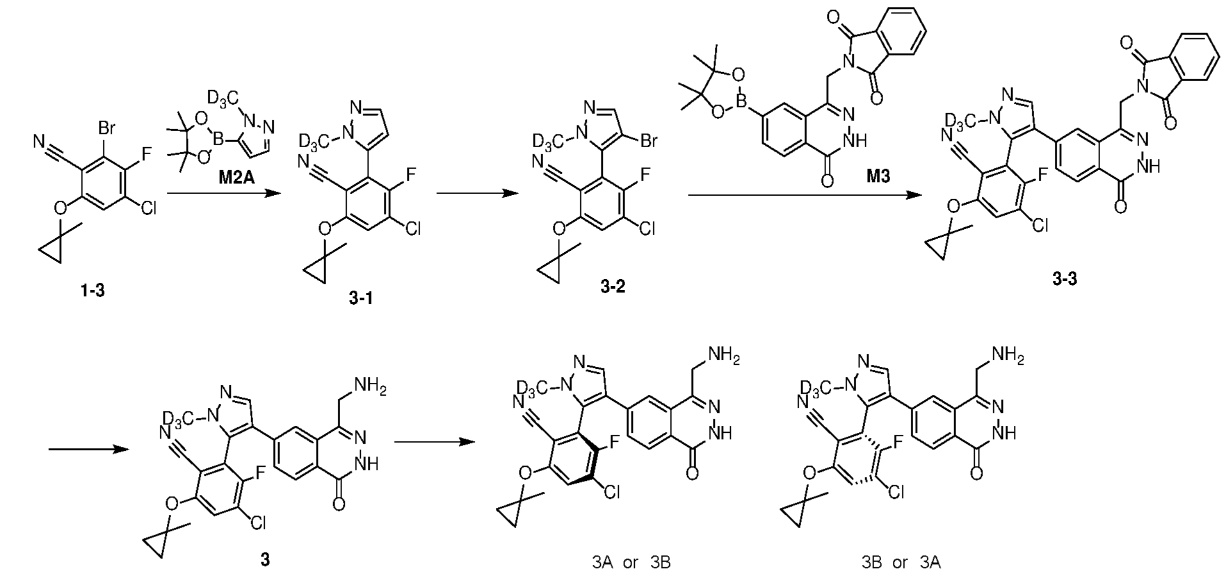

1-3 M2A 3-1 3-2 M3 3-3

3 3A or 3B 3B or 3A

**Step 1**

Compound **1-3** (1 g, 3.28 mmol), compound **M2A** (1.73 g, 8.21 mmol), and potassium phosphate tribasic (2.79 g, 13.13 mmol) were dissolved in a mixture of dioxane (16 mL) and water (4 mL). The reaction mixture was replaced with nitrogen three times, and [1,1'-bis(di-*tert*-butylphosphino)ferrocene]dichloropalladium(II) (214.01 mg, 328.36 μmol) was added thereto. The reaction mixture was replaced with nitrogen three times, heated to 80°C, and stirred for 2 hours. The reaction mixture was concentrated under reduced pressure. The resulting crude product was purified by silica gel column chromatography (petroleum ether: ethyl acetate = 10:1 to 3:1) to obtain compound **3-1.** $^1$H NMR (400 MHz, $CDCl_3$) δ: 7.61 (d, *J* = 2.0 Hz, 1H), 7.42 (d, *J* = 6.0 Hz, 1H), 6.50 (d, *J* = 2.0 Hz, 1H), 1.63 (s, 3H), 1.07 - 1.19 (m, 2H), 0.81 - 0.89 (m, 2H).

**Step 2**

Compound **3-1** (480 mg, 1.55 mmol) and *N*-bromosuccinimide (553.40 mg, 3.11 mmol) were dissolved in acetonitrile (10 mL). The reaction mixture was replaced with nitrogen three times, heated to 40°C, and stirred for 2 hours. The reaction mixture was concentrated under reduced pressure. The resulting crude product was purified by silica gel column chromatography (petroleum ether: ethyl acetate = 10:1 to 3:1) to obtain compound **3-2.** $^1$H NMR (400 MHz, $CDCl_3$) δ: 7.61 (s, 1H), 7.48 (d, *J* = 6.0 Hz, 1H), 1.65 (s, 3H), 1.09 - 1.20 (m, 2H), 0.82 - 0.91 (m, 2H).

**Step 3**

Compound **3-2** (500 mg, 1.29 mmol), compound **M3** (834.35 mg, 1.93 mmol), and potassium phosphate tribasic (821.37 mg, 3.87 mmol) were dissolved in a mixture of dioxane (8 mL) and water (2 mL). The reaction mixture was replaced with nitrogen three times, added with [1,1'-bis(di-*tert*-butylphosphino)fefrocene]dichloropalladium(II) (84.06 mg, 128.98 μmol), heated to 80°C, and stirred for 2 hours. The reaction mixture was concentrated under reduced pressure. The crude product was purified by silica gel column chromatography (dichloromethane: methanol = 100:1 to 30:1) to obtain compound **3-3.**

**Step 4**

Compound **3-3** (600 mg, 980.35 μmol) was dissolved in anhydrous ethanol (12 mL), and 98% hydrazine hydrate (260 mg, 4.15 mmol) was added thereto. The mixture was heated to 80°C and stirred for 2 hours. The reaction mixture was concentrated under reduced pressure. The crude product was first purified by preparative high-performance liquid chromatography (chromatographic column: Phenomenex Luna 75 * 30 mm * 3 μm; mobile phase A: water (0.1% HCl), mobile phase B: acetonitrile; gradient: B%: 20% to 50%) to obtain compound **3.**

**Step 5**

Compound **3** was further purified by SFC (column: DAICEL CHIRALPAK IC (250 mm * 30 mm, 10 μm); mobile phase: A ($CO_2$) and B (acetonitrile/isopropanol = 1:1, containing 0.1% ammonia water); gradient: B% = 60% to 60%). The separated eluent containing the product was subjected to post-processing by concentrating under reduced pressure. The residue was dissolved in acetonitrile/water and then freeze-dried to obtain compound **3A** and compound **3B.**

Compound **3A:** SFC analytical method (column: Chiralpak IC-3, 50 × 4.6 mm I.D., 3 μm; mobile phase: A ($CO_2$) and B (isopropanol containing 0.1% isopropylamine); gradient: B% = 50%). Under the above conditions, the retention time was 2.221 minutes, with ee being 96.70%. $^1$H NMR (400 MHz, $CD_3OD$) δ ppm 8.24 (d, *J* = 8.4 Hz, 1H), 8.14 (s, 1H), 7.66 - 7.82 (m, 2H), 7.52 - 7.66 (m, 1H), 3.89 - 4.13 (m, 2H), 1.62 (s, 3H), 1.02 - 1.15 (m, 2H), 0.79 - 0.98 (m, 2H). MS m/z: 482.2 $[M+H]^+$.

Compound **3B:** SFC analytical method (column: Chiralpak IC-3, 50 × 4.6 mm I.D., 3 μm; mobile phase: A ($CO_2$) and B (isopropanol containing 0.1% isopropylamine); gradient: B% = 50%). Under the above conditions, the retention time was 1.312 minutes, with ee being 95.56%. $^1$H NMR (400 MHz, $CD_3OD$) δ: 8.22 (t, *J* = 9.2 Hz, 1H), 8.13 (d, *J* = 2.0 Hz, 1H), 7.77 (d, *J* = 6.0 Hz, 1H), 7.70 (d, *J* = 4.4 Hz, 1H), 7.59 (t, *J* = 7.0 Hz, 1H), 3.97 (s, 2H), 1.61 (s, 3H), 0.99 - 1.17 (m, 2H), 0.81 - 0.97 (m, 2H). MS m/z: 482.1 $[M+H]^+$.

**Example 4**

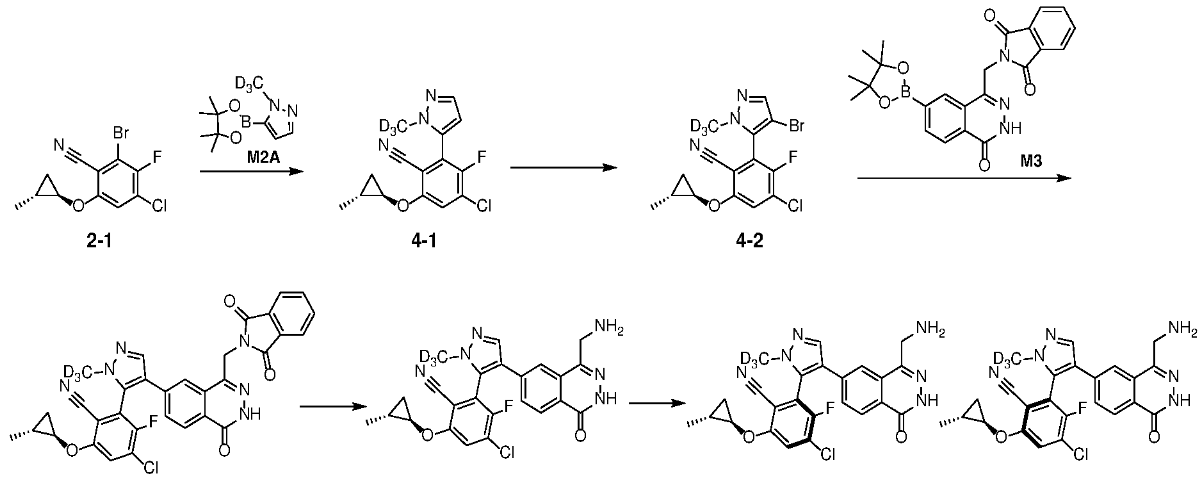

M2A, M3

2-1, 4-1, 4-2

4-3, 4, 4A or 4B, 4B or 4A

**Step 1**

Compound **2-1** (1.04 g, 3.41 mmol), compound **M2A** (2.16 g, 10.24 mmol), and potassium phosphate tribasic (2.90 g, 13.66 mmol) were dissolved in a mixture of dioxane (20 mL) and water (5 mL). The reaction mixture was replaced with nitrogen three times, and [1,1'-bis(di-*tert*-butylphosphino)ferrocene]dichloropalladium(II) (222.57 mg, 341.50 μmol) was added thereto. The reaction mixture was replaced with nitrogen three times, heated to 80°C, and stirred for 12 hours. The reaction mixture was concentrated under reduced pressure, added with 20 mL of water and 10 mL of ethyl acetate, filtered, and the phases were separated. The aqueous phase was extracted with ethyl acetate (10 mL * 2). The organic phases were combined, dried over anhydrous sodium sulfate, filtered, and concentrated under reduced pressure. The crude product was purified by silica gel column chromatography (petroleum ether: ethyl acetate = 10:1 to 3:1) to obtain compound **4-1.** $^1$H NMR (400 MHz, $CDCl_3$) δ: 7.61 (d, *J* = 2.0 Hz, 1H), 7.38 (d, *J* = 5.6 Hz, 1H), 6.50 (d, *J* = 2.0 Hz, 1H), 3.50 - 3.59 (m, 1H), 1.23 - 1.34 (m, 1H), 1.16 - 1.21 (m, 3H), 1.01 - 1.11 (m, 1H), 0.68 - 0.77 (m, 1H).

**Step 2**

Compound **4-1** (440.00 mg, 1.43 mmol) was dissolved in acetonitrile (2 mL). The reaction mixture was replaced with nitrogen three times, added with N-bromosuccinimide (507.29 mg, 2.85 mmol), heated to 40°C, and stirred for 8 hours. The reaction mixture was concentrated under reduced pressure. The crude product was purified by silica gel column chromatography (petroleum ether: ethyl acetate = 10:1 to 3:1) to obtain compound **4-2.** $^1$H NMR (400 MHz,

$CDCl_3$) δ: 7.61 (s, 1 H), 7.45 (d, *J* = 6.0 Hz, 1 H), 3.50 - 3.60 (m, 1 H), 1.25 - 1.38 (m, 1 H), 1.20 (d, *J* = 6.4 Hz, 3 H), 1.02 - 1.14 (m, 1 H), 0.68 - 0.80 (m, 1 H).

**Step 3**

Compound **4-2** (500 mg, 1.29 mmol), compound **M3** (834.35 mg, 1.93 mmol), and potassium phosphate tribasic (821.37 mg, 3.87 mmol) were dissolved in a mixture of dioxane (10 mL) and water (2 mL). The reaction mixture was replaced with nitrogen three times, added with [1,1'-bis(di-*tert*-butylphosphino)ferrocene]dichloropalladium(II) (84.06 mg, 128.98 μmol), heated to 80°C, and stirred for 12 hours. The reaction mixture was concentrated under reduced pressure. The crude product was purified by silica gel column chromatography (dichloromethane: methanol = 100:1, 50:1, 30:1) to obtain compound **4-3.** $^1H$ NMR (400 MHz, DMSO-$d_6$) δ: 12.44 (s, 1H), 8.33 (s, 1H), 8.12 - 8.20 (m, 1H), 7.87 - 8.01 (m, 5H), 7.82 - 7.87 (m, 1H), 7.52 - 7.65 (m, 1H), 5.00 - 5.15 (m, 2H), 3.89 - 3.98 (m, 1H), 1.14 - 1.23 (m, 1H), 1.04 - 1.12 (m, 3H), 0.89 - 0.96 (m, 1H), 0.68 - 0.76 (m, 1H).

**Step 4**

Compound **4-3** was dissolved in anhydrous ethanol (12 mL), and hydrazine hydrate (420 mg, 8.22 mmol) was added thereto. The mixture was heated to 80°C and stirred for 2 hours. The reaction mixture was concentrated under reduced pressure. The crude product was purified by preparative high-performance liquid chromatography (chromatographic column: Phenomenex Luna 80 * 30 mm * 3 μm; mobile phase A: water (0.1% HCl), mobile phase B: acetonitrile; gradient: B%: 1% to 30%) to obtain compound **4.**

**Step 5**

Compound **4** was purified by SFC (column: DAICEL CHIRALPAK IC (250 mm * 30 mm, 10 μm); mobile phase: A ($CO_2$) and B (ethanol containing 0.1% ammonia water); gradient: B% = 60% to 60%) to obtain compound **4A** and compound **4B.**

Compound **4A:** In SFC analysis (column: Chiralpak IC-3, 50 × 4.6 mm I.D., 3 μm; mobile phase: A ($CO_2$) and B (EtOH containing 0.1% isopropylamine); gradient: B% = 40%), the retention time was 2.056 minutes, with ee being 100%. $^1H$ NMR (400 MHz, $CD_3OD$) δ: 8.21 (d, *J* = 8.0 Hz, 1H), 8.13 (s, 1H), 7.66 - 7.79 (m, 2H), 7.59 (d, *J* = 8.0 Hz, 1H), 3.98 (s, 2 H), 3.73 - 3.84 (m, 1H), 1.12 - 1.32 (m, 4H), 0.90 - 1.02 (m, 1H), 0.73 (*J*= 6.0 Hz, 1H). MS m/z: 482.1 $[M+H]^+$.

Compound **4B:** In SFC analysis (column: Chiralpak IC-3, 50 × 4.6 mm I.D., 3 μm; mobile phase: A ($CO_2$) and B (EtOH containing 0.1% isopropylamine); gradient: B% = 40%), the retention time was 1.479 minutes, with ee being 98.83%. $^1H$ NMR (400 MHz, $CD_3OD$) δ: 8.23 (d, *J* = 8.4 Hz, 1H), 8.13 (s, 1H), 7.76 (d, *J* = 6.0 Hz, 1H), 7.71 (s, 1H), 7.60 (dd, *J* = 8.4, 1.2 Hz, 1H), 3.97 (d, *J* = 3.2 Hz, 2H), 3.73 - 3.82 (m, 1H), 1.19 - 1.26 (m, 1H), 1.13 - 1.17 (m, 3H), 0.92 - 1.02 (m, 1H), 0.73 (q, *J*= 6.4 Hz, 1H). MS m/z: 482.1 $[M+H]^+$.

**Example 5**

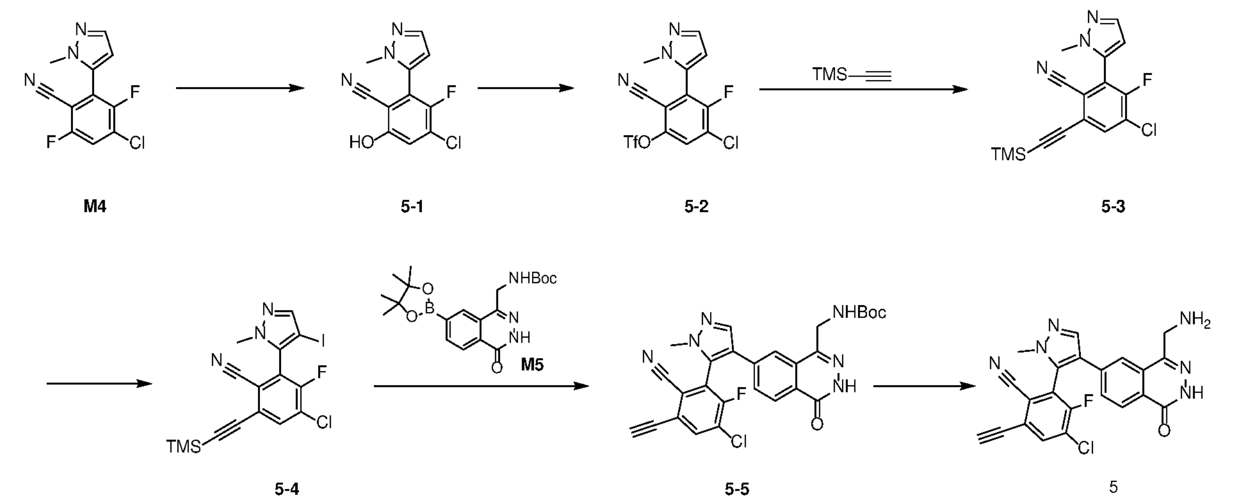

M4 5-1 5-2 5-3

M5

5-4 5-5 5

**Step 1**

2-(Methylsulfonyl)ethanol (587.42 mg) was added to *N,N*-dimethylformamide (6 mL), and the reaction system was under a nitrogen atmosphere. Sodium hydride (189.23 mg, purity of 60%) was added thereto in batches at 0°C, and the reaction mixture was stirred for 0.5 hours. A solution of compound **M4** (600 mg) in *N,N*-dimethylformamide (6 mL) was added dropwise thereto at 0°C, and the reaction mixture was stirred at 20°C for 1 hour. The reaction mixture was diluted with water (30 mL) and extracted with ethyl acetate (10 mL × 2). The aqueous phase was added with 1 M hydrochloric acid to adjust the pH to 2 to 3, and then extracted with ethyl acetate (15 mL × 3). The organic phases were combined, washed with saturated brine (10 mL), dried over anhydrous sodium sulfate, and filtered. The filtrate was concentrated under reduced pressure to obtain compound **5-1.** MS m/z: 252.0 $[M+H]^+$.

**Step 2**

Compound **5-1** (500 mg) was added to dichloromethane (10 mL), followed by the addition of pyridine (628.67 mg). Trifluoromethanesulfonic anhydride (840.89 mg) was added thereto at 0°C, and the reaction mixture was stirred at 20°C for 1 hour. The reaction mixture was added with water (20 mL) and stirred for 5 minutes, and the phases were separated. The aqueous phase was extracted with dichloromethane (10 mL). The organic phases were combined, washed with saturated brine (10 mL), dried over anhydrous sodium sulfate, and filtered. The filtrate was concentrated under reduced pressure to obtain a crude product. The crude product was purified by flash silica gel column chromatography (ethyl acetate/petroleum ether, ethyl acetate ratio of 0 to 20%) to obtain compound **5-2.** MS m/z: 383.9 $[M+H]^+$.

**Step 3**

Compound **5-2** (600 mg) was added to *N,N*-dimethylformamide (9 mL), followed by the addition of trimethylsilylacetylene (168.94 mg), bis(triphenylphosphine)palladium(II) dichloride (109.76 mg), cuprous iodide (14.89 mg), and triethylamine (237.34 mg). The reaction mixture was stirred at 20°C for 3 hours. The reaction mixture was added with water (30 mL) and ethyl acetate (20 mL), stirred for 5 minutes, and filtered. The filtrate was subjected to phase separation, and the aqueous phase was extracted with ethyl acetate (10 mL × 2). The organic phases were combined, washed with saturated brine, dried over anhydrous sodium sulfate, and filtered. The filtrate was concentrated under reduced pressure to obtain a crude product. The crude product was purified by flash silica gel column chromatography (ethyl acetate/petroleum ether, ethyl acetate ratio of 0 to 20%) to obtain compound **5-3.** MS m/z: 332.1 $[M+H]^+$.

**Step 4**

Compound **5-3** (170 mg) was added to acetic acid (3 mL), then *N*-iodosuccinimide (230.51 mg) was added thereto, and the reaction mixture was stirred at 20°C for 12 hours. The reaction mixture was then concentrated under reduced pressure, added with saturated sodium bicarbonate solution (10 mL) and ethyl acetate (10 mL), stirred for 5 minutes, and the phases were separated. The aqueous phase was extracted with ethyl acetate (5 mL). The organic phases were combined, washed with saturated brine (5 mL), dried over anhydrous sodium sulfate, and filtered. The filtrate was concentrated under reduced pressure to obtain a crude product. The crude product was purified by flash silica gel column chromatography (ethyl acetate/petroleum ether, ethyl acetate ratio of 0 to 20%) to obtain compound **5-4.** MS m/z: 457.9 $[M+H]^+$.

**Step 5**

Compound **5-4** (50 mg) was added to 1,4-dioxane (2 mL), and water (0.2 mL) was added thereto. Compound **M5** (65.75 mg), [1,1'-bis(di-*tert*-butylphosphino)ferrocene]dichloropalladium(II) (3.56 mg), and potassium phosphate (46.37 mg) were then added thereto. The reaction mixture was stirred at 80°C for 12 hours under a nitrogen atmosphere. The reaction mixture was cooled to room temperature, added with water (5 mL) and ethyl acetate (5 mL), stirred for 5 minutes, and filtered. The filtrate was subjected to phase separation, and the aqueous phase was extracted with ethyl acetate (5 mL). The organic phases were combined, washed with saturated brine (5 mL), dried over anhydrous sodium sulfate, and filtered. The filtrate was concentrated under reduced pressure to obtain a crude product. The crude product was purified by preparative thin-layer chromatography (petroleum ether: ethyl acetate = 1:1) to obtain compound **5-5.** MS m/z: 533.2 $[M+H]^+$.

**Step 6**

Compound **5-5** (5 mg) was added to dichloromethane (0.6 mL), then trifluoroacetic acid (0.2 mL) was added

thereto, and the reaction mixture was stirred at 20°C for 0.5 hours. The reaction mixture was concentrated under reduced pressure to obtain a crude product. The crude product was purified by preparative high-performance liquid chromatography (column: Welch Xtimate C18 100 * 40 mm * 3 μm; mobile phase: [water (trifluoroacetic acid)-acetonitrile]; gradient (acetonitrile%): 10% to 40%) to obtain the trifluoroacetate salt of compound **5.**

MS m/z: 433.1 $[M+H]^+$. $^1H$ NMR (400 MHz, $CD_3OD$) δ 8.28 (d, *J* = 8.03 Hz, 1H), 8.18 (s, 1H), 8.14 (d, *J* = 7.03 Hz, 1H), 7.85 (s, 1H), 7.50 (d, *J* = 10.04 Hz, 1H), 4.51 (br d, *J* = 5.02 Hz, 2H), 4.28 (s, 1H), 3.86 (s, 3H).

**Bioassay data**

**Experimental example 1: *In vitro* proliferation assay of HCT116 WT/MTAP KO cells**

**Experimental materials:**

McCoy's 5A medium, penicillin/streptomycin antibiotics, purchased from Wisent; fetal bovine serum, purchased from Biosera. 3D CellTiter-Glo (chemiluminescent cell viability assay reagent), purchased from Promega. HCT116 WT cell line, purchased from Nanjing Cobioer Biosciences Co., Ltd. HCT116 MTAP KO cell line, purchased from Horizon Discovery. Envision multilabel plate reader (PerkinElmer).

**Experimental methods:**

HCT116 WT and MTAP KO cells were seeded into an ultra-low attachment 96-well U-bottom plate at a density of 80 μL cell suspension per well containing 1000 HCT116 cells. The cell plate was incubated overnight in a $CO_2$ incubator.

The test compound was serially diluted 5-fold across eight concentrations using a pipette, ranging from 2 mM to 25.6 nM, and tested in duplicate. 78 μL of medium per well was added to an intermediate plate. Subsequently, 2 μL of the serially diluted compound per well was transferred to the corresponding wells of the intermediate plate and mixed well. 20 μL of the mixture per well was then transferred to the cell plate. The concentration of the compound transferred into the cell plate ranged from 10 μM to 0.128 nM. The cell plate was incubated for 10 days in a $CO_2$ incubator. An additional cell plate was prepared to read the signal value on the day of compound addition, which served as the maximum value (Max in the following equation) for data analysis.

The cell plate was added with 100 μL of chemiluminescent cell viability assay reagent per well and incubated at room temperature for 10 minutes to stabilize the luminescent signal. A multilabel microplate reader was used for reading.

**Data analysis:**

Using the equation (Sample - Min) / (Max - Min) * 100% to convert the raw data into inhibition rate, the $IC_{50}$ value can be obtained by curve fitting with four parameters (obtained by "log (inhibitor) vs. response -- Variable slope" mode in GraphPad Prism). Table 1 provides the inhibitory activity of the compounds of the present disclosure on the proliferation of HCT116 WT and MTAP KO cells.

Table 1: *In vitro* screening test results of compounds of the present disclosure

| Compound No. | HCT116 MTAP KO $IC_{50}$ (nM) | HCT116 WT $IC_{50}$ (μM) |
|---|---|---|
| **1A** | 8.00 | > 10 |
| **2A** | 3.99 | > 10 |
| **3A** | 7.43 | 4.7 |

Conclusion: The compounds of the present disclosure exhibit significant inhibitory activity against MTAP-deficient HCT116 cells, while showing a weak inhibitory effect on wild-type tumor cells, thereby demonstrating good selectivity.

**Experimental example 2: Enzyme inhibitory activity assay**

**Experimental purpose:** To test the inhibitory effect of the compounds of the present disclosure on the PRMT5·MTA complex.

**Experimental materials:** PRMT5/MEP50, peptide H4 (1-21), LANCE® Ultra Europium-anti-methyl-Histone H4 Arginine 3 (H4R3me) antibody, ULight streptavidin, LANCE detection buffer, SAM (S-adenosylmethionine), porcine skin collagen, and MTA (methylthioadenosine).

**Experimental methods:**

(1) Buffer preparation: Taking 10 mL as an example. 10 mM MTA: 10 mg of MTA was dissolved in 3296 μL of DMSO. The mixture was then aliquoted and stored at -80°C. The buffer was prepared on the day of the experiment, according to the schemes shown in Tables 2 and 3.

Table 2: Preparation scheme of buffer with MTA

| Component | Storage | Final | Addition |
|---|---|---|---|
| Bicine, pH 7.6 | 1000 mM | 20 mM | 200 μL |
| NaCl | 5000 mM | 25 mM | 50 μL |
| DTT | 1000 mM | 2 mM | 20 μL |
| Porcine skin collagen (gelatin) | 0.08% | 0.005% | 625 μL |
| MTA | 10 mM | 2.6 μM | 2.6 μL |
| Tween-20 | 1% | 0.01% | 100 μL |
| Deionized water | - | - | 9002.4 μL |

Table 3: Preparation scheme of buffer without MTA

| Component | Storage | Final | Addition |
|---|---|---|---|
| Bicine, pH 7.6 | 1000 mM | 20 mM | 200 μL |
| NaCl | 5000 mM | 25 mM | 50 μL |
| DTT | 1000 mM | 2 mM | 20 μL |
| Porcine skin collagen (gelatin) | 0.08% | 0.005% | 625 μL |
| Tween-20 | 1% | 0.01% | 100 μL |
| Deionized water | - | - | 9005.0 μL |

(2) Compound preparation

The compound was dissolved in DMSO to obtain a stock solution with a concentration of 10 mM. A gradient dilution was performed in a compound dilution plate to obtain four compound wells at the following concentrations: 1 mM, 37.037 μM, 1.3717 μM, and 0.0508 μM. These compounds at four concentrations were then transferred to a compound transfer plate with a transfer volume of 8 μL for each concentration. Additionally, DMSO was added to the empty wells of the compound transfer plate for later use. The Echo550 liquid handler was employed to perform serial dilutions and transfer the liquid to the experimental plate. Upon completion of the liquid transfer, the experimental plate was ready for use.

(3) Reaction

The enzyme solution and substrate mixture were prepared using the buffer, with PRMT5 at a concentration of 7.6 nM, peptide H4 (1-21) at a concentration of 0.32 μM, and SAM at a concentration of 2.6 μM.

5 μL of the PRMT5 solution per well was added to the compound wells and negative control wells of the experimental plate using an electronic multichannel pipette. An equal volume of buffer was added to the positive control wells. The experimental plate was centrifuged at 1000 rpm for one minute and then incubated at 25°C for 30 minutes in a temperature-controlled incubator. Subsequently, the substrate mixture was added to the positive control wells, negative control wells, and compound wells of the experimental plate using the same method. The plate was centrifuged and incubated at 25°C for 90 minutes.

(4) Detection and calculation

Principle: The experiment employed PerkinElmer's time-resolved fluorescence resonance energy transfer technology (LANCE® Ultra) for detection. During the reaction, two antibodies were added after PRMT methylated the substrate peptide H4 (1-21). The LANCE® Ultra Europium-anti-methyl-Histone H4 Arginine 3 (H4R3me) antibody acted as an energy donor and specifically bound to the methylation site on the peptide H4 (1-21), while ULight acted as an energy

acceptor and specifically bound to the biotin tag on the peptide H4 (1-21). When excited with a laser at a specific wavelength (the excitation wavelength in this experiment was 340 nm), the energy donor emitted light at a wavelength of 615 nm. If the spatial distance between the energy donor and the energy acceptor was close enough (i.e., both antibodies were simultaneously attached to the peptide H4 (1-21)), energy transfer occurred between the energy donor and the energy acceptor, causing the energy acceptor to emit light at a wavelength of 665 nm. The emitted lights at both 615 nm and 665 nm were detected using a plate reader, and the ratio of the 665 nm signal to the 615 nm signal was calculated. By plotting and calculation, the relevant parameters of the sample to be tested could be determined.

An antibody mixture was prepared using LANCE buffer, with a concentration of 4 nM for LANCE® Ultra Europium-anti-methyl-Histone H4 Arginine 3 (H4R3me) antibody and 53.3 nM for ULight. 10 μL of the detection solution per well was added to the positive control wells, negative control wells, and compound wells of the experimental plate using an electronic multichannel pipette. The plate was centrifuged and incubated at room temperature for 1 hour, and an Envision 2104 plate reader was used for reading. The inhibition rate of the compounds was calculated using interpolation. The inhibition rate was plotted using the four-parameter logistic curve and XLfit software to generate the inhibition curve of the compounds and calculate relevant parameters, including minimum inhibition rate, maximum inhibition rate, and $IC_{50}$.

Conclusion: The compounds of the present disclosure exhibit a significant inhibitory effect on the PRMT5. MTA complex.

**Experimental example 3: *In vitro* microsomal stability test of compounds**

**Experimental materials**

Liver microsomes: Human and animal microsomes were purchased from Corning or Xenotech and stored in a -80°C freezer.

Reduced nicotinamide adenine dinucleotide phosphate (NADPH), supplier: Chem-Impex International, Cat. No.: 00616.

Control compounds: Testosterone, Diclofenac, Propafenone.

**Experimental steps**

2.1 Preparation of working solution

Stock solution: 10 mM DMSO solution

Preparation of working concentration: The stock solution was diluted to 100 μM using 100% acetonitrile (organic phase content: 99% acetonitrile, 1% DMSO).

2.2 Experimental steps

Two 96-well incubation plates were prepared and named as T60 incubation plate and NCF60 incubation plate.

445 μL of microsomal working solution (liver microsomal protein concentration of 0.56 mg/mL) was added to both the T60 incubation plate and the NCF60 incubation plate. The two incubation plates were pre-incubated in a 37°C water bath for approximately 10 minutes.

After pre-incubation, 5 μL of the test compound or control compound working solution was added to both the T60 incubation plate and the NCF60 incubation plate and mixed well. 50 μL of potassium phosphate buffer per well was added to the NCF60 incubation plate to initiate the reaction. 180 μL of termination solution (acetonitrile solution containing 200 ng/mL tolbutamide and 200 ng/mL labetalol) and 6 μL of NADPH regeneration system working solution were added to a T0 termination plate. 54 μL of sample was then removed from the T60 incubation plate and added to the T0 termination plate to generate T0 samples. 44 μL of NADPH regeneration system working solution per well was added to the T60 incubation plate to initiate the reaction. Only 54 μL of microsomal working solution, 6 μL of NADPH regeneration system working solution, and 180 μL of termination solution were added to the blank plate. Therefore, in the samples containing the test compounds or control compounds, the final concentrations of the compounds, testosterone, diclofenac, and propafenone in the reaction were 1 μM, the concentration of liver microsomes was 0.5 mg/mL, and the final concentrations of DMSO and acetonitrile in the reaction system were 0.01% (v/v) and 0.99% (v/v), respectively.

After incubating for an appropriate period (*e.g.,* 5, 15, 30, 45, and 60 minutes), 180 μL of termination solution (acetonitrile solution containing 200 ng/mL tolbutamide and 200 ng/mL labetalol) was added to the sample wells of each termination plate, and then 60 μL of sample was removed from the T60 incubation plate to terminate the reaction.

All sample plates were shaken well and centrifuged at 3220 × g for 20 minutes. 80 μL of supernatant from each well was then diluted to 240 μL with pure water for liquid chromatography-tandem mass spectrometry analysis.

The experimental results are shown in Table 4.

Table 4: Liver microsomal stability test results of the compound of the present disclosure

| Compound | Liver microsomal stability $CL_{int}$ (mL/min/kg) | | |
|---|---|---|---|
| | **Human** | **Dog** | **Rat** |
| **3A** | **20.4** | **47.7** | **53.6** |

Conclusion: The compound of the present disclosure exhibits moderate metabolism in the liver microsome test.

**Experimental example 4: Pharmacokinetic testing in mice**

Experimental purpose: To test the pharmacokinetic properties of the compound of the present disclosure in CD-1 mice.

Experimental materials: CD-1 mice (female, 15 to 30 g, 6 to 9 weeks, Beijing Vital River Laboratory Animal Technology Co., Ltd.)

Experimental operation:

The pharmacokinetic characteristics of the compound in rodents were tested following intravenous injection and oral administration according to a standard protocol. In the experiment, the candidate compound was prepared as a clear solution for single intravenous injection and as a homogeneous suspension for single oral administration in mice. In the case of intravenous injection, the dose was 3 mg/kg, with the vehicle being 5% DMSO/10% Solutol/85% water. In the case of oral administration, the dose was 30 mg/kg, with the vehicle being 0.5% MC (4000 cps)/0.2% Tween-80. Whole blood samples were collected within 24 hours, centrifuged at 3000 g for 15 minutes at 4°C, and the supernatant was separated to obtain plasma samples. 20 times the volume of acetonitrile solution containing internal standard was added to precipitate the protein. The samples were vortexed for 10 minutes and centrifuged at 3220 g for 15 minutes at 4°C. The supernatant was transferred to a 96-well plate and centrifuged at 3220 g for 5 minutes at 4°C. The supernatant was then injected for LC-MS/MS analysis to quantify plasma concentration, and pharmacokinetic parameters were calculated.

The experimental results are shown in Table 5.

Table 5: Results of pharmacokinetic testing in CD-1 mice

| Test sample | Injection | | Oral | |
|---|---|---|---|---|
| | Clearance Cl (mL/min/kg) | Half-life $T_{1/2}$ (h) | Concentration integral $AUC_{0\text{-last}}$ (h*nmol/L) | Bioavailability F (%) |
| **3A** | 30.0 | 1.29 | 16059 | 46.8 |

Conclusion: The compound of the present disclosure has good oral absorption bioavailability and plasma exposure in mice, indicating favorable pharmacokinetic properties.

**Experimental example 5: Pharmacokinetic testing in Beagles**

Experimental purpose: To test the pharmacokinetic properties of the compound of the present disclosure in Beagles.

Experimental materials: Beagles (male, ≥ 6 months old, Beijing Marshall Biotechnology Co., Ltd.)

Experimental operation:

The pharmacokinetic characteristics of the compound in dogs were tested following intravenous injection and oral administration according to a standard protocol. In the experiment, the test compound was prepared as a clear solution for single intravenous injection and single oral administration in dogs. In the case of intravenous injection, the dose was 2 mg/kg, with the vehicle being 5% DMSO/10% Solutol/85% water. In the case of oral administration, the dose

was 10 mg/kg, with the vehicle being 5% DMSO/10% Solutol/85% water. Whole blood samples were collected within 48 hours, centrifuged at 3000 g for 15 minutes at 4°C, and the supernatant was separated to obtain plasma samples. 20 times the volume of acetonitrile solution containing internal standard was added to precipitate the protein. The samples were vortexed for 10 minutes and centrifuged at 3220 g for 15 minutes at 4°C. The supernatant was transferred to a 96-well plate and centrifuged at 3220 g for 5 minutes at 4°C. The supernatant was then injected for LC-MS/MS analysis to quantify plasma concentration, and pharmacokinetic parameters were calculated.

Experimental results: The experimental results are shown in Table 6.

Table 6: Results of pharmacokinetic testing in Beagles

| Test sample | Injection | | Oral | |
|---|---|---|---|---|
| | Clearance (mL/min/kg) | Half-life $T_{1/2}$ (h) | Plasma concentration integral $AUC_{0\text{-last}}$ (h*nmol/L) | Bioavailability F (%) |
| 3A | 4.29 | 5.90 | 91502 | 113 |

Conclusion: The compound of the present disclosure has good oral absorption bioavailability and plasma exposure in Beagles, indicating favorable pharmacokinetic properties.

**Experimental example *6: In vivo* efficacy test**

**Experimental purpose:**

To evaluate the tumor inhibitory effect of the compounds of the present disclosure on a human large cell lung cancer LU99 subcutaneous xenograft tumor model.

**Experimental methods:**

Female BALB/c nude mice were subcutaneously inoculated with the human large cell lung cancer LU99 cell line. After inoculation, the mice were randomly divided into groups based on body weight and tumor volume, with six animals per group, and were administered as described below.

Group 1 (vehicle group): When the tumor volume reached 207 ± 9 $mm^3$ after inoculation, the mice were administered daily by gavage with the vehicle (5% DMSO/10% Solutol/85% double-distilled water) at a dose of 0.1 mL/10 g.

Group 2 (treatment group): When the tumor volume reached 207 ± 10 $mm^3$ after inoculation, the mice were administered daily by gavage with the test compound (compound dissolved in 5% DMSO/10% Solutol/85% double-distilled water) at a dose of 12.5 mg/kg.

Group 3 (treatment group): When the tumor volume reached 207 ± 10 $mm^3$ after inoculation, the mice were administered daily by gavage with the test compound (compound dissolved in 5% DMSO/10% Solutol/85% double-distilled water) at a dose of 25 mg/kg.

Group 4 (treatment group): When the tumor volume reached 207 ± 14 $mm^3$ after inoculation, the mice were administered daily by gavage with the test compound (compound dissolved in 5% DMSO/10% Solutol/85% double-distilled water) at a dose of 50 mg/kg.

After the experimental grouping, the mice were weighed twice weekly, and the tumor diameters were measured using a vernier caliper. The tumor volume was calculated, and then the tumor growth inhibition (TGI) was calculated. On day 29 after experimental administration, the mice were euthanized and sampled. The calculation formulas are as follows:

$$\text{Tumor volume} = 0.5 \times \text{Length} \times \text{Width}^2$$

TGI (%) = $[1 - (T_i - T_0) / (C_i - C_0)] \times 100\%$, where $T_i$ is the average tumor volume of a given administration group on a given day, $T_0$ is the average tumor volume of the administration group at the start of administration, $C_i$ is the average tumor volume of the control group on a given day (the same day as $T_i$), and $C_0$ is the average tumor volume of the control group at the start of administration.

**Experimental results:** The experimental results are shown in Table 7.

Table 7: Evaluation of tumor inhibitory efficacy of the compounds of the present disclosure on human large cell lung cancer LU99 subcutaneous xenograft tumor model

| Group | Tumor volume ($mm^3$) | | | | | | | | | TGI* (%) |
|---|---|---|---|---|---|---|---|---|---|---|
| | Day 0 | Day 4 | Day 8 | Day 11 | Day 15 | Day 18 | Day 22 | Day 25 | Day 29 | |
| Vehicle group | 207 | 313 | 425 | 549 | 699 | 861 | 1151 | 1387 | 1832 | / |
| Compound 3A (12.5 mg/kg) | 207 | 253 | 297 | 331 | 382 | 440 | 494 | 565 | 755 | 66 |
| Compound 3A (25 mg/kg) | 207 | 254 | 247 | 240 | 238 | 248 | 282 | 363 | 491 | 83 |
| Compound 3A (50 mg/kg) | 207 | 239 | 238 | 209 | 206 | 193 | 226 | 273 | 412 | 87 |
| *: calculated based on tumor volume on day 29 after administration | | | | | | | | | | |

Conclusion: The compounds of the present disclosure exhibit a significant tumor inhibitory effect on the human large cell lung cancer LU99 subcutaneous xenograft tumor model, while maintaining the body weight in mice well after administration.

## Claims

1. A compound of formula (III) or a pharmaceutically acceptable salt thereof

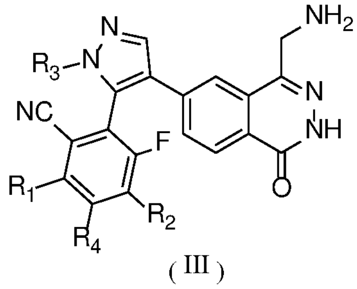

(III)

,

wherein

$R_1$ is selected from $C_{2-5}$ alkenyl, $C_{2-5}$ alkynyl, $-O-C_{3-5}$ cycloalkyl, $-O-C_{2-3}$ alkynyl, and $C_{3-5}$ cycloalkyl, and the $C_{2-5}$ alkenyl, $C_{2-5}$ alkynyl, $-O-C_{3-5}$ cycloalkyl, $-O-C_{2-3}$ alkynyl, and $C_{3-5}$ cycloalkyl are each independently and optionally substituted by 1, 2, or 3 $R_a$;
$R_2$ is selected from halogen and $C_{2-3}$ alkynyl, and the $C_{2-3}$ alkynyl is optionally substituted by 1, 2, or 3 halogens;
$R_3$ is selected from $CH_3$ and $CD_3$;
$R_4$ is selected from H and halogen;
each $R_a$ is independently selected from halogen and $C_{1-3}$ alkyl, and the $C_{1-3}$ alkyl is optionally substituted by 1, 2, or 3 halogens;
and at least one of the following conditions is satisfied:

(1) when $R_2$ is selected from halogen, $R_1$ is selected from $C_{2-5}$ alkenyl, $C_{2-5}$ alkynyl, $-O-C_{3-5}$ cycloalkyl, $-O-C_{2-3}$ alkynyl, and $C_{3-5}$ cycloalkyl; the $C_{2-5}$ alkenyl, $C_{2-5}$ alkynyl, and $-O-C_{2-3}$ alkynyl are optionally substituted by 1, 2, or 3 $R_a$, and the $-O-C_{3-5}$ cycloalkyl and $C_{3-5}$ cycloalkyl are substituted by 1, 2, or 3 $R_a$;
(2) when $R_2$ is selected from halogen, $R_3$ is selected from $CD_3$;
(3) when $R_2$ is selected from halogen, $R_4$ is selected from halogen.

2. The compound or the pharmaceutically acceptable salt thereof according to claim 1, wherein each $R_a$ is independently

selected from F, Cl, $CH_3$, $CHF_2$, and $CF_3$.

**3.** The compound or the pharmaceutically acceptable salt thereof according to claim 1, wherein $R_1$ is selected from $C_{2-3}$ alkenyl, $C_{2-3}$ alkynyl, -O-cyclopropyl, -O-$C_{2-3}$ alkynyl, and cyclopropyl, and the $C_{2-3}$ alkenyl, $C_{2-3}$ alkynyl, -O-cyclopropyl, -O-$C_{2-3}$ alkynyl, and cyclopropyl are each independently and optionally substituted by 1, 2, or 3 $R_a$; or, $R_1$ is selected from

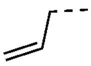, 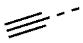, 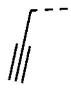, 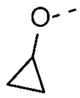, 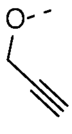, and 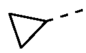, and the 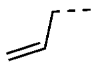, 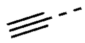, 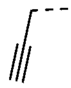,

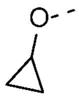, 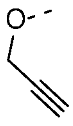, and 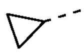

are independently and optionally substituted by 1, 2, or 3 $R_a$.

**4.** The compound or the pharmaceutically acceptable salt thereof according to claim 3, wherein $R_1$ is selected from

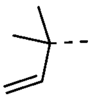, 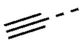, 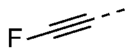, 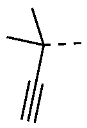, 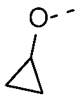, 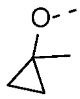, 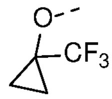,

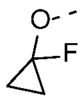, 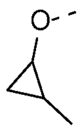, 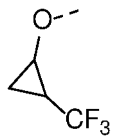, 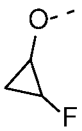, 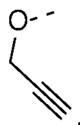, 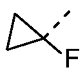, and 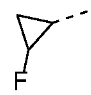.

**5.** The compound or the pharmaceutically acceptable salt thereof according to claim 1, wherein $R_2$ is selected from F, Cl, and

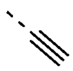,

and the

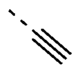

is optionally substituted by 1, 2, or 3 halogens; or, $R_2$ is selected from Cl,

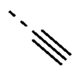,

and

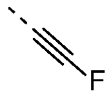.

6. The compound or the pharmaceutically acceptable salt thereof according to claim 1, wherein $R_2$ is selected from Cl, and $R_1$ is selected from

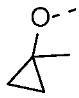, 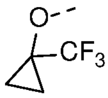, 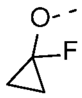, 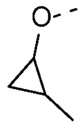,

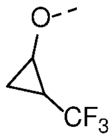, and 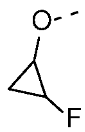.

7. The compound or the pharmaceutically acceptable salt thereof according to claim 1, wherein $R_2$ is selected from Cl and $R_3$ is selected from $CD_3$.

8. The compound or the pharmaceutically acceptable salt thereof according to claim 1, wherein $R_4$ is selected from H and F.

9. The compound or the pharmaceutically acceptable salt thereof according to any one of claims 1 to 8, selected from:

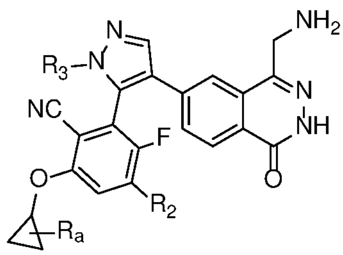

(P) ,

wherein $R_2$, $R_3$, and $R_a$ are as defined in any one of claims 1 to 8.

10. The compound or the pharmaceutically acceptable salt thereof according to claim 9, selected from:

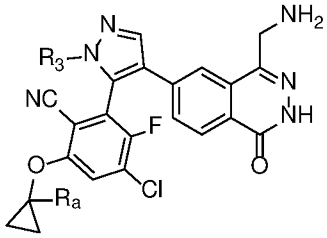

(P-1) ,

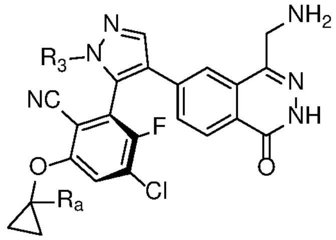

(P-1a) , and

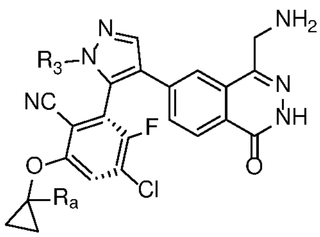

(P-1b) ,

wherein $R_3$ and $R_a$ are as defined in claim 9.

11. The compound or the pharmaceutically acceptable salt thereof according to claim 9, selected from:

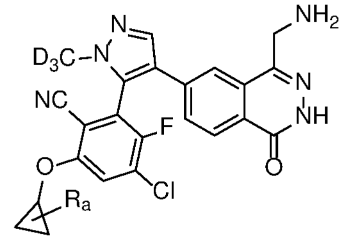
(P-2) ,
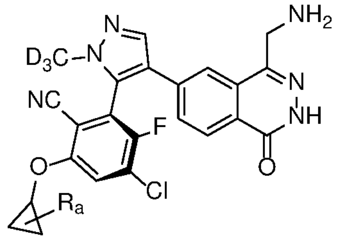
(P-2a) , and
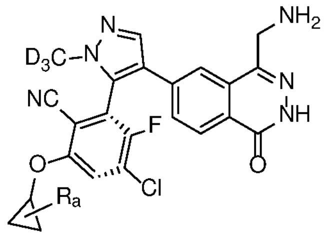
(P-2b) ,

wherein $R_a$ is as defined in claim 9.

**12.** The compound or the pharmaceutically acceptable salt thereof according to claim 11, selected from:

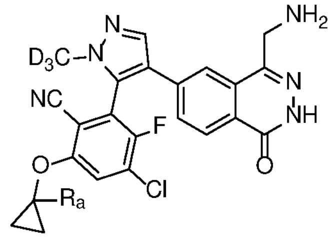
(P-3) ,
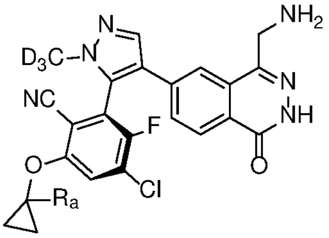
(P-3a) , and
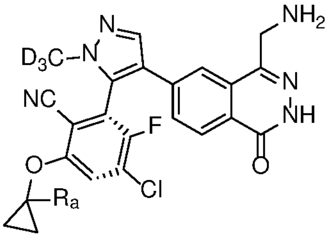
(P-3b) ,

wherein $R_a$ is as defined in claim 11.

**13.** A compound of the following formulas or a pharmaceutically acceptable salt thereof,

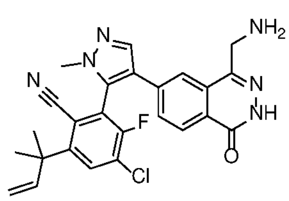
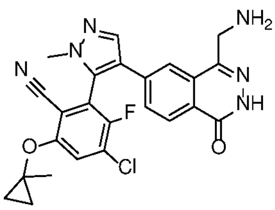
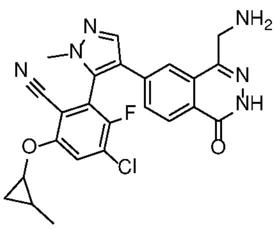
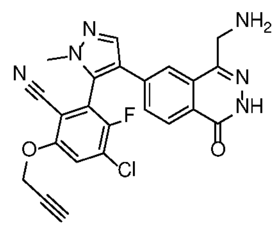
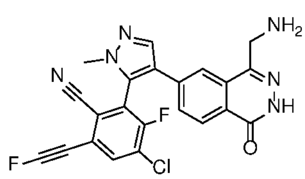
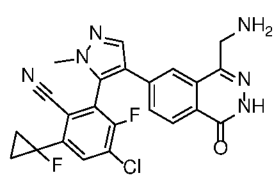
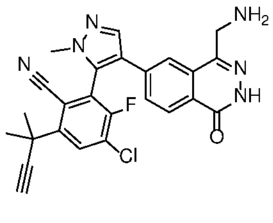
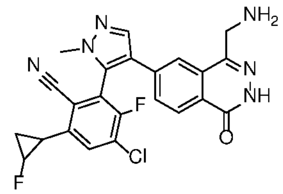
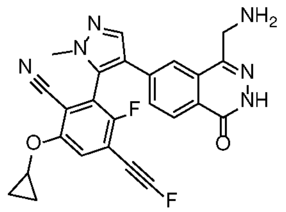
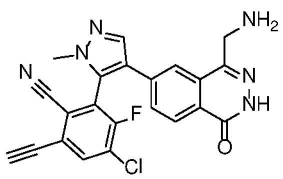
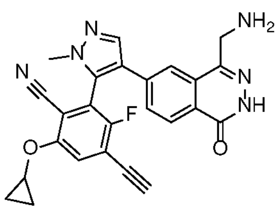
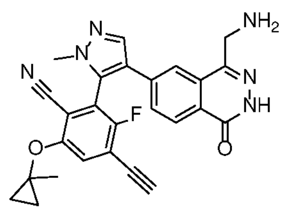

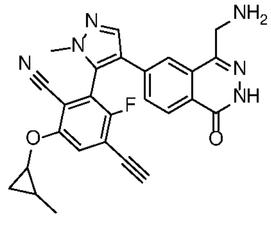
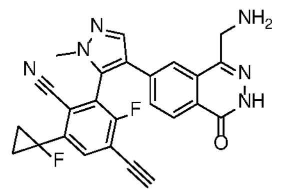
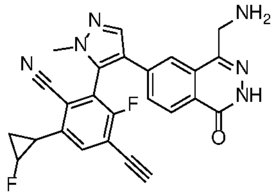
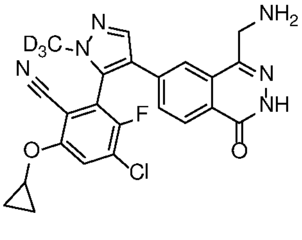

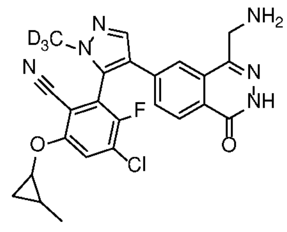
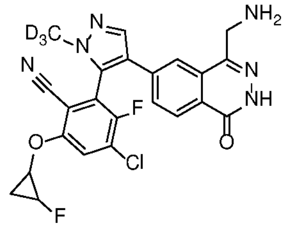
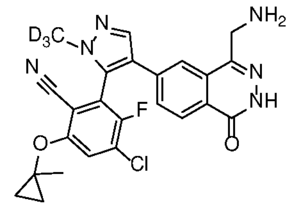
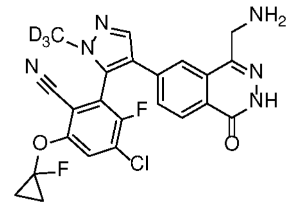

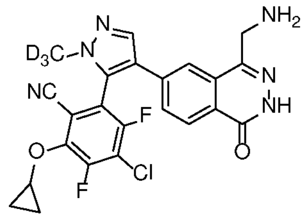
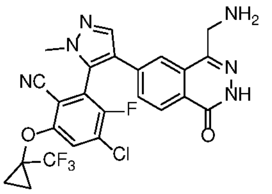
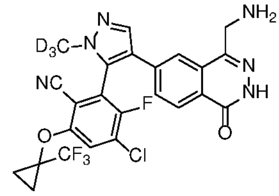
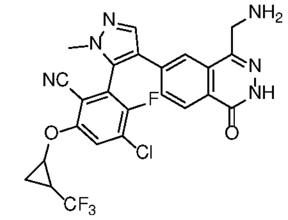

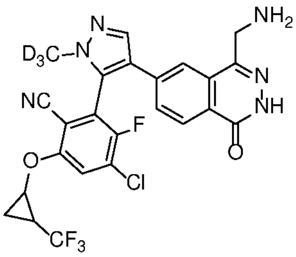
.

**14.** The compound or the pharmaceutically acceptable salt thereof according to claim 13, selected from:

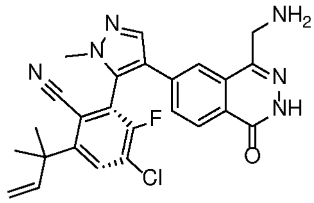
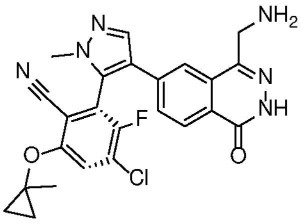
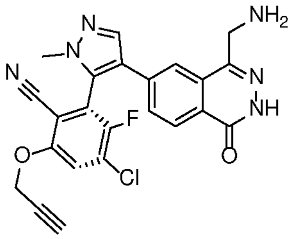

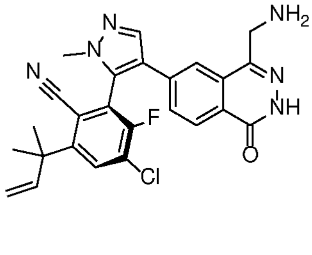
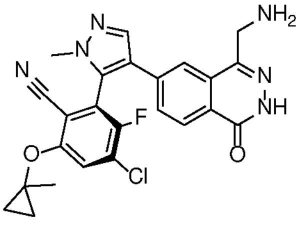
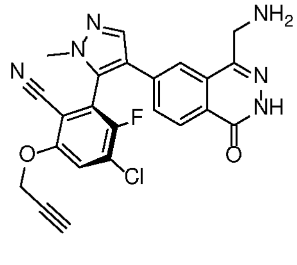

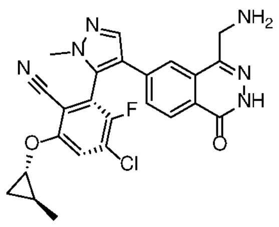 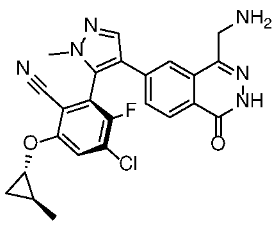 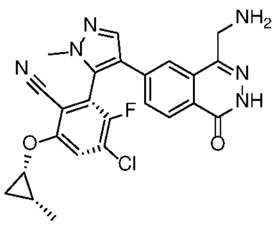 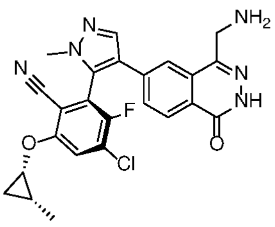

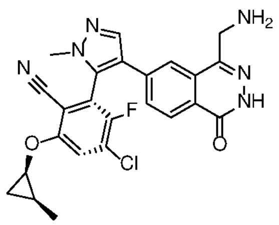 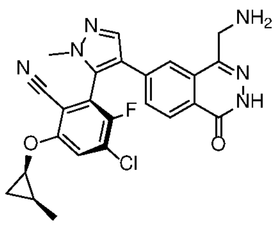 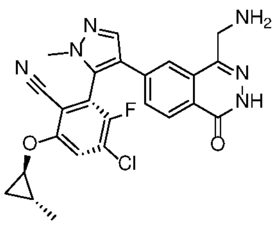 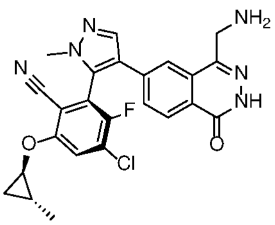

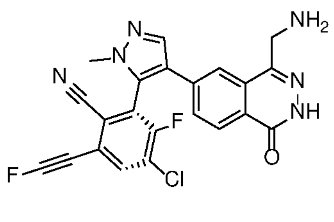 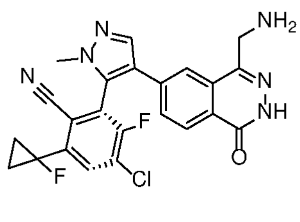 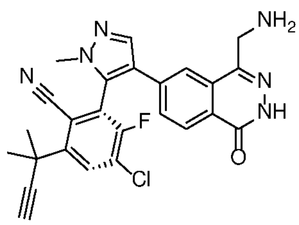

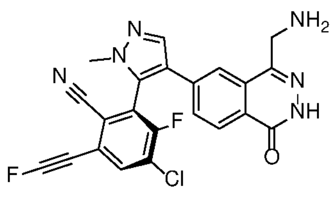 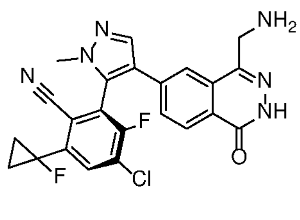 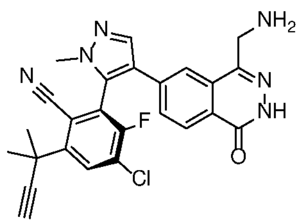

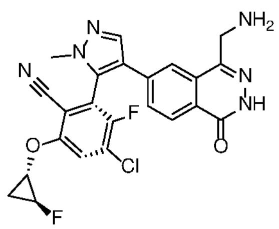 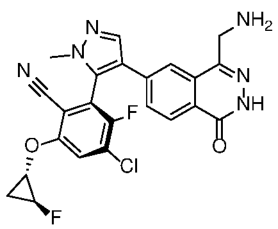 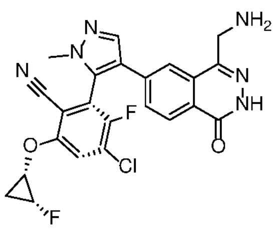 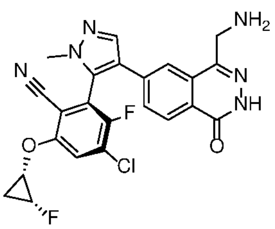

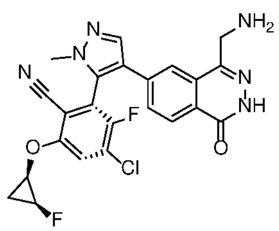 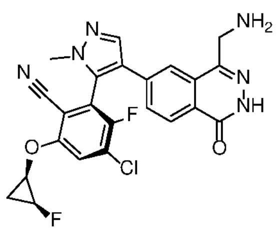 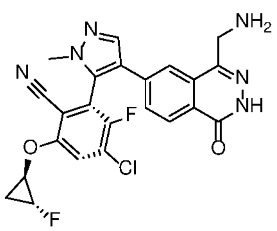 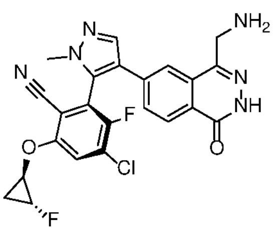

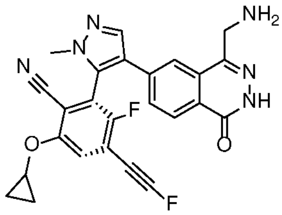
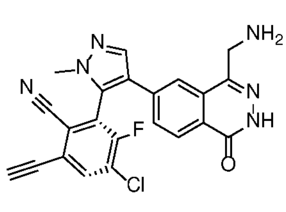
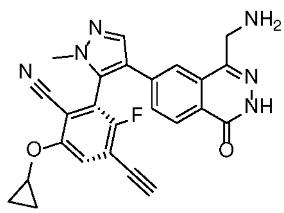
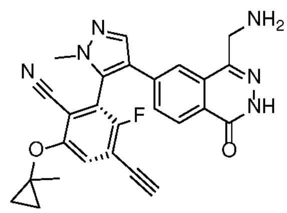
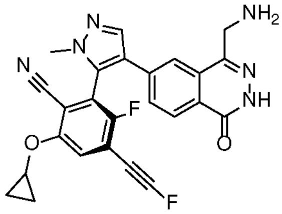
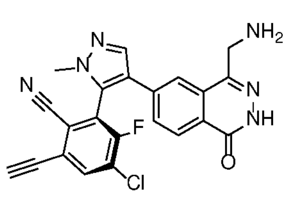
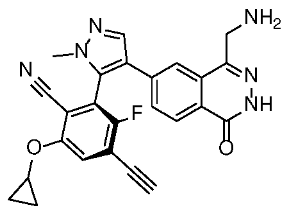
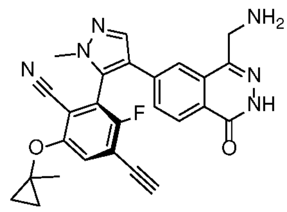
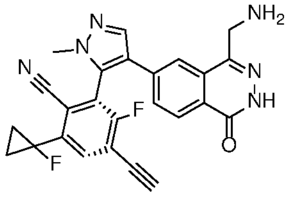
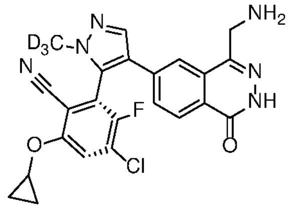
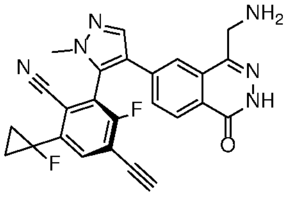
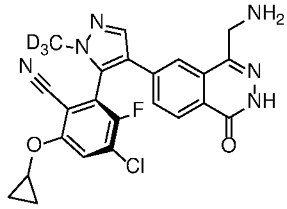
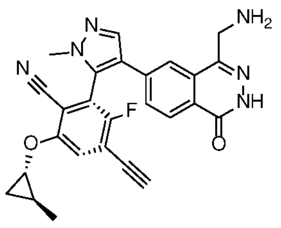
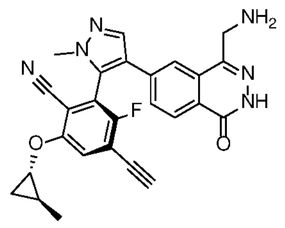
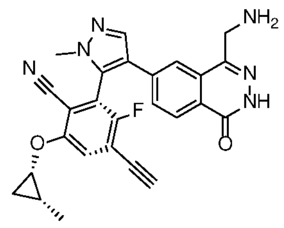
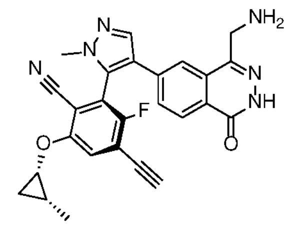
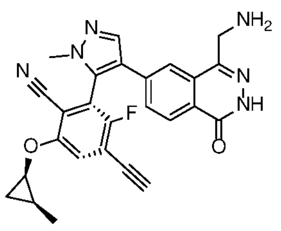
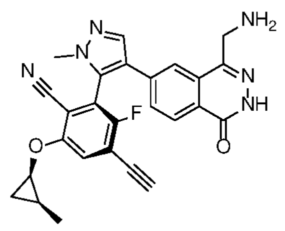
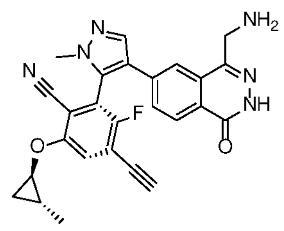
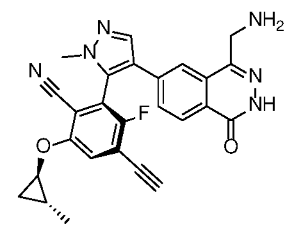
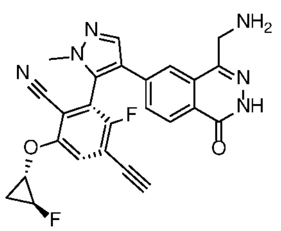
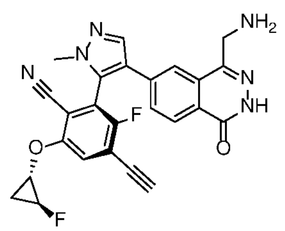
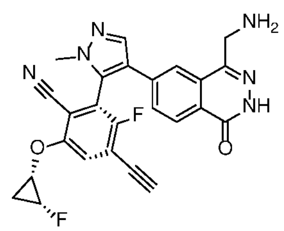
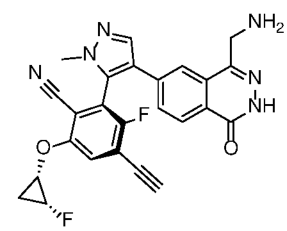

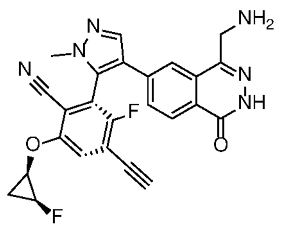
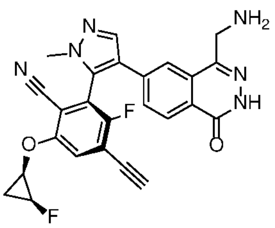
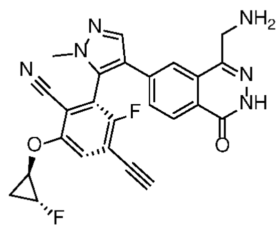
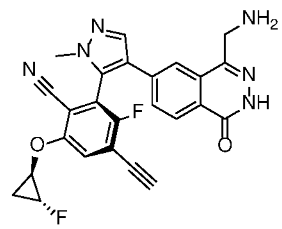

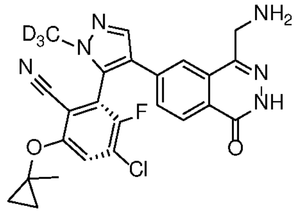
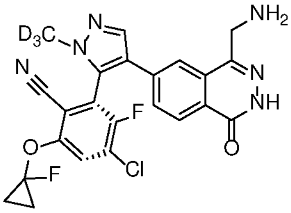
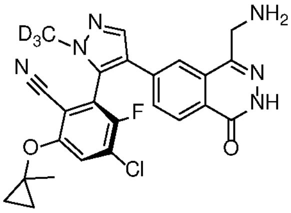
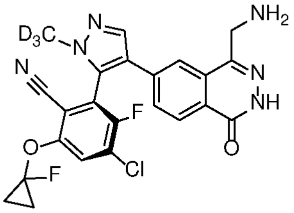

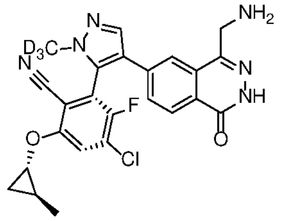
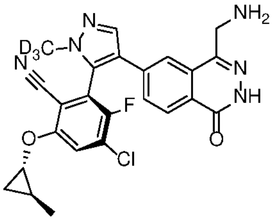
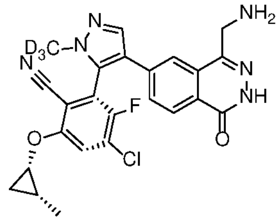
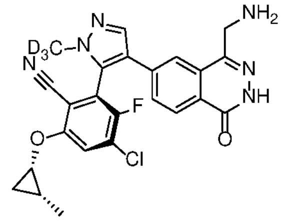

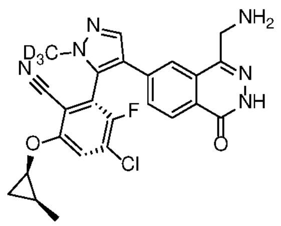
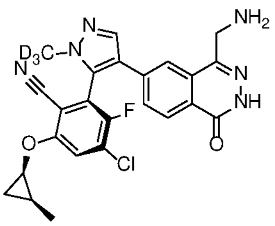
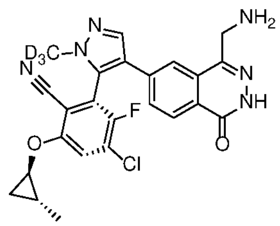
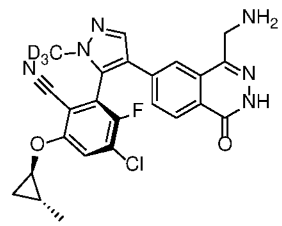

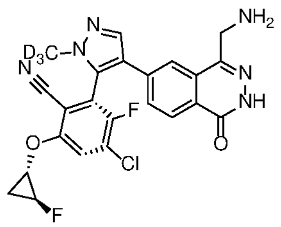
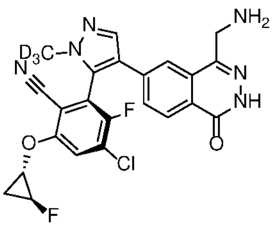
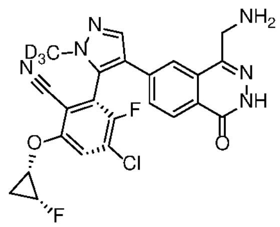
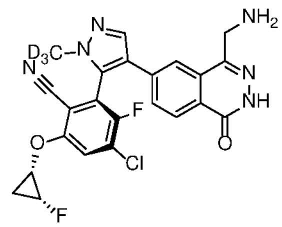

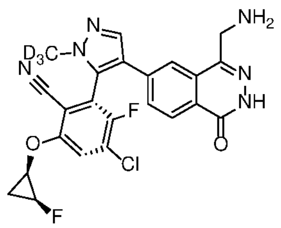
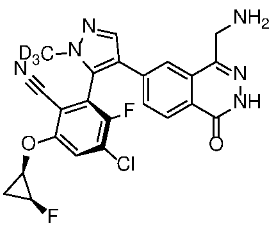
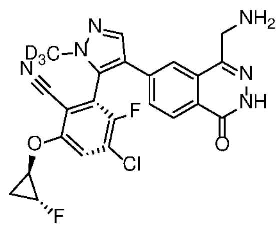
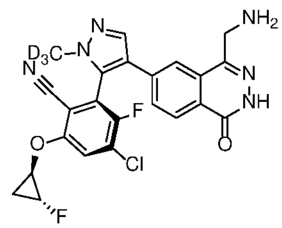

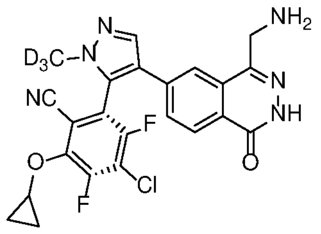 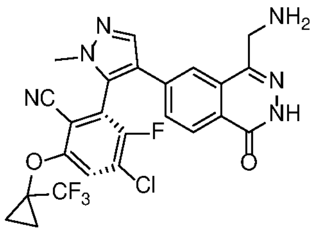 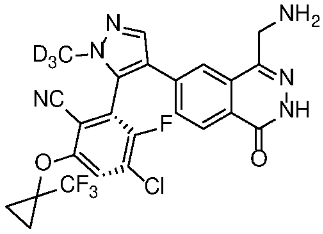

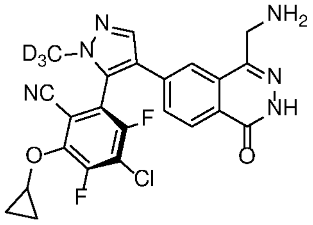 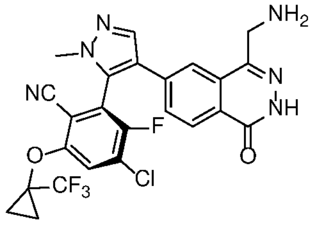 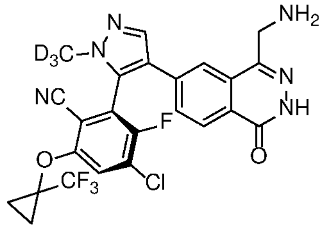

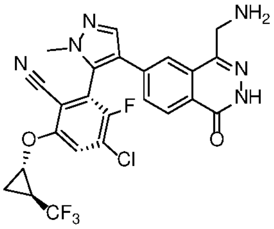 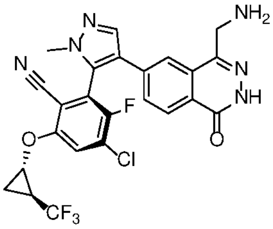 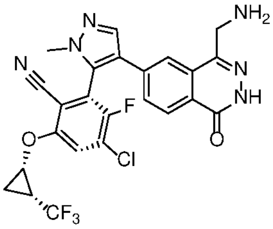 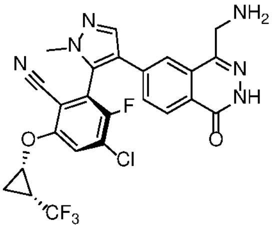

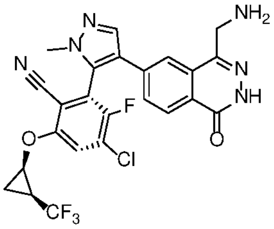 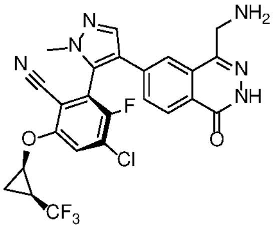 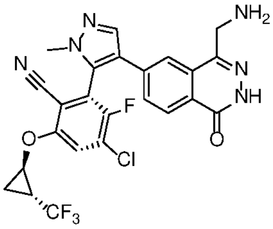 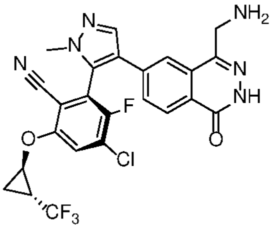

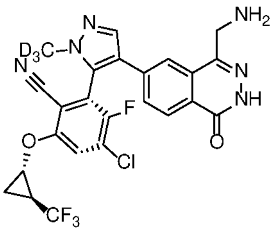 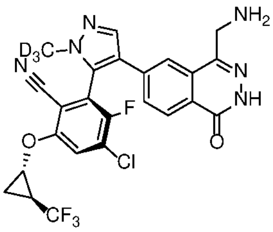 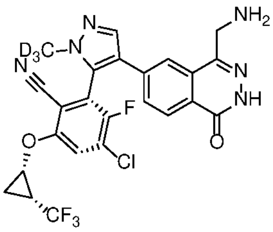 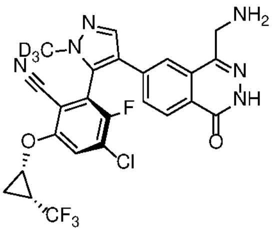

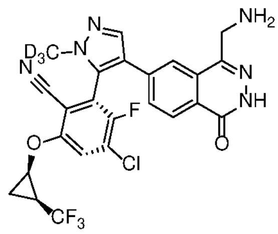 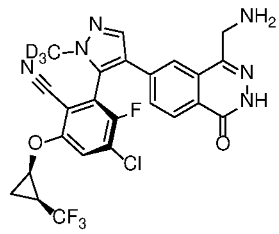 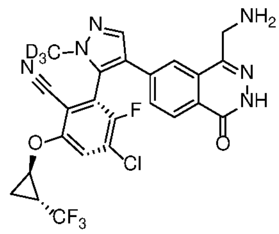 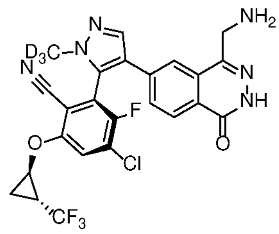 .

**15.** A use of the compound or the pharmaceutically acceptable salt thereof according to any one of claims 1 to 14 in a medicament related to a PRMT5·MTA complex inhibitor.

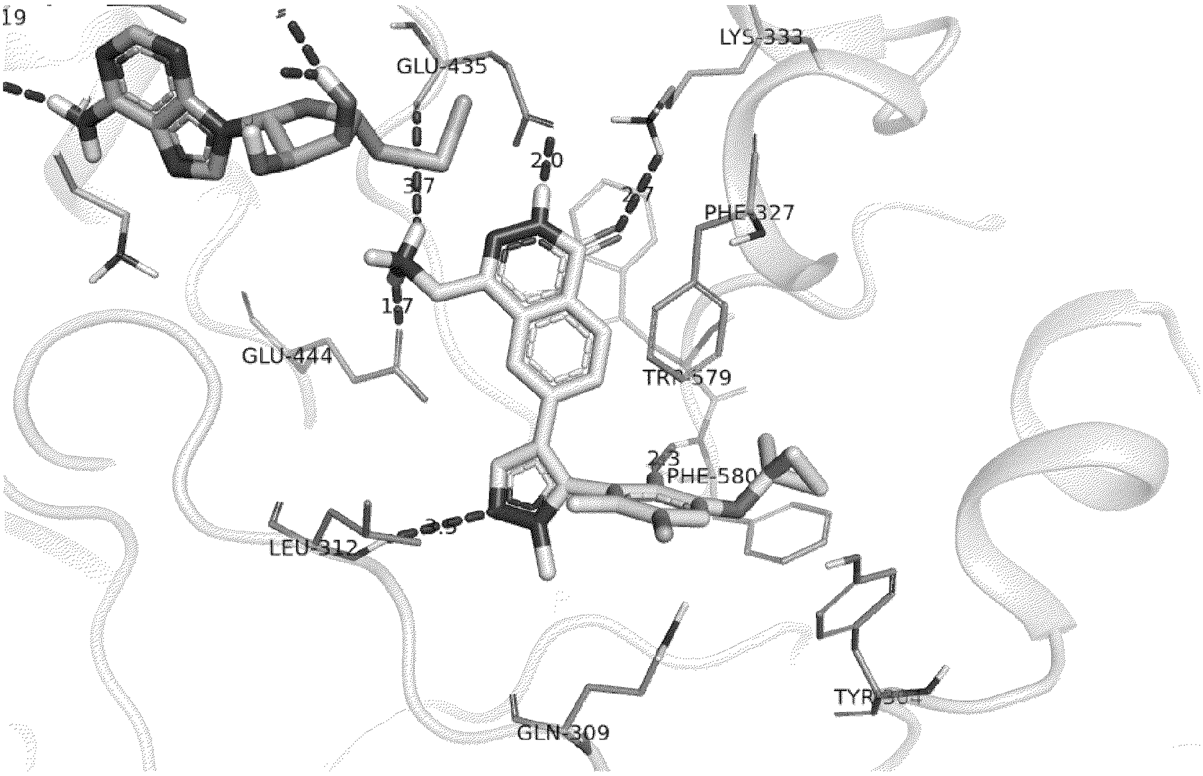
19
GLU-435
LYS-333
2.0
3.7
2.7
PHE-327
1.7
GLU-444
2.3
PHE-580
LEU-312
GLN-309

Fig. 1

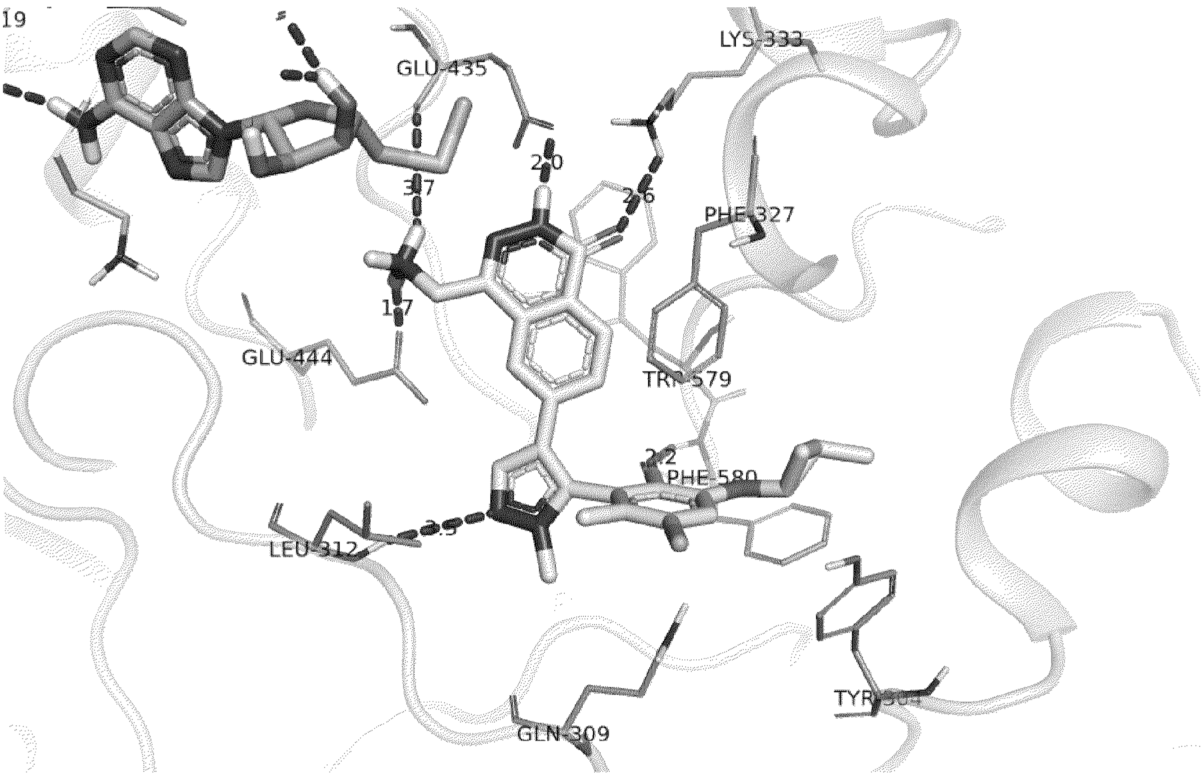
19
GLU-435
LYS-333
2.0
3.7
2.6
PHE-327
1.7
GLU-444
2.2
PHE-580
LEU-312
GLN-309

Fig. 2

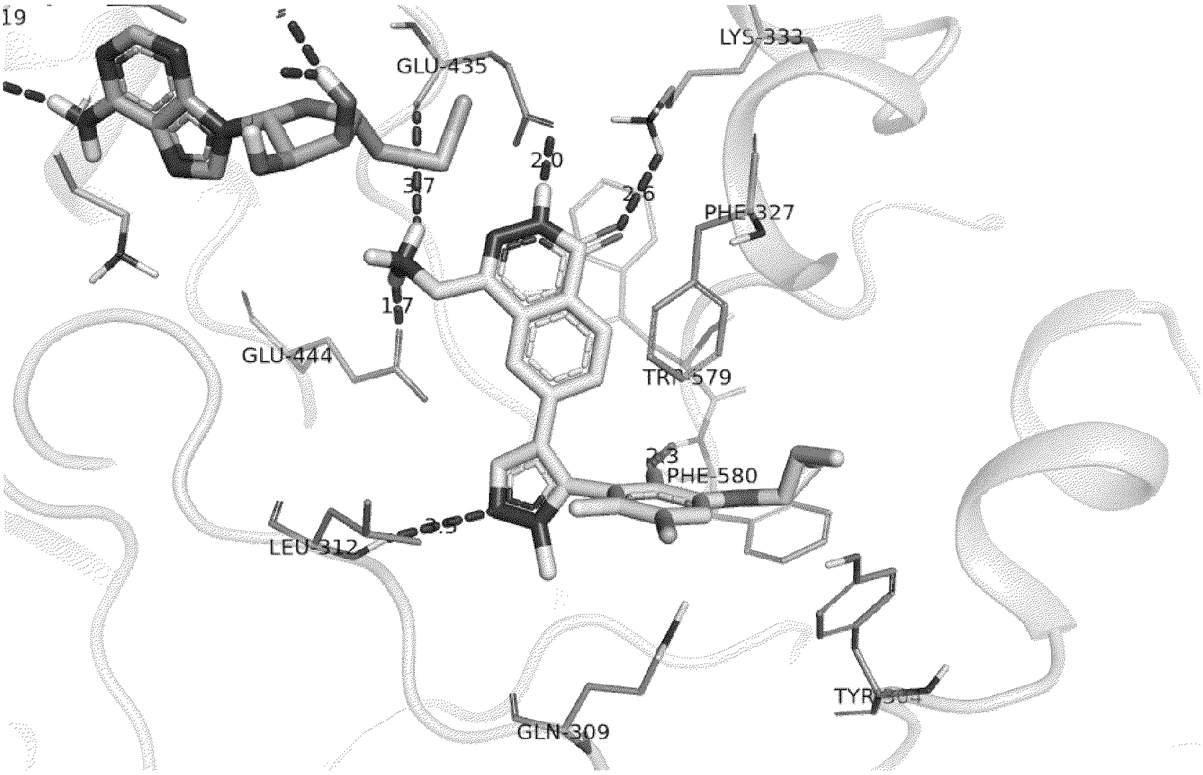
19
GLU-435
LYS-333
2.0
3.7
2.6
PHE-327
1.7
GLU-444
TRP-579
2.3
PHE-580
LEU-312
TYR-304
GLN-309

Fig. 3

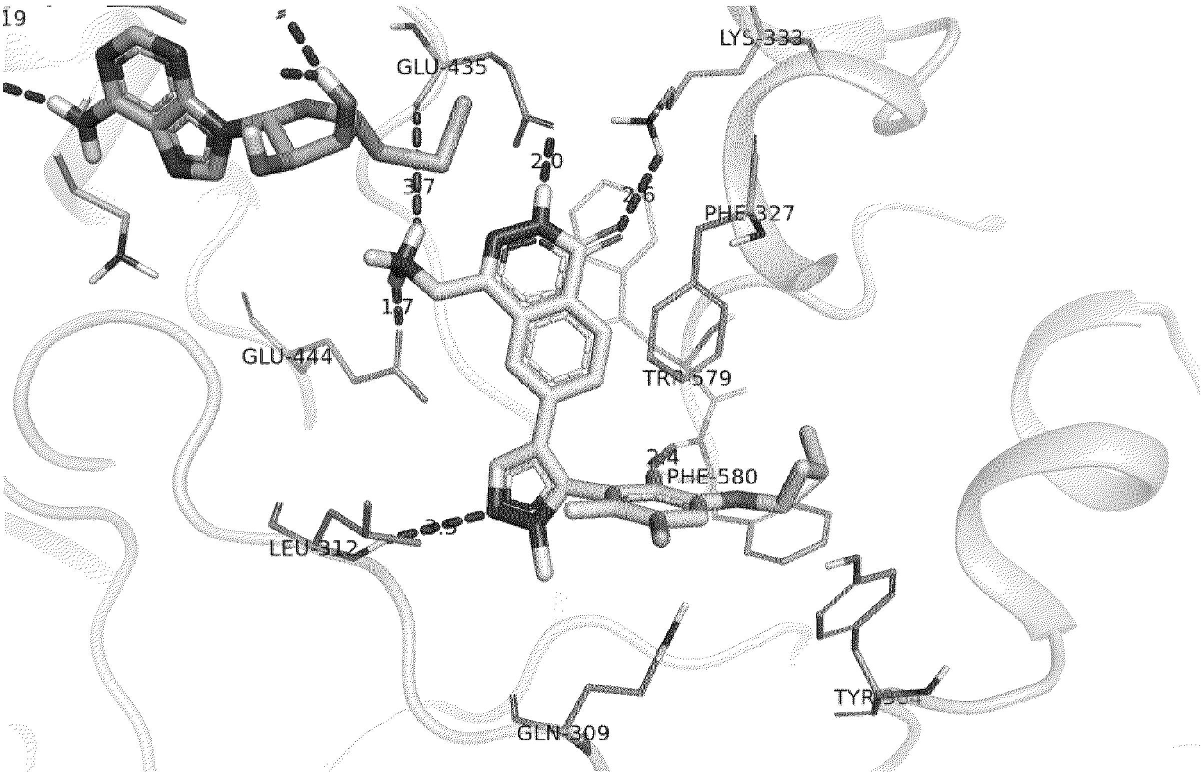
19
GLU-435
LYS-333
2.0
3.7
2.6
PHE-327
1.7
GLU-444
TRP-579
2.4
PHE-580
LEU-312
TYR-304
GLN-309

Fig. 4

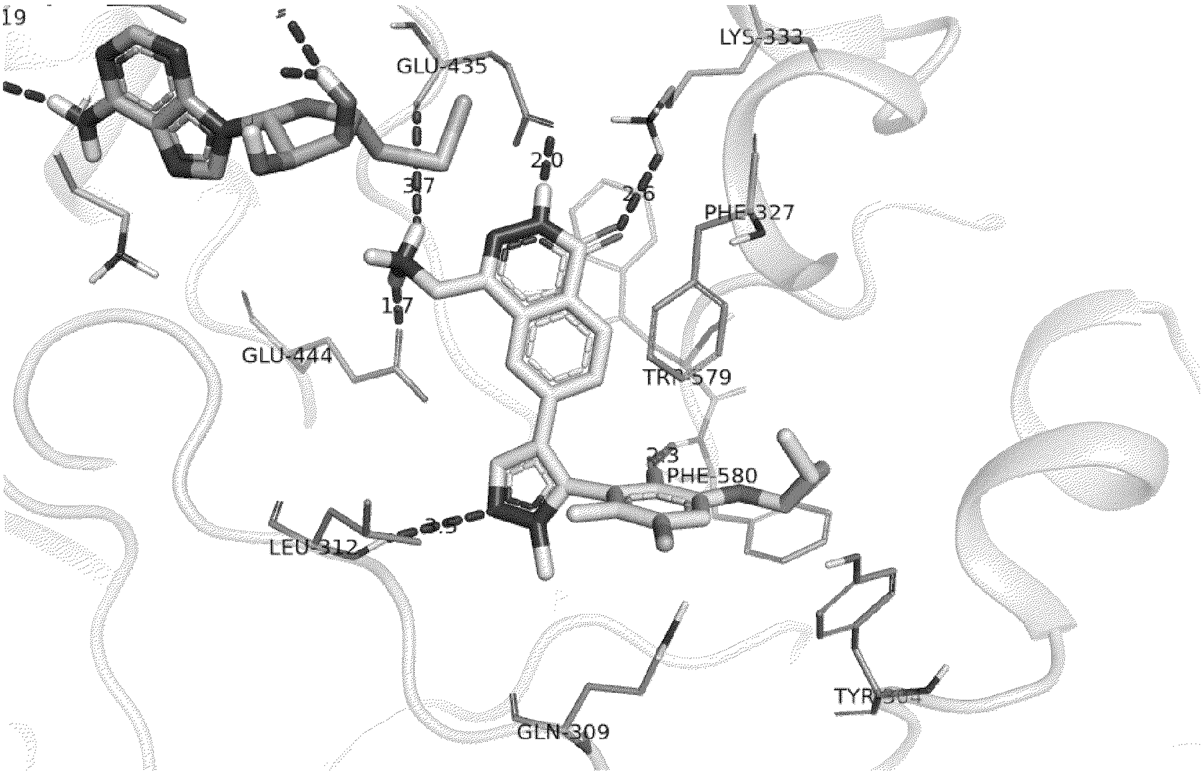
GLU-435
LYS-333
2.0
2.6
PHE-327
GLU-444
PHE-580
LEU-312
GLN-309

Fig. 5

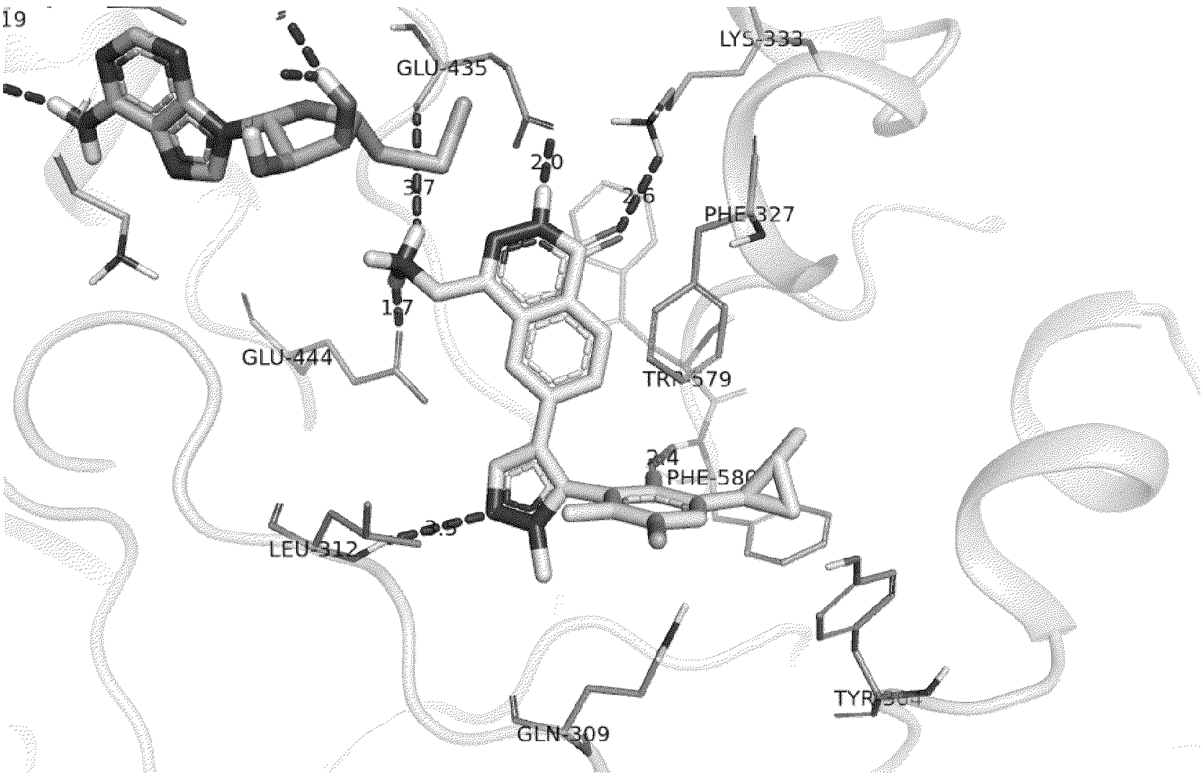
GLU-435
LYS-333
2.0
2.6
PHE-327
GLU-444
2.4
LEU-312
GLN-309

Fig. 6

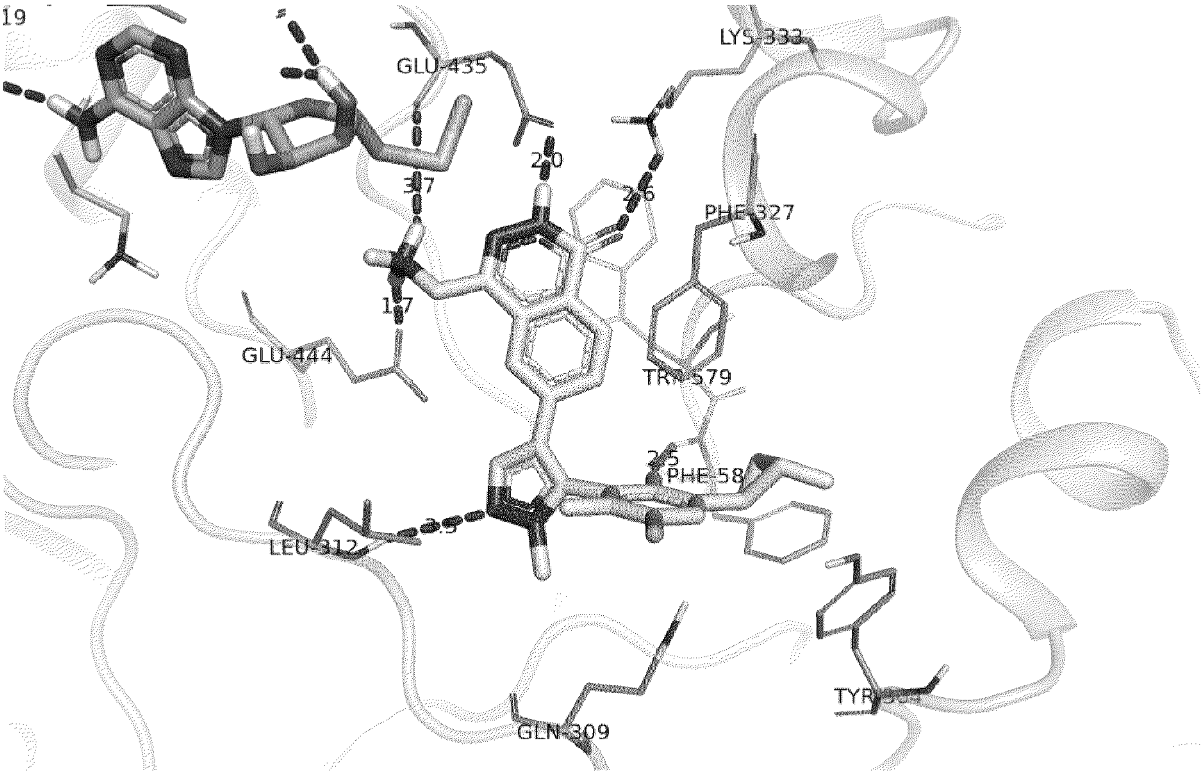
19
GLU-435
LYS-333
2.0
3.7
2.6
PHE-327
1.7
GLU-444
PHE-58
LEU-312
GLN-309

Fig. 7

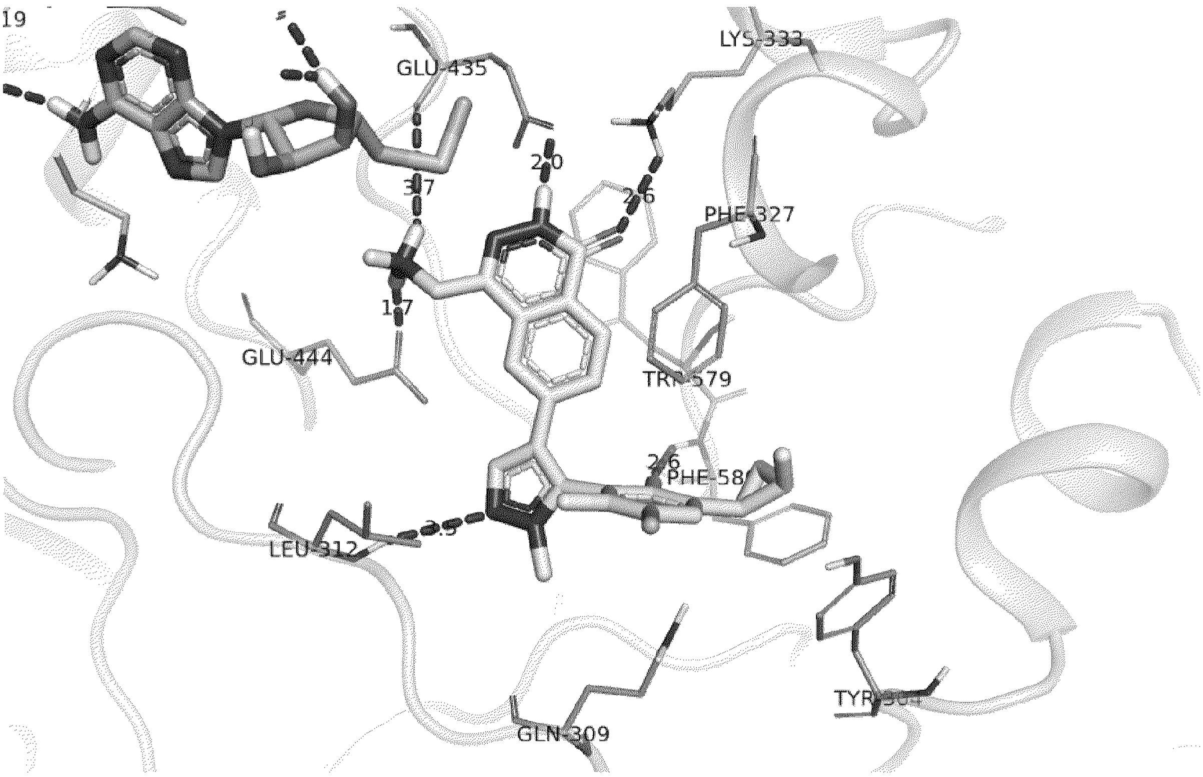
19
GLU-435
LYS-333
2.0
3.7
2.6
PHE-327
1.7
GLU-444
PHE-58
LEU-312
GLN-309

Fig. 8

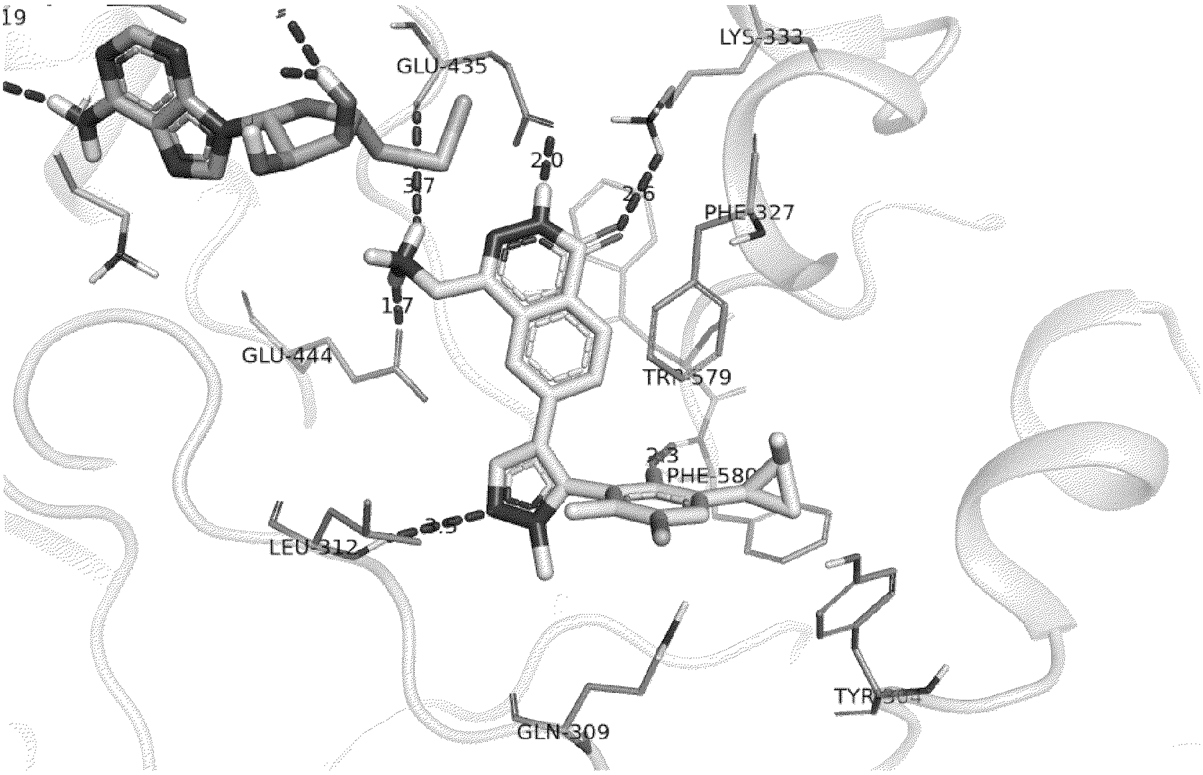
19
LYS-333
GLU-435
2.0
3.7
2.6
PHE-327
1.7
GLU-444
TRP-579
2.3
PHE-580
LEU-312
TYR-304
GLN-309

Fig. 9

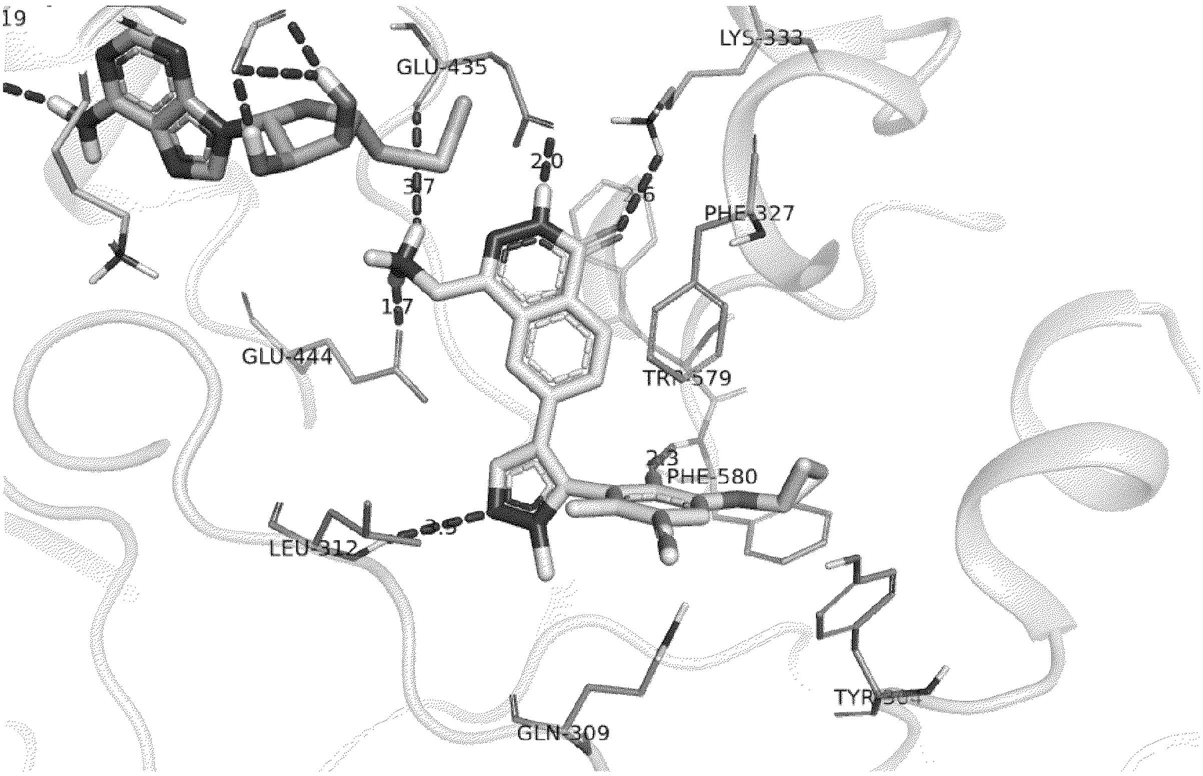
19
LYS-333
GLU-435
2.0
3.7
2.6
PHE-327
1.7
GLU-444
TRP-579
2.3
PHE-580
LEU-312
TYR-304
GLN-309

Fig. 10

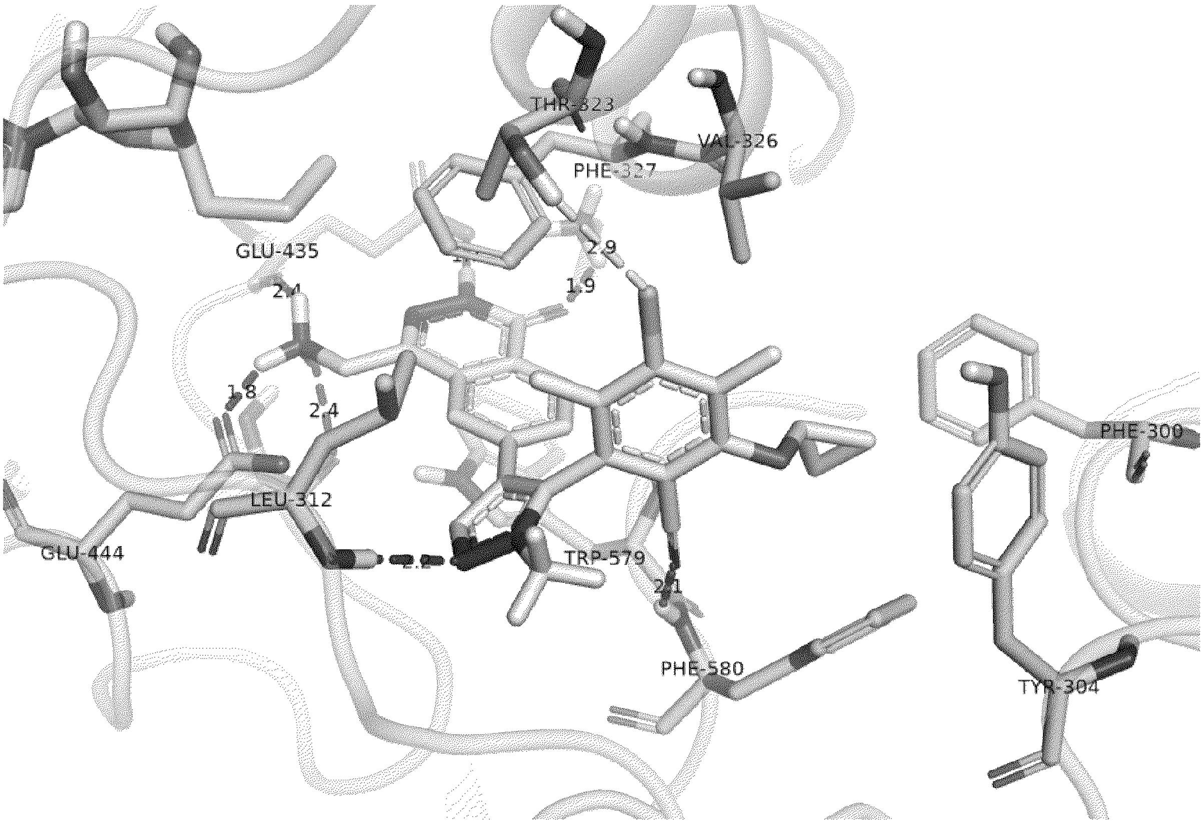
THR-323
VAL-326
PHE-327
GLU-435
2.9
2.4
1.9
1.8
2.4
PHE-300
LEU-312
GLU-444
TRP-579
2.1
PHE-580
TYR-304

Fig. 11

**INTERNATIONAL SEARCH REPORT**

International application No.
**PCT/CN2022/142362**

**A. CLASSIFICATION OF SUBJECT MATTER**

C07D237/32(2006.01)i;C07D403/10(2006.01)i;C07D487/00(2006.01)i;A61K31/502(2006.01)i;A61P35/00(2006.01)i

According to International Patent Classification (IPC) or to both national classification and IPC

**B. FIELDS SEARCHED**

Minimum documentation searched (classification system followed by classification symbols)

IPC：C07D; A61K; A61P

Documentation searched other than minimum documentation to the extent that such documents are included in the fields searched

Electronic data base consulted during the international search (name of data base and, where practicable, search terms used)

CNTXT, ENTXT, DWPI, CJFD, CNKI, Caplus(STN), Registry(STN), Marpat(STN): 南京明德, 吡唑, 酞嗪酮, 苯, 抑制剂, 癌症, dihydrophthalazin, pyrazol, benz, oxo, inhibitor, cancer, PRMT5, MTA, structure search

**C. DOCUMENTS CONSIDERED TO BE RELEVANT**

| Category* | Citation of document, with indication, where appropriate, of the relevant passages | Relevant to claim No. |
|---|---|---|
| X | WO 2021050915 A1 (MIRATI THERAPEUTICS INC.) 18 March 2021 (2021-03-18) description page 243 embodiments 4-230, page 307 embodiments 16-7, 16-8, claims 1-97 | 1-15 |
| PX | WO 2022192745 A1 (MIRATI THERAPEUTICS INC.) 15 September 2022 (2022-09-15) claims 1-35 | 1-15 |
| PX | WO 2022216645 A1 (MIRATI THERAPEUTICS INC.) 13 October 2022 (2022-10-13) abstract, claims 15, 19, 20 | 1-15 |
| PX | WO 2022216648 A1 (MIRATI THERAPEUTICS INC.) 13 October 2022 (2022-10-13) abstract, claims 15, 19, 20 | 1-15 |
| PX | WO 2022256806 A1 (IDEAYA BIOSCIENCES, INC.) 08 December 2022 (2022-12-08) abstract, description, pages 173-174 table the last two compounds, and claim 17 | 1-15 |
| A | CN 101085759 A (PHARMACIA ITALIA S.P.A.) 12 December 2007 (2007-12-12) entire document | 1-15 |

☐ Further documents are listed in the continuation of Box C. ☑ See patent family annex.

* Special categories of cited documents:
“A” document defining the general state of the art which is not considered to be of particular relevance
“D” document cited by the applicant in the international application
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **17 March 2023** | **22 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/142362**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

Claim 15 relates to a method for diagnosing and treating diseases (PCT Rule 39.1(iv)). Therefore, the following search is carried out on the basis of the pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/142362**

| Patent document cited in search report | Publication date (day/month/year) | Patent family member(s) | Publication date (day/month/year) |
|---|---|---|---|
| WO 2021050915 A1 | 18 March 2021 | US 2023054883 A1 | 23 February 2023 |
| | | IL 290341 A | 01 April 2022 |
| | | US 2021079003 A1 | 18 March 2021 |
| | | US 11492351 B2 | 08 November 2022 |
| | | AU 2020345899 A1 | 03 March 2022 |
| | | JP 2022548255 A | 17 November 2022 |
| | | NO 20220309 A1 | 11 March 2022 |
| | | TW 202122387 A | 16 June 2021 |
| | | CL 2022000603 A1 | 27 January 2023 |
| | | CA 3150515 A1 | 18 March 2021 |
| | | CO 2022004513 A2 | 21 June 2022 |
| | | US 2021078994 A1 | 18 March 2021 |
| | | US 11479551 B2 | 25 October 2022 |
| | | BR 112022004248 A2 | 31 May 2022 |
| | | KR 20220083691 A | 20 June 2022 |
| | | EP 4028388 A1 | 20 July 2022 |
| WO 2022192745 A1 | 15 September 2022 | None | |
| WO 2022216645 A1 | 13 October 2022 | US 2022331323 A1 | 20 October 2022 |
| WO 2022216648 A1 | 13 October 2022 | US 2022331324 A1 | 20 October 2022 |
| WO 2022256806 A1 | 08 December 2022 | None | |
| CN 101085759 A | 12 December 2007 | EA 200400283 A1 | 26 August 2004 |
| | | EA 006645 B1 | 24 February 2006 |
| | | CZ 2004305 A3 | 18 August 2004 |
| | | DK 1427708 T3 | 24 November 2008 |
| | | DE 60228857 D1 | 23 October 2008 |
| | | US 2005020583 A1 | 27 January 2005 |
| | | US 7432263 B2 | 07 October 2008 |
| | | NZ 531310 A | 24 February 2006 |
| | | NO 20040547 L | 03 March 2004 |
| | | MXPA 04000905 A | 02 April 2004 |
| | | EP 1427708 A1 | 16 June 2004 |
| | | EP 1427708 B1 | 10 September 2008 |
| | | PL 368440 A1 | 21 March 2005 |
| | | ES 2315385 T3 | 01 April 2009 |
| | | ZA 200401316 B | 10 March 2005 |
| | | IL 159843 A0 | 20 June 2004 |
| | | CA 2455759 A1 | 20 February 2003 |
| | | US 2003073692 A1 | 17 April 2003 |
| | | JP 2005501085 A | 13 January 2005 |
| | | PT 1427708 E | 20 November 2008 |
| | | BR 0211807 A | 21 September 2004 |
| | | KR 20040029400 A | 06 April 2004 |
| | | AT 407928 T | 15 September 2008 |
| | | WO 03014090 A1 | 20 February 2003 |
| | | SI 1427708 T1 | 31 December 2008 |
| | | CO 5560573 A2 | 30 September 2005 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111616977
- CN 202210062133
- CN 202210142771
- CN 202211213451


### Non-patent literature cited in the description


- **SCHRÖDINGER.** Induced Fit Docking protocol; Glide. LLC, 2021
- **SCHRÖDINGER.** Prime. LLC, 2021
- **SCHRÖDINGER.** Maestro. LLC, 2021
- **SCHRÖDINGER.** LigPrep. LLC, 2021